(19) Europäisches Patentamt / European Patent Office / Office européen des brevets

(11) **EP 2 463 374 A2**

(12) **EUROPEAN PATENT APPLICATION**

(43) Date of publication:
**13.06.2012 Bulletin 2012/24**

(21) Application number: **11191120.2**

(22) Date of filing: **28.01.2009**

(51) Int Cl.:
*C12N 15/53* (2006.01)    *C12N 9/04* (2006.01)
*C12N 1/15* (2006.01)    *C12N 1/19* (2006.01)
*C12N 1/21* (2006.01)    *C12P 5/00* (2006.01)
*C12P 7/00* (2006.01)    *C12P 7/06* (2006.01)
*C12P 7/58* (2006.01)    *C12P 19/02* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **28.01.2008 US 24160 P**

(62) Document number(s) of the earlier application(s) in accordance with Art. 76 EPC:
**09706304.4 / 2 245 162**

(27) Previously filed application:
**28.01.2009 PCT/US2009/032258**

(71) Applicant: **Bio Architecture Lab, Inc.**
**Seattle, WA 98103 (US)**

(72) Inventor: **Kashiyama, Yuki**
**Seattle, WA 98103 (US)**

(74) Representative: **Fagerlin, Heléne et al**
**Albihns.Zacco AB**
**P.O. Box 5581**
**114 85 Stockholm (SE)**

Remarks:
This application was filed on 29-11-2011 as a divisional application to the application mentioned under INID code 62.

(54) **Isolated alcohol dehydrogenase enzymes and uses thereof**

(57)    Bacterial polynucleotides and polypeptides are provided in which the polypeptides have a dehydrogenase activity, such as an alcohol dehydrogenase (ADH) activity, an uronate, a 4-deoxy-L-erythro-5-hexoseulose uronate (DEHU) ((4S,5S)-4,5 dihydroxy-2,6-dioxohexanoate) hydrogenase activity, a 2-keto-3-deoxy-D-gluconate dehydrogenase activity, a D-mannuronate hydrogenase activity, and/or a D-mannnonate dehydrogenase activity. Methods, enzymes, recombinant microorganism, and microbial systems are also provided for converting polysaccharides, such as those derived from biomass, into suitable monosaccharides or oligosaccharides, as well as for converting suitable monosaccharides or oligosaccharides into commodity chemicals, such as biofuels. Commodity chemicals produced by the methods described herein are also provided.

NADPH consumption by ADH enzymes using DEHU as a substrate

*FIG. 1*

EP 2 463 374 A2

**Description**

<u>PRIORITY</u>

This application claims the benefit under 35 U.S.C. § 119(e) of U.S. Provisional Patent Application No. 61/024,160, filed January 28, 2008, which application is herein incorporated by reference in its entirety.

<u>STATEMENT REGARDING SEQUENCE LISTING</u>

The Sequence Listing associated with this application is provided in text format in lieu of a paper copy, and is hereby incorporated by reference into the specification. The name of the text file containing the Sequence Listing is 150097_402PC_SEQUENCE_LISTING.txt. The text file is 92 KB, was created on January 28, 2009, and is being submitted electronically via EFS-Web.

<u>TECHNICAL FIELD</u>

Embodiments of the present invention relate generally to isolated polypeptides, and polynucleotides encoding the same, having a dehydrogenase activity, such as an alcohol dehydrogenase (ADH) activity, an uronate, a 4-deoxy-L-erythro-5-hexoseulose uronate (DEHU) ((4S,5S)-4,5 dihydroxy-2,6-dioxohexanoate) hydrogenase activity, a 2-keto-3-deoxy-D-gluconate dehydrogenase activity, a D-mannuronate hydrogenase activity, and/or a D-mannnonate dehydrogenase activity, and to the use of recombinant microrganisms, microbial systems, and chemical systems comprising such polynucleotides and polypeptides to convert biomass to commodity chemicals such as biofuels.

<u>RELATED ART</u>

Presents method for converting biomass into biofuels focus on the use of lignocellulolic biomass, and there are many problems associated with using this process. Large-scale cultivation of lignocellulolic biomass requires substantial amount of cultivated land, which can be only achieved by replacing food crop production with energy crop production, deforestation, and by recultivating currently uncultivated land. Other problems include a decrease in water availability and quality and an increase in the use of pesticides and fertilizers.

The degradation of lignocellulolic biomass using biological systems is a very difficult challenge due to its substantial mechanistic strength and the complex chemical components. Approximately thirty different enzymes are required to fully convert lignocellulose to monosaccharides. The only available alternate to this complex approach requires a substantial amount of heat, pressure, and strong acids. The art therefore needs an economic and technically simple process for converting biomass into hydrocarbons for use as biofuels or biopetrols.

As one step in this process, enzymes having alcohol dehydrogenase activity are useful in converting polysaccharides from biomass into oligosaccharides or monosaccharides, which may be then converted to various biofuels. Enzymes having alcohol dehydrogenase activity, such as uronate, 4-deoxy-L-erythro-5-hexoseulose uronate (DEHU) and/or D-mannuronate hydrogenase activity, have been previously purified from alginate metabolizing bacteria, but no gene encoding a DEHU or D-mannuronate hydrogenase has been cloned and characterized. The present application provides genes that encode alcohol dehydrogenases having DEHU and/or D-mannuronate hydrogenase activity, and provides as well methods associated with their use in producing commodity chemicals, such as biofuels.

<u>BRIEF SUMMARY</u>

Embodiments of the present invention include isolated polynucleotides, and fragments or variants thereof, selected from

(a) an isolated polynucleotide comprising a nucleotide sequence at least 80% identical to the nucleotide sequence set forth in SEQ ID NO: 1, 3, 5, 7, 9, 11, 13, 15, 17, 19, 21, 23, 25, 27, 29, 31, 33, 35, or 37;
(b) an isolated polynucleotide comprising a nucleotide sequence at least 90% identical to the nucleotide sequence set forth in SEQ ID NO:1, 3, 5, 7, 9, 11, 13, 15, 17, 19, 21, 23, 25, 27, 29, 31, 33, 35, or 37;
(c) an isolated polynucleotide comprising a nucleotide sequence at least 95% identical to the nucleotide sequence set forth in SEQ ID NO:1, 3, 5, 7, 9, 11, 13, 15, 17, 19, 21, 23, 25, 27, 29, 31, 33, 35 or 37;
(d) an isolated polynucleotide comprising a nucleotide sequence at least 97% identical to the nucleotide sequence set forth in SEQ ID NO:1, 3, 5, 7, 9, 11, 13, 15, 17, 19, 21, 23, 25, 27, 29, 31, 33, 35, or 37;
(e) an isolated polynucleotide comprising a nucleotide sequence at least 99% identical to the nucleotide sequence set forth in SEQ ID NO:1, 3, 5, 7, 9, 11, 13, 15, 17, 19, 21, 23, 25, 27, 29, 31, 33, 35, or 37; and

(f) an isolated polynucleotide comprising the nucleotide sequence set forth in SEQ ID NO:1, 3, 5, 7, 9, 11, 13, 15, 17, 19, 21, 23, 25, 27, 29, 31, 33, 35, or 37,

wherein the isolated nucleotide encodes a polypeptide having a dehydrogenase activity. In other embodiments, the polypeptide has an alcohol dehydrogenase activity. In certain embodiments, the polypeptide has a DEHU hydrogenase activity and/or a D-mannuronate hydrogenase activity.

Additional embodiments include methods for converting a polysaccharide to a suitable monosaccharide or oligosaccharide, comprising contacting the polysaccharide with a microbial system, wherein the microbial system comprises a recombinant microorganism, and wherein the recombinant microorganism comprises a polynucleotide according to the present disclosure, wherein the polynucleotide encodes a polypeptide having a hydrogenase activity, such as an alcohol dehydrogenase activity, a DEHU hydrogenase activity, and/or a D-mannuronate hydrogenase activity.

Additional embodiments include methods for catalyzing the reduction (hydrogenation) of D-mannuronate, comprising contacting D-mannuronate with a microbial system, wherein the microbial system comprises a microorganism, and wherein the microorganism comprises a polynucleotide according to the present disclosure.

Additional embodiments include methods for catalyzing the reduction (hydrogenation) of DEHU, comprising contacting DEHU with a microbial system, wherein the microbial system comprises a microorganism, and wherein the microorganism comprises a polynucleotide according to the present disclosure.

Additional embodiments include vectors comprising an isolated polynucleotide or the present disclosure, and may further include such a vector wherein the isolated polynucleotide is operably linked to an expression control region, and wherein the polynucleotide encodes a polypeptide having a hydrogenase activity, such as an alcohol dehydrogenase activity, a DEHU hydrogenase activity, and/or a D-mannuronate hydrogenase activity.

Additional embodiments include a recombinant microorganism, or microbial system that comprises a recombinant microorganism, wherein the recombinant microorganism comprises a polynucleotide or polypeptide as described herein. In certain embodiments, the recombinant microorganism is selected from *Acetobacter aceti, Achromobacter, Acidiphilium, Acinetobacter, Actinomadura, Actinoplanes, Aeropyrum pernix, Agrobacterium, Alcaligenes, Ananas comosus (M), Arthrobacter, Aspargillus niger, Aspargillus oryze, Aspergillus melleus, Aspergillus pulverulentus, Aspergillus saitoi, Aspergillus sojea, Aspergillus usamii, Bacillus alcalophilus, Bacillus amyloliquefaciens, Bacillus brevis, Bacillus circulans, Bacillus clausii, Bacillus lentus, Bacillus licheniformis, Bacillus macerans, Bacillus stearothermophilus, Bacillus subtilis, Bifidobacterium, Brevibacillus brevis, Burkholderia cepacia, Candida cylindracea, Candida rugosa, Carica papaya (L), Cellulosimicrobium, Cephalosporium, Chaetomium erraticum, Chaetomium gracile, Clostridium, Clostridium butyricum, Clostridium acetobutylicum, Clostridium thermocellum, Corynebacterium (glutamicum), Corynebacterium efficiens, Escherichia coli, Enterococcus, Erwina chrysanthemi, Gliconobacter, Gluconacetobacter, Haloarcula, Humicola insolens, Humicola nsolens, Kitasatospora setae, Klebsiella, Klebsiella oxytoca, Kluyveromyces, Kluyveromyces fragilis, Kluyveromyces lactis, Kocuria, Lactlactis, Lactobacillus, Lactobacillus fermentum, Lactobacillus sake, Lactococcus, Lactococcus lactis, Leuconostoc, Methylocystis, Methanolobus siciliae, Methanogenium organophilum,*

*Methanobacterium bryantii, Microbacterium imperiale, Micrococcus lysodeikticus, Microlunatus, Mucor javanicus, Mycobacterium, Myrothecium, Nitrobacter, Nitrosomonas, Nocardia, Papaya carica, Pediococcus, Pediococcus halophilus, Penicillium, Penicillium camemberti, Penicillium citrinum, Penicillium emersonii, Penicillium roqueforti, Penicillum lilactinum, Penicillum multicolor, Paracoccus pantotrophus, Propionibacterium, Pseudomonas, Pseudomonas fluorescens, Pseudomonas denitrificans, Pyrococcus, Pyrococcus furiosus, Pyrococcus horikoshii, Rhizobium, Rhizomucor miehei, Rhizomucor pusillus Lindt, Rhizopus, Rhizopus delemar, Rhizopus japonicus, Rhizopus niveus, Rhizopus oryzae, Rhizopus oligosporus, Rhodococcus, Saccharomyces cerevisiae, Sclerotina libertina, Sphingobacterium multivorum, Sphingobium, Sphingomonas, Streptococcus, Streptococcus thermophilus Y-1, Streptomyces, Streptomyces griseus, Streptomyces lividans, Streptomyces murinus, Streptomyces rubiginosus, Streptomyces violaceoruber, Streptoverticillium mobaraense, Tetragenococcus, Thermus, Thiosphaera pantotropha, Trametes, Trichoderma, Trichoderma longibrachiatum, Trichoderma reesei, Trichoderma viride, Trichosporon penicillatum, Vibrio alginolyticus, Xanthomonas, yeast, Zygosaccharomyces rouxii, Zymomonas,* and *Zymomonus mobilis.*

Additional embodiments include isolated polypeptides, and variants or fragments thereof, selected from

(a) an isolated polypeptide comprising an amino acid sequence at least 80% identical to the amino acid sequence set forth in SEQ ID NO:2, 4, 6, 8, 10, 12, 14, 16, 18, 20, 22, 24, 26, 28, 30, 32, 34, 36, 38, or 78;
(b) an isolated polypeptide comprising an amino acid sequence at least 90% identical to the amino acid sequence set forth in SEQ ID NO:2, 4, 6, 8, 10, 12, 14, 16, 18, 20, 22, 24, 26, 28, 30, 32, 34, 36, 38, or 78;
(c) an isolated polypeptide comprising an amino acid sequence at least 95% identical to the amino acid sequence set forth in SEQ ID NO:2, 4, 6, 8, 10, 12, 14, 16, 18, 20, 22, 24, 26, 28, 30, 32, 34, 36, 38, or 78;
(d) an isolated polypeptide comprising an amino acid sequence at least 97% identical to the amino acid sequence set forth in SEQ ID NO:2, 4, 6, 8, 10, 12, 14, 16, 18, 20, 22, 24, 26, 28, 30, 32, 34, 36, 38, or 78;

(e) an isolated polypeptide comprising an amino acid sequence at least 99% identical to the amino acid sequence set forth in SEQ ID NO:2, 4, 6, 8, 10, 12, 14, 16, 18, 20, 22, 24, 26, 28, 30, 32, 34, 36, 38, or 78; and

(e) an isolated polypeptide comprising the amino acid sequence set forth in SEQ ID NO:2, 4, 6, 8, 10, 12, 14, 16, 18, 20, 22, 24, 26, 28, 30, 32, 34, 36, 38, or 78,

wherein the isolated polypeptide has a hydrogenase activity, such as an alcohol dehydrogenase activity, a DEHU hydrogenase activity, and/or a D-mannuronate hydrogenase activity.

Additional embodiments include methods for converting a polysaccharide to a suitable monosaccharide or oligosaccharide, comprising contacting the polysaccharide with a recombinant microorganism, wherein the recombinant microorganism comprises an ADH polynucleotide or polypeptide according to the present disclosure.

Additional embodiments include methods for catalyzing the reduction (hydrogenation) of D-mannuronate, comprising contacting D-mannuronate with a recombinant microorganism, wherein the recombinant microorganism comprises an ADH polynucleotide or polypeptide according to the present disclosure.

Additional embodiments include methods for catalyzing the reduction (hydrogenation) of uronate, 4-deoxy-L-erythro-5-hexoseulose uronate (DEHU), comprising contacting DEHU with a recombinant microorganism, wherein the recombinant microorganism comprises an ADH polynucleotide or polypeptide according to the present disclosure.

Additional embodiments include microbial systems for converting a polysaccharide to a suitable monosaccharide or oligosaccharide, wherein the microbial system comprises a recombinant microorganism, and wherein the recombinant microorganism comprises an isolated polynucleotide selected from

(a) an isolated polynucleotide comprising a nucleotide sequence at least 80% identical to the nucleotide sequence set forth in SEQ ID NO:1, 3, 5, 7, 9, 11, 13, 15, 17, 19, 21, 23, 25, 27, 29, 31, 33, 35, or 37;

(b) an isolated polynucleotide comprising a nucleotide sequence at least 90% identical to the nucleotide sequence set forth in SEQ ID NO:1, 3, 5, 7, 9, 11, 13, 15, 17, 19,21, 23, 25, 27, 29, 31, 33, 35, or 37;

(c) an isolated polynucleotide comprising a nucleotide sequence at least 95% identical to the nucleotide sequence set forth in SEQ ID NO:1, 3, 5, 7, 9, 11, 13, 15, 17, 19, 21, 23, 25, 27, 29, 31, 33, 35, or 37;

(d) an isolated polynucleotide comprising a nucleotide sequence at least 97% identical to the nucleotide sequence set forth in SEQ ID NO:1, 3, 5, 7, 9, 11, 13, 15, 17, 19, 21, 23, 25, 27, 29, 31, 33, 35, or 37;

(e) an isolated polynucleotide comprising a nucleotide sequence at least 99% identical to the nucleotide sequence set forth in SEQ ID NO:1, 3, 5, 7, 9, 11, 13, 15, 17, 19, 21, 23, 25, 27, 29, 31, 33, 35, or 37; and

(f) an isolated polynucleotide comprising the nucleotide sequence set forth in SEQ ID NO:1, 3, 5, 7, 9, 11, 13, 15, 17, 19, 21, 23, 25, 27, 29, 31, 33, 35, or 37.

Additional embodiments include microbial systems for converting a polysaccharide to a suitable monosaccharide or oligosaccharide, wherein the microbial system comprises a recombinant microorganism, and wherein the recombinant microorganism comprises an isolated polypeptide selected from

(a) an isolated polypeptide comprising an amino acid sequence at least 80% identical to the amino acid sequence set forth in SEQ ID NO:2, 4, 6, 8, 10, 12, 14, 16, 18, 20, 22, 24, 26, 28, 30, 32, 34, 36, 38, or 78;

(b) an isolated polypeptide comprising an amino acid sequence at least 90% identical to the amino acid sequence set forth in SEQ ID NO:2, 4, 6, 8, 10, 12, 14, 16, 18, 20, 22, 24, 26, 28, 30, 32, 34, 36, 38, or 78;

(c) an isolated polypeptide comprising an amino acid sequence at least 95% identical to the amino acid sequence set forth in SEQ ID NO:2, 4, 6, 8, 10, 12, 14, 16, 18, 20, 22, 24, 26, 28, 30, 32, 34, 36, 38, or 78;

(d) an isolated polypeptide comprising an amino acid sequence at least 97% identical to the amino acid sequence set forth in SEQ ID NO:2, 4, 6, 8, 10, 12, 14, 16, 18, 20, 22, 24, 26, 28, 30, 32, 34, 36, 38, or 78;

(e) an isolated polypeptide comprising an amino acid sequence at least 99% identical to the amino acid sequence set forth in SEQ ID NO:2, 4, 6, 8, 10, 12, 14, 16, 18, 20, 22, 24, 26, 28, 30, 32, 34, 36, 38, or 78; and

(e) an isolated polypeptide comprising the amino acid sequence set forth in SEQ ID NO:2, 4, 6, 8, 10, 12, 14, 16, 18, 20, 22, 24, 26, 28, 30, 32, 34, 36, 38, or 78.

In additional embodiments, an isolated polynucleotide as disclosed herein may encode a polypeptide that comprises at least one of a nicotinamide adenine dinucleotide (NAD+), NADH, nicotinamide adenine dinucleotide phosphate (NADP+), or NADPH binding motif. Other embodiments may include an isolated ADH polypeptide, or a fragment, variant, or derivative thereof, wherein the polypeptide comprises at least one of a nicotinamide adenine dinucleotide (NAD+), NADH, nicotinamide adenine dinucleotide phosphate (NADP+), or NADPH binding motif. In certain embodiments, the NAD+, NADH, NADP+, or NADPH binding motif is selected from the group consisting of Y-X-G-G-X-Y (SEQ ID NO:67), Y-X-X-G-G-X-Y (SEQ ID NO:68), Y-X-X-X-G-G-X-Y (SEQ ID NO:69), Y-X-G-X-X-Y (SEQ ID NO:70), Y-X-X-G-G-X-X-Y (SEQ ID NO:71), Y-X-X-X-G-X-X-Y (SEQ ID NO:72), Y-X-G-X-Y (SEQ ID NO:73), Y-X-X-G-X-Y (SEQ ID NO:74), Y-X-X-X-G-X-Y (SEQ ID NO:75), and Y-X-X-X-X-G-X-Y (SEQ ID NO:76); wherein Y is independently selected from alanine, glycine, and serine, wherein G is glycine, and wherein X is independently selected from a genetically encoded amino acid.

Certain embodiments relate to methods for converting a polysaccharide to ethanol, comprising contacting the polysaccharide with a recombinant microorganism, wherein the recombinant microorganism is capable of growing on the polysaccharide as a sole source of carbon. In certain embodiments, the recombinant microorganism comprises at least one polynucleotide encoding at least one pyruvate decarboxylase, and at least one polynucleotide encoding an alcohol dehydrogenase. In certain embodiments, the polysaccharide is alginate. In certain embodiments, the recombinant microorganism comprises one or more polynucleotides that contain a genomic region between V12B01_24189 and V12B01_24249 of *Vibro splendidus.* In certain embodiments, the at least one pyruvate decarboxylase is derived from *Zymomonas mobilis.* In certain embodiments, the at least one alcohol dehydrogenase is derived from *Zymomonas mobilis.* In certain embodiments, the recombinant microorganism is E. *coli.*

BRIEF DESCRIPTION OF THE DRAWINGS

Figure 1 shows the NADPH consumption of the isolated alcohol dehydrogenase (ADH) enzymes using DEHU as a substrate, as performed according to Example 2.

Figure 2 shows the NADPH consumption of the isolated ADH enzymes using D-mannuronate as a substrate, as performed in Example 2.

Figure 3 shows the nucleotide (SEQ ID NO:1) and amino acid (SEQ ID NO:2) sequences of ADH1.

Figure 4 shows the nucleotide (SEQ ID NO:3) and amino acid (SEQ ID NO:4) sequences of ADH2.

Figure 5 shows the nucleotide (SEQ ID NO:5) and amino acid (SEQ ID NO:6) sequences of ADH3.

Figure 6 shows the nucleotide (SEQ ID NO:7) and amino acid (SEQ ID NO:8) sequences of ADH4.

Figure 7 shows the nucleotide (SEQ ID NO:9) and amino acid (SEQ ID NO:10) sequences of ADH5.

Figure 8 shows the nucleotide (SEQ ID NO:11) and amino acid (SEQ ID NO:12) sequences of ADH6.

Figure 9 shows the nucleotide (SEQ ID NO:13) and amino acid (SEQ ID NO:14) sequences of ADH7.

Figure 10 shows the nucleotide (SEQ ID NO:15) and amino acid (SEQ ID NO:16) sequences of ADH8.

Figure 11 shows the nucleotide (SEQ ID NO:17) and amino acid (SEQ ID NO:18) sequences of ADH9.

Figure 12 shows the nucleotide (SEQ ID NO:19) and amino acid (SEQ ID NO:20) sequences of ADH10.

Figure 13 shows the nucleotide (SEQ ID NO:21) and amino acid (SEQ ID NO:22) sequences of ADH11.

Figure 14 shows the nucleotide (SEQ ID NO:23) and amino acid (SEQ ID NO:24) sequences of ADH12.

Figure 15 shows the nucleotide (SEQ ID NO:25) and amino acid (SEQ ID NO:26) sequences of ADH13.

Figure 16 shows the nucleotide (SEQ ID NO:27) and amino acid (SEQ ID NO:28) sequences of ADH14.

Figure 17 shows the nucleotide (SEQ ID NO:29) and amino acid (SEQ ID NO:30) sequences of ADH15.

Figure 18 shows the nucleotide (SEQ ID NO:31) and amino acid (SEQ ID NO:32) sequences of ADH16.

Figure 19 shows the nucleotide (SEQ ID NO:33) and amino acid (SEQ ID NO:34) sequences of ADH17.

Figure 20 shows the nucleotide (SEQ ID NO:35) and amino acid (SEQ ID NO:36) sequences of ADH18.

Figure 21 shows the nucleotide (SEQ ID NO:37) and amino acid (SEQ ID NO:38) sequences of ADH19.

Figure 22 shows shows the results of engineered or recombinant *E. coli* growing on alginate as a sole source of carbon (*see* solid circles), as described in Example 3. *Agrobacterium tumefaciens* cells provide a positive control (*see* hatched circles). The well to the immediate left of the of the *A. tumefaciens* positive control contains DH10B *E. coli* cells, which provide a negative control.

Figure 23 shows the production of alcohol by *E. coli* growing on alginate as a sole source of carbon, as described in Example 4. *E. coli* was transformed with either pBBRPdc-AdhA/B or pBBRPdc-AdhA/B +1.5 FOS and allowed to grow in m9 media containing alginate.

Figure 24 shows the DEHU hydrogenase activity of ADH11 and ADH20. ADH20 is a putative tartronate semialdehyde reductase (TSAR) gene isolated from *Vibrio splendidus* 12B01 (*see* SEQ ID NO:78 for amino acid sequence), and which demonstrates significant DEHU hydrogenation activity, especially with NADH.

<u>DETAILED DESCRIPTION</u>

***Definitions***

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much 30, 25, 20, 25, 10, 9, 8, 7, 6, 5, 4, 3, 2 or 1 % to a reference quantity, level, value, number, frequency, percentage, dimension, size, amount, weight or length.

Examples of "biomass" include aquatic or marine biomass, fruit-based biomass such as fruit waste, and vegetable-based biomass such as vegetable waste, among others. Examples of aquatic or marine biomass include, but are not limited to, kelp, giant kelp, seaweed, algae, and marine microflora, microalgae, sea grass, and the like. In certain aspects, biomass does not include fossilized sources of carbon, such as hydrocarbons that are typically found within the top layer of the Earth's crust (e.g., natural gas, nonvolatile materials composed of almost pure carbon, like anthracite coal, etc).

Examples of "aquatic biomass" or "marine biomass" include, but are not limited to, kelp, giant kelp, sargasso, seaweed, algae, marine microflora, microalgae, and sea grass, and the like.

Examples of fruit and/or vegetable biomass include, but are not limited to, any source of pectin such as plant peel and pomace including citrus, orange, grapefruit, potato, tomato, grape, mango, gooseberry, carrot, sugar-beet, and apple, among others.

Examples of polysaccharides, oligosaccharides, monosaccharides or other sugar components of biomass include, but are not limited to, alginate, agar, carrageenan, fucoidan, pectin, gluronate, mannuronate, mannitol, lyxose, cellulose, hemicellulose, glycerol, xylitol, glucose, mannose, galactose, xylose, xylan, mannan, arabinan, arabinose, glucuronate, galacturonate (including di- and tri-galacturonates), rhamnose, and the like.

Certain examples of alginate-derived polysaccharides include saturated polysaccharides, such as β-D-mannuronate, α-L-gluronate, dialginate, trialginate, pentalginate, hexalginate, heptalginate, octalginate, nonalginate, decalginate, undecalginate, dodecalginate and polyalginate, as well as unsaturated polysaccharides such as 4-deoxy-L-erythro-5-hexoseulose uronic acid, 4-(4-deoxy-beta-D-mann-4-enuronosyl)-D-mannuronate or L-guluronate, 4-(4-deoxy-beta-D-mann-4-enuronosyl)-dialginate, 4-(4-deoxy-beta-D-mann-4-enuronosyl)-trialginate, 4-(4-deoxy-beta-D-mann-4-enuronosyl)-tetralginate, 4-(4-deoxy-beta-D-mann-4-enuronosyl)-pentalginate, 4-(4-deoxy-beta-D-mann-4-enuronosyl)-hexalginate, 4-(4-deoxy-beta-D-mann-4-enuronosyl)-heptalginate, 4-(4-deoxy-beta-D-mann-4-enuronosyl)-octalginate, 4-(4-deoxy-beta-D-mann-4-enuronosyl)-nonalginate, 4-(4-deoxy-beta-D-mann-4-enuronosyl)-undecalginate, and 4-(4-deoxy-beta-D-mann-4-enuronosyl)-dodecalginate.

Certain examples of pectin-derived polysaccharides include saturated polysaccharides, such as galacturonate, digalacturonate, trigalacturonate, tetragalacturonate, pentagalacturonate, hexagalacturonate, heptagalacturonate, octagalacturonate, nonagalacturonate, decagalacturonate, dodecagalacturonate, polygalacturonate, and rhamnopolygalacturonate, as well as saturated polysaccharides such as 4-deoxy-L-threo-5-hexosulose uronate, 4-(4-Deoxy-alpha-D-gluc-4-enuronosyl)-D-galacturonate, 4-(4-Deoxy-alpha-D-gluc-4-enuronosyl)-D-digalacturonate, 4-(4-Deoxy-alpha-D-gluc-4-enuronosyl)-D-trigalacturonate, 4-(4-Deoxy-alpha-D-gluc-4-enuronosyl)-D-tetragalacturonate, 4-(4-Deoxy-alpha-D-gluc-4-enuronosyl)-D-pentagalacturonate, 4-(4-Deoxy-alpha-D-gluc-4-enuronosyl)-D-hexagalacturonate, 4-(4-Deoxy-alpha-D-gluc-4-enuronosyl)-D-heptagalacturonate, 4-(4-Deoxy-alpha-D-gluc-4-enuronosyl)-D-octagalacturonate, 4-(4-Deoxy-alpha-D-gluc-4-enuronosyl)-D-nonagalacturonate, 4-(4-Deoxy-alpha-D-gluc-4-enuronosyl)-D-decagalacturonate, and 4-(4-Deoxy-alpha-D-gluc-4-enuronosyl)-D-dodecagalacturonate.

These polysaccharide or oligosaccharide components may be converted into "suitable monosaccharides" or other "suitable saccharides," such as "suitable oligosaccharides," by the microorganisms described herein which are capable of growing on such polysaccharides or other sugar components as a source of carbon (e.g., a sole source of carbon).

A "monosaccharide," "suitable monosaccharide" or "suitable saccharide" refers generally to any saccharide that may be produced by a recombinant microorganism growing on pectin, alginate, or other saccharide (e.g., galacturonate, cellulose, hemi-cellulose etc.) as a source or sole source of carbon, and also refers generally to any saccharide that may be utilized in a biofuel biosynthesis pathway of the present invention to produce hydrocarbons such as biofuels or biopetrols. Examples of suitable monosaccharides or oligosaccharides include, but are not limited to, 2-keto-3-deoxy D-gluconate (KDG), D-mannitol, gluronate, mannuronate, mannitol, lyxose, glycerol, xylitol, glucose, mannose, galactose, xylose, arabinose, glucuronate, galacturonates, and rhamnose, and the like. As noted herein, a "suitable monosaccharide" or "suitable saccharide" as used herein may be produced by an engineered or recombinant microorganism of the present invention, or may be obtained from commercially available sources.

The recitation "commodity chemical" as used herein includes any saleable or marketable chemical that can be produced either directly or as a byproduct of the methods provided herein, including biofuels and/or biopetrols. General examples of "commodity chemicals" include, but are not limited to, biofuels, minerals, polymer precursors, fatty alcohols, surfactants, plasticizers, and solvents. The recitation "biofuels" as used herein includes solid, liquid, or gas fuels derived, at least in part, from a biological source, such as a recombinant microorganism.

Examples of commodity chemicals include, but are not limited to, methane, methanol, ethane, ethene, ethanol, n-propane, 1-propene, 1-propanol, propanal, acetone, propionate, n-butane, 1-butene, 1-butanol, butanal, butanoate, isobutanal, isobutanol, 2-methylbutanal, 2-methylbutanol, 3-methylbutanal, 3-methylbutanol, 2-butene, 2-butanol, 2-butanone, 2,3-butanediol, 3-hydroxy-2-butanone, 2,3-butanedione, ethylbenzene, ethenylbenzene, 2-phenylethanol, phenylacetaldehyde, 1-phenylbutane, 4-phenyl-1-butene, 4-phenyl-2-butene, 1-phenyl-2-butene, 1-phenyl-2-butanol, 4-phenyl-2-butanol, 1-phenyl-2-butanone, 4-phenyl-2-butanone, 1-phenyl-2,3-butandiol, 1-phenyl-3-hydroxy-2-butanone, 4-phenyl-3-hydroxy-2-butanone, 1-phenyl-2,3-butanedione, n-pentane, ethylphenol, ethenylphenol, 2-(4-hydroxyphenyl) ethanol, , 4-hydroxyphenylacetaldehyde, 1-(4-hydroxyphenyl) butane, 4-(4-hydroxyphenyl)-1-butene, 4-(4-hydroxyphenyl)-2-butene, 1-(4-hydroxyphenyl)-1-butene, 1-(4-hydroxyphenyl)-2-butanol, 4-(4-hydroxyphenyl)-2-butanol, 1 -(4-hydroxyphenyl)-2-butanone, 4-(4-hydroxyphenyl)-2-butanone, 1-(4-hydroxyphenyl)-2,3-butandiol, 1-(4-hydroxyphenyl)-3-hydroxy-2-butanone, 4-(4-hydroxyphenyl)-3-hydroxy-2-butanone, 1-(4-hydroxyphenyl)-2,3-butanonedione, indolylethane, indolylethene, 2-(indole-3-)ethanol, n-pentane,1-pentene, 1-pentanol, pentanal, pentanoate, 2-pentene, 2-pentanol, 3-pentanol, 2-pentanone, 3-pentanone, 4-methylpentanal, 4-methylpentanol, 2,3-pentanediol, 2-hydroxy-3-pentanone, 3-hydroxy-2-pentanone, 2,3-pentanedione, 2-methylpentane, 4-methyl-1-pentene, 4-methyl-2-pentene, 4-methyl-3-pentene, 4-methyl-2-pentanol, 2-methyl-3-pentanol, 4-methyl-2-pentanone, 2-methyl-3-pentanone, 4-methyl-2,3-pentanediol, 4-methyl-2-hydroxy-3-pentanone, 4-methyl-3-hydroxy-2-pentanone, 4-methyl-2,3-pentanedione, 1-phenylpentane, 1-phenyl-1-pentene, 1-phenyl-2-pentene, 1-phenyl-3-pentene, 1-phenyl-2-pentanol, 1-phenyl-3-pentanol, 1-phenyl-2-pentanone, 1-phenyl-3-pentanone, 1-phenyl-2,3-pentanediol, 1-phenyl-2-hydroxy-3-pentanone, 1-phenyl-3-hydroxy-2-pentanone, 1-phenyl-2,3-pentanedione, 4-methyl-1-phenylpentane, 4-methyl-1-phenyl-1-pentene, 4-methyl-1-phenyl-2-pentene, 4-methyl-1-phenyl-3-pentene, 4-methyl-1-phenyl-3-pentanol, 4-methyl-1-phenyl-2-pentanol, 4-methyl-1-phenyl-3-pentanone, 4-methyl-1-phenyl-2-pentanone, 4-methyl-1-phenyl-2,3-pentanediol, 4-methyl-1-phenyl-2,3-pentanedione , 4-methyl-1-phenyl-3-hydroxy-2-pentanone, 4-methyl-1-phenyl-2-hydroxy-3-pentanone, 1-(4-hydroxyphenyl) pentane, 1-(4-hydroxyphenyl)-1-pentene, 1-(4-hydroxyphenyl)-2-pentene, 1-(4-hydroxyphenyl)-3-pentene, 1-(4-hydroxyphenyl)-2-pentanol, 1-(4-hydroxyphenyl)-3-pentanol, 1-(4-hydroxyphenyl)-2-pentanone, 1-(4-hydroxyphenyl)-3-pentanone, 1-(4-hydroxyphenyl)-2,3-pentanediol, 1-(4-hydroxyphenyl)-2-hydroxy-3-pentanone, 1-(4-hydroxyphenyl)-3-hydroxy-2-pentanone, 1-(4-hydroxyphenyl)-2,3-pentanedione, 4-methyl-1-(4-hydroxyphenyl) pentane, 4-methyl-1-(4-hydroxyphenyl)-2-pentene, 4-methyl-1-(4-hydroxyphenyl)-3-pentene, 4-methyl-1-(4-hydroxyphenyl)-1-pentene, 4-methyl-1-(4-hydroxyphenyl)-3-pentanol, 4-methyl-1-(4-hydroxyphenyl)-2-pentanol, 4-methyl-1-(4-hydroxyphenyl)-3-pentanone, 4-methyl-1-(4-hydroxyphenyl)-2-pentanone, 4-methyl-1-(4-hydroxyphenyl)-2,3-pentanediol, 4-methyl-1-(4-hydroxyphenyl)-2,3-pentanedione , 4-methyl-1-(4-hydroxyphenyl)-3-hydroxy-2-pentanone, 4-methyl-1-(4-hydroxyphenyl)-2-hydroxy-3-pentanone, 1-indole-3-pentane, 1-(indole-3)-1-pentene, 1-(indole-3)-2-pentene, 1-(indole-3)-3-pentene, 1-(indole-3)-2-pentanol, 1-(indole-3)-3-pentanol, 1-(indole-3)-2-pentanone, 1-(indole-3)-3-pentanone, 1-(indole-3)-2,3-pentanediol, 1-(indole-3)-2-hydroxy-3-pentanone, 1-(indole-3)-3-hydroxy-2-pentanone, 1-(indole-3)-2,3-pentanedione, 4-methyl-1-(indole-3-)pentane, 4-methyl-1-(indole-3)-2-pentene, 4-methyl-1-(indole-3)-3-pentene, 4-methyl-1-(indole-3)-1-pentene, 4-methyl-2-(indole-3)-3-pentanol, 4-methyl-1-(indole-3)-2-pentanol, 4-methyl-1-(indole-3)-3-pentanone, 4-methyl-1-(indole-3)-2-pentanone, 4-methyl-1-(indole-3)-2,3-pentanediol, 4-methyl-1-(indole-3)-2,3-pentanedione, 4-methyl-1-(indole-3)-3-hydroxy-2-pentanone, 4-methyl-1-(indole-3)-2-hydroxy-3-pentanone, n-hexane, 1-hexene, 1-hexanol, hexanal, hexanoate, 2-hexene, 3-hexene, 2-hexanol, 3-hexanol, 2-hexanone, 3-hexanone, 2,3-hexanediol, 2,3-hexanedione, 3,4-hexanediol, 3,4-hexanedione, 2-hydroxy-3-hexanone, 3-hydroxy-2-hexanone, 3-hydroxy-4-hexanone, 4-hydroxy-3-hexanone, 2-methylhexane, 3-methylhexane, 2-methyl-2-hexene, 2-methyl-3-hexene, 5-methyl-1-hexene, 5-methyl-2-hexene, 4-methyl-1-hexene, 4-methyl-2-hexene, 3-methyl-3-hexene, 3-methyl-2-hexene, 3-methyl-1-hexene, 2-methyl-3-hexanol, 5-methyl-2-hexanol, 5-methyl-3-hexanol, 2-methyl-3-hexanone, 5-methyl-2-hexanone, 5-methyl-3-hexanone, 2-methyl-3,4-hexanediol, 2-methyl-3,4-hexanedione , 5-methyl-2,3-hexanediol, 5-methyl-2,3-hexanedione, 4-methyl-2,3-hexanediol, 4-methyl-2,3-hexanedione, 2-methyl-3-hydroxy-4-hexanone, 2-methyl-4-hydroxy-3-hexanone, 5-methyl-2-hydroxy-3-hexanone, 5-methyl-3-hydroxy-2-hexanone, 4-methyl-2-hydroxy-3-hexanone, 4-methyl-3-hydroxy-2-hexanone, 2,5-dimethylhexane, 2,5-dimethyl-2-hexene, 2,5-dimethyl-3-hexene, 2,5-dimethyl-3-hexanol, 2,5-dimethyl-3-hexanone, 2,5-dimethyl-3,4-hexanediol, 2,5-dimethyl-3,4-hexanedione, 2,5-dimethyl-3-hydroxy-4-hexanone, 5-methyl-l-phenylhexane, 4-methyl-1-phenylhexane, 5-methyl-1-phenyl-1-hexene, 5-methyl-1-phenyl-2-hexene, 5-methyl-1-phenyl-3-hexene, 4-methyl-1-phenyl-1-hexene, 4-methyl-1-phenyl-2-hexene, 4-methyl-1-phenyl-3-hexene, 5-methyl-1-phenyl-2-hexanol, 5-methyl-1-phenyl-3-hexanol, 4-methyl-1-phenyl-2-hexanol, 4-methyl-1-phenyl-3-hexanol, 5-methyl-1-phenyl-2-hexanone, 5-methyl-1-phenyl-3-hexanone, 4-methyl-1-phenyl-2-hexanone, 4-methyl-1-phenyl-3-hexanone, 5-methyl-1-phenyl-2,3-hexanediol, 4-methyl-1-phenyl-2,3-hexanediol, 5-methyl-1-phenyl-3-hydroxy-2-hexanone, 5-methyl-1-phenyl-2-hydroxy-3-hexanone, 4-methyl-1-phenyl-3-hydroxy-2-hexanone, 4-methyl-1-phenyl-2-hydroxy-3-hexanone, 5-methyl-1-phenyl-2,3-hexanedione, 4-methyl-1-phenyl-2,3-hexanedione, 4-methyl-1-(4-hydroxyphenyl)hexane, 5-methyl-1-(4-hydroxyphenyl)-1-hexene, 5-methyl-1-(4-hydroxyphenyl)-2-hexene, 5-methyl-1-(4-hydroxyphenyl)-3-hexene, 4-methyl-1-(4-hydroxyphenyl)-1-hexene, 4-methyl-1-(4-hydroxyphenyl)-2-hexene, 4-methyl-1-(4-hydroxyphenyl)-3-hexene, 5-methyl-1-(4-hydroxyphenyl)-2-hexanol, 5-methyl-1-(4-hydroxyphenyl)-3-hexanol, 4-methyl-1-(4-hydroxyphenyl)-2-hexanol, 4-methyl-1-(4-hydroxyphenyl)-3-hexanol, 5-methyl-1-(4-hydroxyphenyl)-2-hexanone, 5-methyl-1-(4-hydroxyphenyl)-3-hexanone, 4-methyl-1-(4-hydroxyphenyl)-2-hexanone, 4-methyl-1-(4-hydroxyphenyl)-3-hexanone, 5-methyl-1-(4-hydroxyphenyl)-2,3-hexanediol, 4-methyl-1-(4-hydroxyphenyl)-2,3-hexanediol, 5-methyl-1-(4-hydroxyphenyl)-3-hydroxy-2-hexanone, 5-methyl-1-(4-hydroxyphenyl)-2-hydroxy-3-hexanone, 4-methyl-1-(4-hydroxyphenyl)-3-hydroxy-2-hexanone, 4-methyl-1-(4-hydroxyphenyl)-2-hydroxy-3-hexanone, 5-methyl-1-(4-hydroxyphenyl)-2,3-hexanedione, 4-methyl-1-(4-hydroxyphenyl)-2,3-hexanedione, 4-methyl-1-(indole-3-)hexane, 5-methyl-1-(indole-3)-1-hexene, 5-methyl-1-(indole-3)-2-hexene, 5-methyl-1-(indole-3)-3-hexene, 4-methyl-1-(indole-3)-1-hexene, 4-methyl-1-(indole-3)-2-hexene, 4-methyl-1-(indole-3)-3-hexene, 5-methyl-1-(indole-3)-2-hexanol, 5-methyl-1-(indole-3)-3-hexanol, 4-methyl-1-(indole-3)-2-hexanol, 4-methyl-1-(indole-3)-3-hexanol, 5-methyl-1-(indole-3)-2-hexanone, 5-methyl-1-(indole-3)-3-hexanone, 4-methyl-1-(indole-3)-2-hexanone, 4-methyl-1-(indole-3)-3-hexanone, 5-methyl-1-(indole-3)-2,3-hexanediol, 4-methyl-1-(indole-3)-2,3-hexanediol, 5-methyl-1-(indole-3)-3-hydroxy-2-hexanone, 5-methyl-1-(indole-3)-2-hydroxy-3-hexanone, 4-methyl-1-(indole-3)-3-hydroxy-2-hexanone, 4-methyl-1-(indole-3)-2-hydroxy-3-hexanone, 5-methyl-1-(indole-3)-2,3-hexanedione, 4-methyl-1-(indole-3)-2,3-hexanedione, n-heptane, 1-heptene, 1-heptanol, heptanal, heptanoate, 2-heptene, 3-heptene, 2-heptanol, 3-heptanol, 4-heptanol, 2-heptanone, 3-heptanone, 4-heptanone, 2,3-heptanediol, 2,3-heptanedione, 3,4-heptanediol, 3,4-heptanedione, 2-hydroxy-3-heptanone, 3-hydroxy-2-heptanone, 3-hydroxy-4-heptanone, 4-hydroxy-3-heptanone, 2-methylheptane, 3-methylheptane, 6-methyl-2-heptene, 6-methyl-3-heptene, 2-methyl-3-heptene, 2-methyl-2-heptene, 5-methyl-2-heptene, 5-methyl-3-heptene, 3-methyl-3-heptene, 2-methyl-3-heptanol, 2-methyl-4-heptanol, 6-methyl-3-heptanol, 5-methyl-3-heptanol, 3-methyl-4-heptanol, 2-methyl-3-heptanone, 2-methyl-4-heptanone, 6-methyl-3-heptanone, 5-methyl-3-heptanone, 3-methyl-4-heptanone, 2-methyl-3,4-heptanediol, 2-methyl-3,4-heptanedione, 6-methyl-3,4-heptanediol, 6-methyl-3,4-heptanedione, 5-methyl-3,4-heptanediol, 5-methyl-3,4-heptanedione, 2-methyl-3-hydroxy-4-heptanone, 2-methyl-4-hydroxy-3-heptanone, 6-methyl-3-hydroxy-4-heptanone, 6-methyl-4-hydroxy-3-heptanone, 5-methyl-3-hydroxy-4-heptanone, 5-methyl-4-hydroxy-3-heptanone, 2,6-dimethylheptane, 2,5-dimethylheptane, 2,6-dimethyl-2-heptene, 2,6-dimethyl-3-heptene, 2,5-dimethyl-2-heptene, 2,5-dimethyl-3-heptene, 3,6-dimethyl-3-heptene, 2,6-dimethyl-3-heptanol, 2,6-dimethyl-4-heptanol, 2,5-dimethyl-3-heptanol, 2,5-dimethyl-4-heptanol, 2,6-dimethyl-3,4-heptanediol, 2,6-dimethyl-3,4-heptanedione, 2,5-dimethyl-3,4-heptanediol, 2,5-dimethyl-3,4-heptanedione, 2,6-dimethyl-3-hydroxy-4-heptanone, 2,6-dimethyl-4-hydroxy-3-heptanone, 2,5-dimethyl-3-hydroxy-4-heptanone, 2,5-dimethyl-4-hydroxy-3-heptanone, n-octane, 1-octene, 2-octene, 1-octanol, octanal, octanoate, 3-octene, 4-octene, 4-octanol, 4-octanone, 4,5-octanediol, 4,5-octanedione, 4-hydroxy-5-octanone, 2-methyloctane, 2-methyl-3-octene, 2-methyl-4-octene, 7-methyl-3-octene, 3-methyl-3-octene, 3-methyl-4-octene, 6-methyl-3-octene, 2-methyl-4-octanol, 7-methyl-4-octanol, 3-methyl-4-octanol, 6-methyl-4-octanol, 2-methyl-4-octanone, 7-methyl-4-octanone, 3-methyl-4-octanone, 6-methyl-4-octanone, 2-methyl-4,5-octanediol, 2-methyl-4,5-octanedione, 3-methyl-4,5-octanediol, 3-methyl-4,5-octanedione, 2-methyl-4-hydroxy-5-octanone, 2-methyl-5-hydroxy-4-octanone, 3-methyl-4-hydroxy-5-octanone, 3-methyl-5-hydroxy-4-octanone, 2,7-dimethyloctane, 2,7-dimethyl-3-octene, 2,7-dimethyl-4-octene, 2,7-dimethyl-4-octanol, 2,7-dimethyl-4-octanone, 2,7-dimethyl-4,5-octanediol, 2,7-dimethyl-4,5-octanedione, 2,7-dimethyl-4-hydroxy-5-octanone, 2,6-dimethyloctane, 2,6-dimethyl-3-octene, 2,6-dimethyl-4-octene, 3,7-dimethyl-3-octene, 2,6-dimethyl-4-octanol, 3,7-dimethyl-4-octanol, 2,6-dimethyl-4-octanone, 3,7-dimethyl-4-octanone, 2,6-dimethyl-4,5-octanediol, 2,6-dimethyl-4,5-octanedione, 2,6-dimethyl-4-hydroxy-5-octanone, 2,6-dimethyl-5-hydroxy-4-octanone, 3,6-dimethyloctane, 3,6-dimethyl-3-octene, 3,6-dimethyl-4-octene, 3,6-dimethyl-4-octanol, 3,6-dimethyl-4-octanone, 3,6-dimethyl-4,5-octanediol, 3,6-dimethyl-4,5-octanedione, 3,6-dimethyl-4-hydroxy-5-octanone, n-nonane, 1-nonene, 1-nonanol, nonanal, nonanoate, 2-methylnonane, 2-methyl-4-nonene, 2-methyl-5-nonene, 8-methyl-4-nonene, 2-methyl-5-nonanol, 8-methyl-4-nonanol, 2-methyl-5-nonanone, 8-methyl-4-nonanone, 8-methyl-4,5-nonanediol, 8-methyl-4,5-nonanedione, 8-methyl-4-hydroxy-5-nonanone, 8-methyl-5-hydroxy-4-nonanone, 2,8-dimethylnonane, 2,8-dimethyl-3-nonene, 2,8-dimethyl-4-nonene, 2,8-dimethyl-5-nonene, 2,8-dimethyl-4-nonanol, 2,8-dimethyl-5-nonanol, 2,8-dimethyl-4-nonanone, 2,8-dimethyl-5-nonanone, 2,8-dimethyl-4,5-nonanediol, 2,8-dimethyl-4,5-nonanedione, 2,8-dimethyl-4-hydroxy-5-nonanone, 2,8-dimethyl-5-hydroxy-4-nonanone, 2,7-dimethylnonane, 3,8-dimethyl-3-nonene, 3,8-dimethyl-4-nonene, 3,8-dimethyl-5-nonene, 3,8-dimethyl-4-nonanol, 3,8-dimethyl-5-nonanol, 3,8-dimethyl-4-nonanone, 3,8-dimethyl-5-nonanone, 3,8-dimethyl-4,5-nonanediol, 3,8-dimethyl-4,5-nonanedione, 3,8-dimethyl-4-hydroxy-5-nonanone, 3,8-dimethyl-5-hydroxy-4-nonanone, n-decane, 1-decene, 1-decanol, decanoate, 2,9-dimethyldecane, 2,9-dimethyl-3-decene, 2,9-dimethyl-4-decene, 2,9-dimethyl-5-decanol, 2,9-dimethyl-5-decanone, 2,9-dimethyl-5,6-decanediol, 2,9-dimethyl-6-hydroxy-5-decanone, 2,9-dimethyl-5,6-decanedionen-undecane, 1-undecene, 1-undecanol, undecanal. undecanoate, n-dodecane, 1-dodecene, 1-dodecanol, dodecanal, dodecanoate, n-dodecane, 1-decadecene, 1-dodecanol, ddodecanal, dodecanoate, n-tridecane, 1-tridecene, 1-tridecanol, tridecanal, tridecanoate, n-tetradecane, 1-tetradecene, 1-tetradecanol, tetradecanal, tetradecanoate, n-pentadecane, 1-pentadecene, 1-pentadecanol, pentadecanal, pentadecanoate, n-hexadecane, 1-hexadecene, 1-hexadecanol, hexadecanal, hexadecanoate, n-heptadecane, 1-heptadecene, 1-heptadecanol, heptadecanal, heptadecanoate, n-octadecane, 1-octadecene, 1-octadecanol, octadecanal, octadecanoate, n-nonadecane, 1-nonadecene, 1-nonadecanol, nonadecanal, nonadecanoate, eicosane, 1-eicosene, 1-eicosanol, eicosanal, eicosanoate, 3-hydroxy propanal, 1, 3-propanediol, 4-hydroxybutanal, 1, 4-butanediol, 3-hydrxy-2-butanone, 2, 3-butandiol, 1,5-pentane diol, homocitrate, homoisocitorate, b-hydroxy adipate, glutarate, glutarsemialdehyde, glutaraldehyde, 2-hydroxy-1-cyclopentanone, 1,2-cyclopentanediol, cyclopentanone, cyclopentanol, (S)-2-acetolactate, (R)-2,3-Dihydroxy-isovalerate, 2-oxoisovalerate, isobutyryl-CoA, isobutyrate, isobutyraldehyde, 5-amino pentaldehyde, 1,10-diaminodecane, 1,10-diamino-5-decene, 1,10-diamino-5-hydroxydecane, 1,10-diamino-5-decanone, 1,10-diamino-5,6-decanediol, 1,10-diamino-6-hydroxy-S-decanone, phenylacetoaldehyde, 1,4-diphenylbutane, 1,4-diphenyl-1-butene, 1,4-diphenyl-2-butene, 1,4-diphenyl-2-butanol, 1,4-diphenyl-2-butanone, 1,4-diphenyl-2,3-butanediol, 1,4-diphenyl-3-hydroxy-2-butanone, 1-(4-hydeoxyphenyl)-4-phenylbutane, 1-(4-hydeoxyphenyl)-4-phenyl-1-butene, 1-(4-hydeoxyphenyl)-4-phenyl-2-butene, 1-(4-hydeoxyphenyl)-4-phenyl-2-butanol, 1-(4-hydeoxyphenyl)-4-phenyl-2-butanone, 1-(4-hydeoxyphenyl)-4-phenyl-2,3-butanediol, 1-(4-hydeoxyphenyl)-4-phenyl-3-hydroxy-2-butanone, 1-(indole-3)-4-phenylbutane, 1-(indole-3)-4-phenyl-1-butene, 1-(indole-3)-4-phenyl-2-butene, 1-(indole-3)-4-phenyl-2-butanol, 1-(indole-3)-4-phenyl-2-butanone, 1-(indole-3)-4-phenyl-2,3-butanediol, 1-(indole-3)-4-phenyl-3-hydroxy-2-butanone, 4-hydroxyphenylacetoaldehyde, 1,4-di(4-hydroxyphenyl)butane, 1,4-di(4-hydroxyphenyl)-1-butene, 1,4-di(4-hydroxyphenyl)-2-butene, 1,4-di(4-hydroxyphenyl)-2-butanol, 1,4-di(4-hydroxyphenyl)-2-butanone, 1,4-di(4-hydroxyphenyl)-2,3-butanediol, 1,4-di(4-hydroxyphenyl)-3-hydroxy-2-butanone, 1-(4-hydroxyphenyl)-4-(indole-3-)butane, 1-(4-hydroxyphenyl)-4-(indole-3)-1-butene, 1-di(4-hydroxyphenyl)-4-(indole-3)-2-butene, 1-(4-hydroxyphenyl)-4-(indole-3)-2-butanol, 1-(4-hydroxyphenyl)-4-(indole-3)-2-butanone, 1-(4-hydroxyphenyl)-4-(indole-3)-2,3-butanediol, 1-(4-hydroxyphenyl-4-(indole-3)-3-hydroxy-2-butanone, indole-3-acetoaldehyde, 1,4-di(indole-3-)butane, 1,4-di(indole-3)-1-butene, 1,4-di(indole-3)-2-butene, 1,4-di(indole-3)-2-butanol, 1,4-di(indole-3)-2-butanone, 1,4-di(indole-3)-2,3-butanediol, 1,4-di(indole-3)-3-hydroxy-2-butanone, succinate semialdehyde, hexane-1,8-dicarboxylic acid, 3-hexene-1,8-dicarboxylic acid, 3-hydroxy-hexane-1,8-dicarboxylic acid, 3-hexanone-1,8-dicarboxylic acid, 3,4-hexanediol-1,8-dicarboxylic acid, 4-hydroxy-3-hexanone-1,8-dicarboxylic acid, fucoidan, iodine, chlorophyll, carotenoid, calcium, magnesium, iron, sodium, potassium, phosphate, and the like.

The term "biologically active fragment", as applied to fragments of a reference or full-length polynucleotide or polypeptide sequence, refers to a fragment that has at least about 0.1, 0.5, 1, 2, 5, 10, 12, 14, 16, 18, 20, 22, 24, 26, 28, 30, 35, 40, 45, 50, 55, 60, 65, 70, 75, 80, 85, 90, 95, 96, 97, 98, 99% of the activity of a reference sequence. Included within the scope of the present invention are biologically active fragments of at least about 18, 19, 20, 21, 22, 23, 24, 25, 26, 27, 28, 29, 30, 40, 50, 60, 70, 80, 90, 100, 120, 140, 160, 180, 200, or more nucleotides or residues in length, which comprise or encode an activity of a reference polynucleotide or polypeptide. Representative biologically active fragments generally participate in an interaction, e.g., an intramolecular or an inter-molecular interaction. An inter-molecular interaction can be a specific binding interaction or an enzymatic interaction. An inter-molecular interaction can be between a ADH polypeptide and co-factor molecule, such as a nicotinamide adenine dinucleotide (NAD+), NADH, nicotinamide adenine dinucleotide phosphate (NADP+), or NADPH molecule. Biologically active portions of a ADH polypeptides include peptides comprising amino acid sequences with sufficient similarity or identity to or derived from the amino acid sequences of any of SEQ ID NOs:2, 4, 6, 8, 10, 12, 14, 16, 18, 20, 22, 24, 26, 28, 30, 32, 34, 36, 38, or 78.

By "coding sequence" is meant any nucleic acid sequence that contributes to the code for the polypeptide product of a gene. By contrast, the term "non-coding sequence" refers to any nucleic acid sequence that does not contribute to the code for the polypeptide product of a gene.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of' indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that no other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

By "corresponds to" or "corresponding to" is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

By "derivative" is meant a polypeptide that has been derived from the basic sequence by modification, for example by conjugation or complexing with other chemical moieties or by post-translational modification techniques as would be understood in the art. The term "derivative" also includes within its scope alterations that have been made to a parent sequence including additions or deletions that provide for functional equivalent molecules.

As used herein, the terms "function" and "functional" and the like refer to a biological, enzymatic, or therapeutic function.

The term "exogenous" refers generally to a polynucleotide sequence or polypeptide that does not naturally occur in a wild-type cell or organism, but is typically introduced into the cell by molecular biological techniques, i.e., engineering to produce a recombinant microorganism. Examples of "exogenous" polynucleotides include vectors, plasmids, and/or man-made nucleic acid constructs encoding a desired protein or enzyme. The term "endogenous" refers generally to naturally occurring polynucleotide sequences or polypeptides that may be found in a given wild-type cell or organism. For example, certain naturally-occuring bacterial or yeast species do not typically contain a benzaldehyde lyase gene, and, therefore, do not comprise an "endogenous" polynucleotide sequence that encodes a benzaldehyde lyase. In this regard, it is also noted that even though an organism may comprise an endogenous copy of a given polynucleotide sequence or gene, the introduction of a plasmid or vector encoding that sequence, such as to overexpress or otherwise regulate the expression of the encoded protein, represents an "exogenous" copy of that gene or polynucleotide sequence. Any of the of pathways, genes, or enzymes described herein may utilize or rely on an "endogenous" sequence, or may be provided as one or more "exogenous" polynucleotide sequences, and/or may be utilized according to the endogenous sequences already contained within a given microorganism.

A "recombinant" microorganism comprises one or more exogenous nucleotide sequences, such as in a plasmid or vector.

A "microbial system" relates generally to a population of recombinant microorganism, such as that contained within an incubator or other type of microbial culturing flask/device/well, or such as that found growing on a dish or plate (*e.g.,* an agarose containing petri dish).

By "gene" is meant a unit of inheritance that occupies a specific locus on a chromosome and consists of transcriptional and/or translational regulatory sequences and/or a coding region and/or non-translated sequences (*i.e.,* introns, 5' and 3' untranslated sequences).

"Homology" refers to the percentage number of nucleic or amino acids that are identical or constitute conservative substitutions. Homology may be determined using sequence comparison programs such as GAP (Deveraux et al., 1984, Nucleic Acids Research 12, 387-395) which is incorporated herein by reference. In this way sequences of a similar or substantially different length to those cited herein could be compared by insertion of gaps into the alignment, such gaps being determined, for example, by the comparison algorithm used by GAP.

The term "host cell" includes an individual cell or cell culture which can be or has been a recipient of any recombinant vector(s) or isolated polynucleotide of the invention. Host cells include progeny of a single host cell, and the progeny may not necessarily be completely identical (in morphology or in total DNA complement) to the original parent cell due to natural, accidental, or deliberate mutation and/or change. A host cell includes cells transfected or infected *in vivo* or *in vitro* with a recombinant vector or a polynucleotide of the invention. A host cell which comprises a recombinant vector of the invention is a recombinant host cell.

By "isolated" is meant material that is substantially or essentially free from components that normally accompany it in its native state. For example, an "isolated polynucleotide", as used herein, refers to a polynucleotide, which has been purified from the sequences which flank it in a naturally-occurring state, *e.g.,* a DNA fragment which has been removed from the sequences that are normally adjacent to the fragment. Alternatively, an "isolated peptide" or an "isolated polypeptide" and the like, as used herein, refer to *in vitro* isolation and/or purification of a peptide or polypeptide molecule from its natural cellular environment, and from association with other components of the cell, *i.e.,* it is not associated with *in vivo* substances.

A "polysaccharide," "suitable monosaccharide" or "suitable oligosaccharide," as the recitation is used herein, may be used as a source of energy and carbon in a microorganism, and may be suitable for use in a biofuel biosynthesis pathway for producing hydrocarbons such as biofuels or biopetrols. Examples of polysaccharides, suitable monosaccharides, and suitable oligosaccharides include, but are not limited to, alginate, agar, fucoidan, pectin, gluronate, mannuronate, mannitol, lyxose, glycerol, xylitol, glucose, mannose, galactose, xylose, arabinose, glucuronate, galacturonate, rhamnose, and 2-keto-3-deoxy D-gluconate-6-phosphate (KDG), and the like.

By "obtained from" is meant that a sample such as, for example, a polynucleotide extract or polypeptide extract is isolated from, or derived from, a particular source of the subject. For example, the extract can be obtained from a tissue or a biological fluid isolated directly from the subject.

The term "oligonucleotide" as used herein refers to a polymer composed of a multiplicity of nucleotide residues (deoxyribonucleotides or ribonucleotides, or related structural variants or synthetic analogues thereof) linked via phosphodiester bonds (or related structural variants or synthetic analogues thereof). Thus, while the term "oligonucleotide" typically refers to a nucleotide polymer in which the nucleotide residues and linkages between them are naturally occurring, it will be understood that the term also includes within its scope various analogues including, but not restricted to, peptide nucleic acids (PNAs), phosphoramidates, phosphorothioates, methyl phosphonates, 2-O-methyl ribonucleic acids, and the like. The exact size of the molecule can vary depending on the particular application. An oligonucleotide is typically rather short in length, generally from about 10 to 30 nucleotide residues, but the term can refer to molecules of any length, although the term "polynucleotide" or "nucleic acid" is typically used for large oligonucleotides.

The term "operably linked" as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e., the genes from which it is derived.

The recitation "optimized" as used herein refers to a pathway, gene, polypeptide, enzyme, or other molecule having an altered biological activity, such as by the genetic alteration of a polypeptide's amino acid sequence or by the alteration/modification of the polypeptide's surrounding cellular environment, to improve its functional characteristics in relation to the original molecule or original cellular environment (*e.g.,* a wild-type sequence of a given polypeptide or a wild-type microorganism). Any of the polypeptides or enzymes described herein may be optionally "optimized," and any of the genes or nucleotide sequences described herein may optionally encode an optimized polypeptide or enzyme. Any of the pathways described herein may optionally contain one or more "optimized" enzymes, or one or more nucleotide sequences encoding for an optimized enzyme or polypeptide.

Typically, the improved functional characteristics of the polypeptide, enzyme, or other molecule relate to the suitability of the polypeptide or other molecule for use in a biological pathway (*e.g.,* a biosynthesis pathway, a C-C ligation pathway) to convert a monosaccharide or oligosaccharide into a biofuel. Certain embodiments, therefore, contemplate the use of "optimized" biological pathways. An exemplary "optimized" polypeptide may contain one or more alterations or mutations in its amino acid coding sequence (*e.g.,* point mutations, deletions, addition of heterologous sequences) that facilitate improved expression and/or stability in a given microbial system or microorganism, allow regulation of polypeptide activity in relation to a desired substrate (*e.g.,* inducible or repressible activity), modulate the localization of the polypeptide within a cell (*e.g.,* intracellular localization, extracellular secretion), and/or effect the polypeptide's overall level of activity in relation to a desired substrate (*e.g.,* reduce or increase enzymatic activity). A polypeptide or other molecule may also be "optimized" for use with a given microbial system or microorganism by altering one or more pathways within that system or organism, such as by altering a pathway that regulates the expression (*e.g.,* up-regulation), localization, and/or activity of the "optimized" polypeptide or other molecule, or by altering a pathway that minimizes the production of undesirable byproducts, among other alterations. In this manner, a polypeptide or other molecule may be "optimized" with or without altering its wild-type amino acid sequence or original chemical structure. Optimized polypeptides or biological pathways may be obtained, for example, by direct mutagenesis or by natural selection for a desired phenotype, according to techniques known in the art.

In certain aspects, "optimized" genes or polypeptides may comprise a nucleotide coding sequence or amino acid sequence that is 50% to 99% identical (including all integeres in between) to the nucleotide or amino acid sequence of a reference (e.g., wild-type) gene or polypeptide described herein. In certain aspects, an "optimized" polypeptide or enzyme may have about 1, 2, 3, 4, 5, 6, 7, 8, 9, 10, 20, 30, 40, 50, 100 (including all integers and decimal points in between e.g., 1.2, 1.3, 1.4, 1.5, 5.5, 5.6, 5.7, 60, 70, etc.), or more times the biological activity of a reference polypeptide.

The recitation "polynucleotide" or "nucleic acid" as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA.

The terms "polynucleotide variant" and "variant" and the like refer to polynucleotides displaying substantial sequence identity with a reference polynucleotide sequence or polynucleotides that hybridize with a reference sequence under stringent conditions that are defined hereinafter. These terms also encompass polynucleotides that are distinguished from a reference polynucleotide by the addition, deletion or substitution of at least one nucleotide. Accordingly, the terms "polynucleotide variant" and "variant" include polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide. Polynucleotide variants include polynucleotides having at least 50% (and at least 51 % to at least 99% and all integer percentages in between) sequence identity with the sequence set forth in any one of SEQ ID NOs:1, 3, 5, 7, 9, 11, 13, 15, 17, 19, 21, 23, 25, 27, 29, 31, 33, 35, or 37. The terms "polynucleotide variant" and "variant" also include naturally occurring allelic variants.

"Polypeptide", "peptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers.

The recitations "ADH polypeptide" or "variants thereof' as used herein encompass, without limitation, polypeptides having the amino acid sequence that shares at least 50% (and at least 51 % to at least 99% and all integer percentages in between) sequence identity with the sequence set forth in any one of SEQ ID NOs:2, 4, 6, 8, 10, 12, 14, 16, 18, 20, 22, 24, 26, 28, 30, 32, 34, 36, 38, or 78. These recitations further encompass natural allelic variation of ADH polypeptides that may exist and occur from one bacterial species to another.

ADH polypeptides, including variants thereof, encompass polypeptides that exhibit at least about 10%, 20%, 30%, 40%, 50%, 60%, 70%, 80%, 90%, 100%, 110%, 120%, and 130% of the specific activity of wild-type ADH polypeptides (i.e., such as having an alchohol dehydrogenase activity, including DEHU hydrogenase activity and/or D-mannuronate hydrogenase activity). ADH polypeptides, including variants, having substantially the same or improved biological activity relative to wildtype ADH polypeptides, encompass polypeptides that exhibit at least about 25%, 50%, 75%, 100%, 110%, 120% or 130% of the specific biological activity of wild-type polypeptdies. For purposes of the present application, ADH-related biological activity may be quantified, for example, by measuring the ability of an ADH polypeptide, or variant thereof, to consume NADPH using DEHU or D-mannuronate as a substrate (see, e.g., Example 2). ADH polypeptides, including variants, having substantially reduced biological activity relative to wild-type ADH are those that exhibit less than about 25%, 10%, 5% or 1% of the specific activity of wild-type ADH.

The recitation polypeptide "variant" refers to polypeptides that are distinguished from a reference polypeptide by the addition, deletion or substitution of at least one amino acid residue. In certain embodiments, a polypeptide variant is distinguished from a reference polypeptide by one or more substitutions, which may be conservative or non-conservative. In certain embodiments, the polypeptide variant comprises conservative substitutions and, in this regard, it is well understood in the art that some amino acids may be changed to others with broadly similar properties without changing the nature of the activity of the polypeptide. Polypeptide variants also encompass polypeptides in which one or more amino acids have been added or deleted, or replaced with different amino acid residues.

The present invention contemplates the use in the methods and microbial systems of the present application of full-length ADH sequences as well as their biologically active fragments. Typically, biologically active fragments of a full-length ADH polypeptides may participate in an interaction, for example, an intramolecular or an inter-molecular interaction. An inter-molecular interaction can be a specific binding interaction or an enzymatic interaction (e.g., the interaction can be transient and a covalent bond is formed or broken). Biologically active fragments of a full-length ADH polypeptide include peptides comprising amino acid sequences sufficiently similar to or derived from the amino acid sequences of a (putative) full-length ADH. Typically, biologically active fragments comprise a domain or motif with at least one activity of a full-length ADH polypeptide and may include one or more (and in some cases all) of the various active domains, and include fragments having fragments having a hydrogenase activity, such as an alcohol dehydrogenase activity, a DEHU hydrogenase activity, and/or a D-mannuronate hydrogenase activity. A biologically active fragment of a full-length ADH polypeptide can be a polypeptide which is, for example, 10, 11, 12, 13, 14, 15, 16, 17, 18, 19, 20, 21, 22, 23, 24, 25, 26, 27, 28, 29,30, 40, 50, 60, 70, 80, 90, 100, 120, 150, or more contiguous amino acids of the amino acid sequences set forth in any one of SEQ ID NO:2, 4, 6, 8, 10, 12, 14, 16, 18, 20, 22, 24, 26, 28, 30, 32, 34, 36, 38, or 78. In certain embodiments, a biologically active fragments comprises a NAD+, NADH, NADP+, or NADPH binding motif as described herein. Suitably, the biologically-active fragment has no less than about 1%, 10%, 25% 50% of an activity of the full-length polypeptide from which it is derived.

The recitations "sequence identity" or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (*e.g.,* A, T, C, G, I) or the identical amino acid residue (*e.g.,* Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (*i.e.,* the window size), and multiplying the result by 100 to yield the percentage of sequence identity.

Terms used to describe sequence relationships between two or more polynucleotides or polypeptides include "reference sequence", "comparison window", "sequence identity", "percentage of sequence identity" and "substantial identity". A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul *et al.,* 1997, *Nucl. Acids Res.* **25:**3389. A detailed discussion of sequence analysis can be found in Unit 19.3 of Ausubel *et al.,* "Current Protocols in Molecular Biology", John Wiley & Sons Inc, 1994-1998, Chapter 15.

By "vector" is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, *i.e.,* a vector that exists as an extra-chromosomal entity, the replication of which is independent of chromosomal replication, *e.g.,* a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In the present case, the vector is preferably one which is operably functional in a bacterial cell. The vector can also include a selection marker such as an antibiotic resistance gene that can be used for selection of suitable transformants.

The terms "wild-type" and "naturally occurring" are used interchangeably to refer to a gene or gene product that has the characteristics of that gene or gene product when isolated from a naturally occurring source. A wild type gene or gene product (*e.g.,* a polypeptide) is that which is most frequently observed in a population and is thus arbitrarily designed the "normal" or "wild-type" form of the gene.

Embodiments of the present invention relate in part to the isolation and characterization of bacterial dehydrogenase genes, and the polypeptides encoded by these genes. Certain embodiments may include isolated dehydrogenase polypeptides having an alcohol dehydrogenase activity, which may be referred to as alcohol dehydrogenase (ADH) polypeptides. ADH polypeptides according to the present application may have a DEHU hydrogenase activity, a D-mannuronate activity, or both DEHU and D-mannuronate hydrogenase activities. Other embodiments may include polynucleotides encoding such polypeptides. For example, the molecules of the present application may include isolated polynucleotides, and fragments or variants thereof, selected from

(a) an isolated polynucleotide comprising a nucleotide sequence at least 80% identical to the nucleotide sequence set forth in SEQ ID NO:1, 3, 5, 7, 9, 11, 13, 15, 17, 19, 21, 23, 25, 27, 29, 31, 33, 35, or 37;
(b) an isolated polynucleotide comprising a nucleotide sequence at least 90% identical to the nucleotide sequence set forth in SEQ ID NO:1, 3, 5, 7, 9, 11, 13, 15, 17, 19, 21, 23, 25, 27, 29, 31, 33, 35, or 37;

(c) an isolated polynucleotide comprising a nucleotide sequence at least 95% identical to the nucleotide sequence set forth in SEQ ID NO:1, 3, 5, 7, 9, 11, 13, 15, 17, 19, 21, 23, 25, 27, 29, 31, 3 3, 3 5, or 3 7;

(d) an isolated polynucleotide comprising a nucleotide sequence at least 97% identical to the nucleotide sequence set forth in SEQ ID NO:1, 3, 5, 7, 9, 11, 13, 15, 17, 19, 21, 23, 25, 27, 29, 31, 33, 35, or 37;

(e) an isolated polynucleotide comprising a nucleotide sequence at least 99% identical to the nucleotide sequence set forth in SEQ ID NO:1, 3, 5, 7, 9, 11, 13, 15, 17, 19, 21, 23, 25, 27, 29, 31, 33, 35, or 37; and

(f) an isolated polynucleotide comprising the nucleotide sequence set forth in SEQ ID NO:1, 3, 5, 7, 9, 11, 13, 15, 17, 19, 21, 23, 25, 27, 29, 31, 33, 35, or 37,

wherein the isolated nucleotide encodes a polypeptide having a dehydrogenase activity. In certain embodiments, the polypeptide has an alcohol dehydrogenase activity, such as a DEHU hydrogenase activity and/or a D-mannuronate hydrogenase activity.

Molecules of the present inventio may also include isolated ADH polypeptides, or variants, fragments, or derivatives, thereof, which embodiments may be selected from

(a) an isolated polypeptide comprising an amino acid sequence at least 80% identical to the amino acid sequence set forth in SEQ ID NO:2, 4, 6, 8, 10, 12, 14, 16, 18, 20, 22, 24, 26, 28, 30, 32, 34, 36, 38, or 78;

(b) an isolated polypeptide comprising an amino acid sequence at least 90% identical to the amino acid sequence set forth in SEQ ID NO:2, 4, 6, 8, 10, 12, 14, 16, 18, 20, 22, 24, 26, 28, 30, 32, 34, 36, 38, or 78;

(c) an isolated polypeptide comprising an amino acid sequence at least 95% identical to the amino acid sequence set forth in SEQ ID NO:2, 4, 6, 8, 10, 12, 14, 16, 18, 20, 22, 24, 26, 28, 30, 32, 34, 36, 38, or 78;

(d) an isolated polypeptide comprising an amino acid sequence at least 97% identical to the amino acid sequence set forth in SEQ ID NO:2, 4, 6, 8, 10, 12, 14, 16, 18, 20, 22, 24, 26, 28, 30, 32, 34, 36, 38, or 78;

(e) an isolated polypeptide comprising an amino acid sequence at least 99% identical to the amino acid sequence set forth in SEQ ID NO:2, 4, 6, 8, 10, 12, 14, 16, 18, 20, 22, 24, 26, 28, 30, 32, 34, 36, 38, or 78; and

(e) an isolated polypeptide comprising the amino acid sequence set forth in SEQ ID NO:2, 4, 6, 8, 10, 12, 14, 16, 18, 20, 22, 24, 26, 28, 30, 32, 34, 36, 38, or 78,

wherein the isolated polypeptide has a dehydrogenase activity. In certain embodiments, the polypeptide has an alcohol dehydrogenase activity, such as a DEHU hydrogenase activity, and/or a D-mannuronate hydrogenase activity.

In additional embodiments, an isolated polynucleotide as disclosed herein encodes a polypeptide that comprises at least one of a nicotinamide adenine dinucleotide (NAD+), NADH, nicotinamide adenine dinucleotide phosphate (NADP+), or NADPH binding motif. Other embodiments include ADH polypeptides, variants, fragments, or derivatives thereof, as disclosed herein, wherein the polypeptides comprise at least one of a NAD+, NADH, NADP+, or NADPH binding motif. In certain embodiments, the binding motif is selected from the group consisting of Y-X-G-G-X-Y (SEQ ID NO:67), Y-X-X-G-G-X-Y (SEQ ID NO:68), Y-X-X-X-G-G-X-Y (SEQ ID NO:69), Y-X-G-X-X-Y (SEQ ID NO:70), Y-X-X-G-G-X-X-Y (SEQ ID NO:71), Y-X-X-X-G-X-X-Y (SEQ ID NO:72), Y-X-G-X-Y (SEQ ID NO:73), Y-X-X-G-X-Y (SEQ ID NO:74), Y-X-X-X-G-X-Y (SEQ ID NO:75), and Y-X-X-X-X-G-X-Y (SEQ ID NO:76); wherein Y is independently selected from alanine, glycine, and serine, wherein G is glycine, and wherein X is independently selected from a genetically encoded amino acid. Not wishing to be bound by any theory, NAD+ and related molecules serve as co-factors in dehydrogenase reactions, and these binding motifs are generally conserved in alcohol dehydrogenases and play an important role in NAD+, NADH, NADP+, or NADPH binding.

Variant proteins encompassed by the present application are biologically active, that is, they continue to possess the desired biological activity of the native protein. Such variants may result from, for example, genetic polymorphism or from human manipulation. Biologically active variants of a native or wild-type ADH polypeptide will have at least 40%, 50%, 60%, 70%, generally at least 75%, 80%, 85%, usually about 90% to 95% or more, and typically about 98% or more sequence similarity or identity with the amino acid sequence for the native protein as determined by sequence alignment programs described elsewhere herein using default parameters. A biologically active variant of a wild-type ADH polypeptide may differ from that protein generally by as much 200, 100, 50 or 20 amino acid residues or suitably by as few as 1-15 amino acid residues, as few as 1-10, such as 6-10, as few as 5, as few as 4, 3, 2, or even 1 amino acid residue. In some embodiments, a ADH polypeptide differs from the corresponding sequences in SEQ ID NO: 2, 4, 6, 8, 10, 12, 14, 16, 18, 20, 22, 24, 26, 28, 30, 32, 34, 36, 38, or 78 by at least one but by less than 15, 10 or 5 amino acid residues. In other embodiments, it differs from the corresponding sequences in SEQ ID NO: 2, 4, 6, 8, 10, 12, 14, 16, 18, 20, 22, 24, 26, 28, 30, 32, 34, 36, 38, or 78 by at least one residue but less than 20%, 15%, 10% or 5% of the residues.

An ADH polypeptide may be altered in various ways including amino acid substitutions, deletions, truncations, and insertions. Methods for such manipulations are generally known in the art. For example, amino acid sequence variants of an ADH polypeptide can be prepared by mutations in the DNA. Methods for mutagenesis and nucleotide sequence alterations are well known in the art. See, for example, Kunkel (1985, Proc. Natl. Acad. Sci. USA. 82: 488-492), Kunkel et al., (1987, Methods in Enzymol, 154: 367-382), U.S. Pat. No. 4,873,192, Watson, J. D. et al., ("Molecular Biology of the Gene", Fourth Edition, Benjamin/Cummings, Menlo Park, Calif., 1987) and the references cited therein. Guidance as to appropriate amino acid substitutions that do not affect biological activity of the protein of interest may be found in the model of Dayhoff et al., (1978) Atlas of Protein Sequence and Structure (Natl. Biomed. Res. Found., Washington, D.C.). Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of ADH polypeptides. Recursive ensemble mutagenesis (REM), a technique which enhances the frequency of functional mutants in the libraries, can be used in combination with the screening assays to identify ADH polypeptide variants (Arkin and Yourvan (1992) Proc. Natl. Acad. Sci. USA 89: 7811-7815; Delgrave et al., (1993) Protein Engineering, 6: 327-331). Conservative substitutions, such as exchanging one amino acid with another having similar properties, may be desirable as discussed in more detail below.

Variant ADH polypeptides may contain conservative amino acid substitutions at various locations along their sequence, as compared to the parent ADH amino acid sequences. A "conservative amino acid substitution" is one in which the amino acid residue is replaced with an amino acid residue having a similar side chain. Families of amino acid residues having similar side chains have been defined in the art, which can be generally sub-classified as follows:

Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.

Basic: The residue has a positive charge due to association with H ion at physiological pH or within one or two pH units thereof (*e.g.,* histidine) and the residue is attracted by aqueous solution so as to seek the surface positions in the conformation of a peptide in which it is contained when the peptide is in aqueous medium at physiological pH. Amino acids having a basic side chain include arginine, lysine and histidine.

Charged: The residues are charged at physiological pH and, therefore, include amino acids having acidic or basic side chains (*i.e.,* glutamic acid, aspartic acid, arginine, lysine and histidine).

Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.

Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterizes certain amino acids as "small" since their side chains are not sufficiently large, even if polar groups are lacking, to confer hydrophobicity. With the exception of proline, "small" amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the α-amino group, as well as the α-carbon. Several amino acid similarity matrices (*e.g.,* PAM120 matrix and PAM250 matrix as disclosed for example by Dayhoff et al., (1978), A model of evolutionary change in proteins. Matrices for determining distance relationships In M. O. Dayhoff, (ed.), Atlas of protein sequence and structure, Vol. 5, pp. 345-358, National Biomedical Research Foundation, Washington DC; and by Gonnet et al., (1992, Science, 256(5062): 14430-1445), however, include proline in the same group as glycine, serine, alanine and threonine. Accordingly, for the purposes of the present invention, proline is classified as a "small" amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behaviour.

Amino acid residues can be further sub-classified as cyclic or noncyclic, and aromatic or non-aromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always non-aromatic. Dependent on their structural properties, amino acid residues may fall in two or more classes. For the naturally-occurring protein amino acids, sub-classification according to this scheme is presented in Table A.

**TABLE A**

| AMINO ACID SUB-CLASSIFICATION | |
|---|---|
| **SUB-CLASSES** | **AMINO ACIDS** |
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, Threonine |
| Polar/large | Asparagine, Glutamine |
| Hydrophobic | Tyrosine, Valine, Isoleucine, Leucine, Methionine, Phenylalanine, Tryptophan |
| Aromatic | Tryptophan, Tyrosine, Phenylalanine |
| Residues that influence chain orientation | Glycine and Proline |

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional ADH polypeptide can readily be determined by assaying its activity, as described herein (*see, e.g.,* Example 2). Conservative substitutions are shown in Table B under the heading of exemplary substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants are screened for biological activity.

**TABLE B**

| EXEMPLARY AMINO ACID SUBSTITUTIONS | | |
|---|---|---|
| **ORIGINAL RESIDUE** | **EXEMPLARY SUBSTITUTIONS** | **PREFERRED SUBSTITUTIONS** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | Ile |
| Lys | Arg, Gln, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |

(continued)

| EXEMPLARY AMINO ACID SUBSTITUTIONS | | |
|---|---|---|
| ORIGINAL RESIDUE | EXEMPLARY SUBSTITUTIONS | PREFERRED SUBSTITUTIONS |
| Val | Ile, Leu, Met, Phe, Ala, Norleu | Leu |

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G., *Biochemistry,* third edition, Wm.C. Brown Publishers (1993).

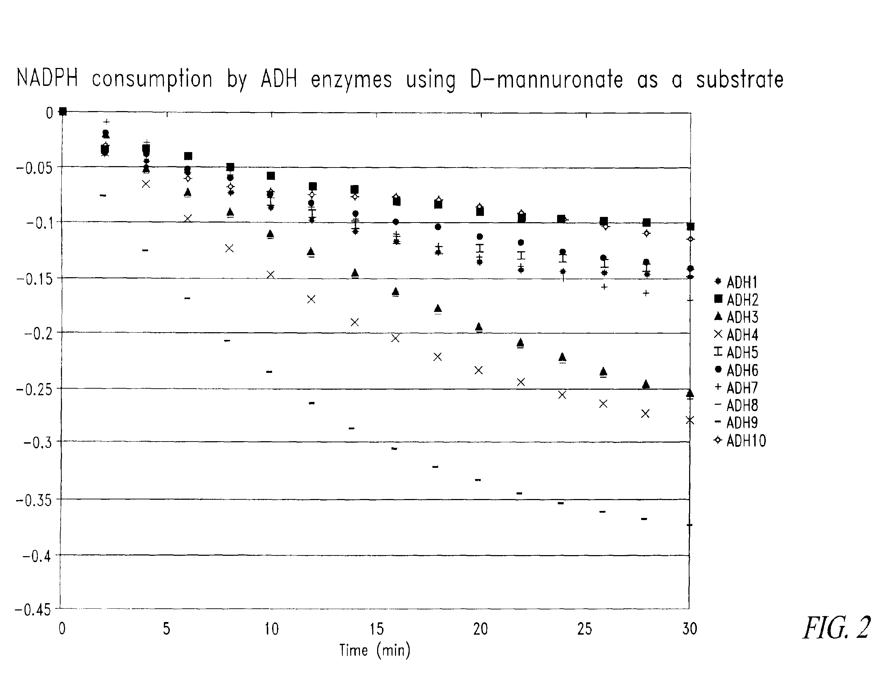

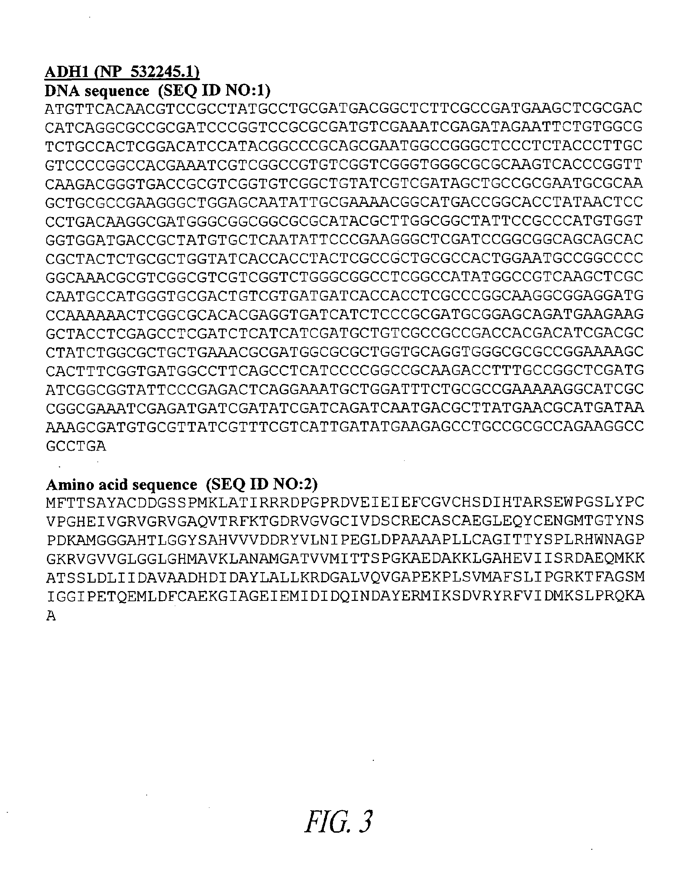

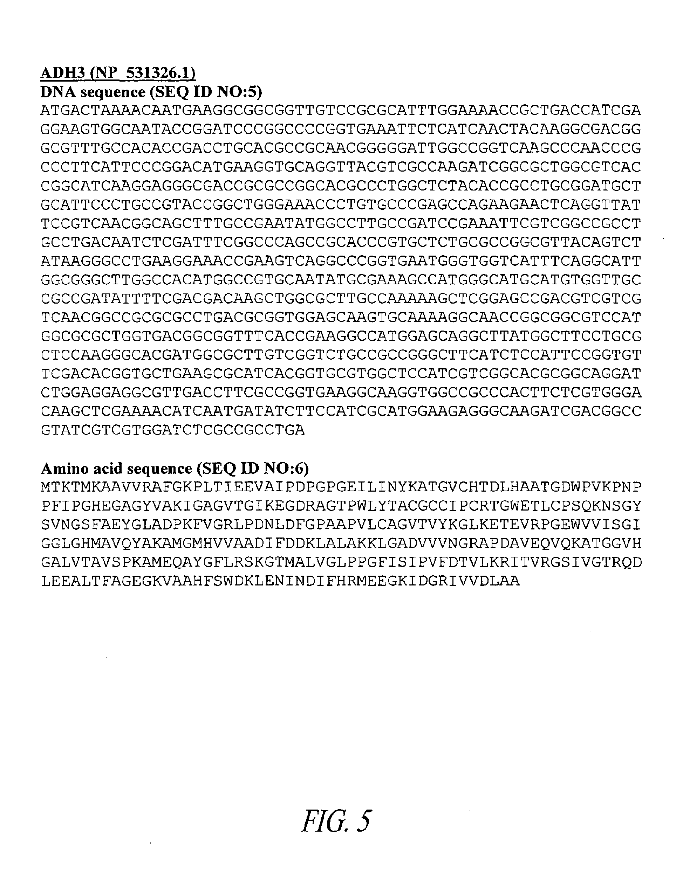

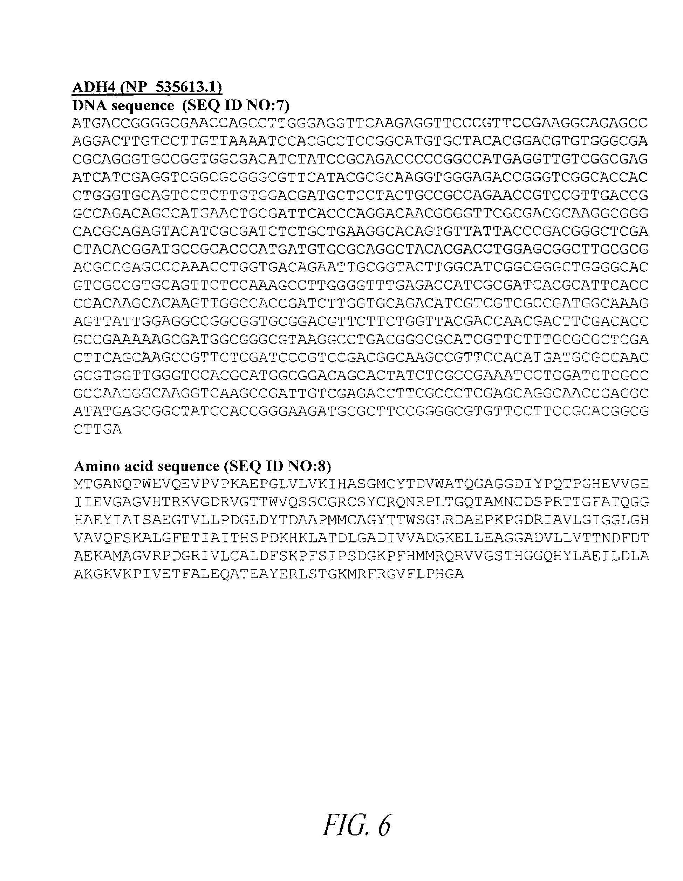

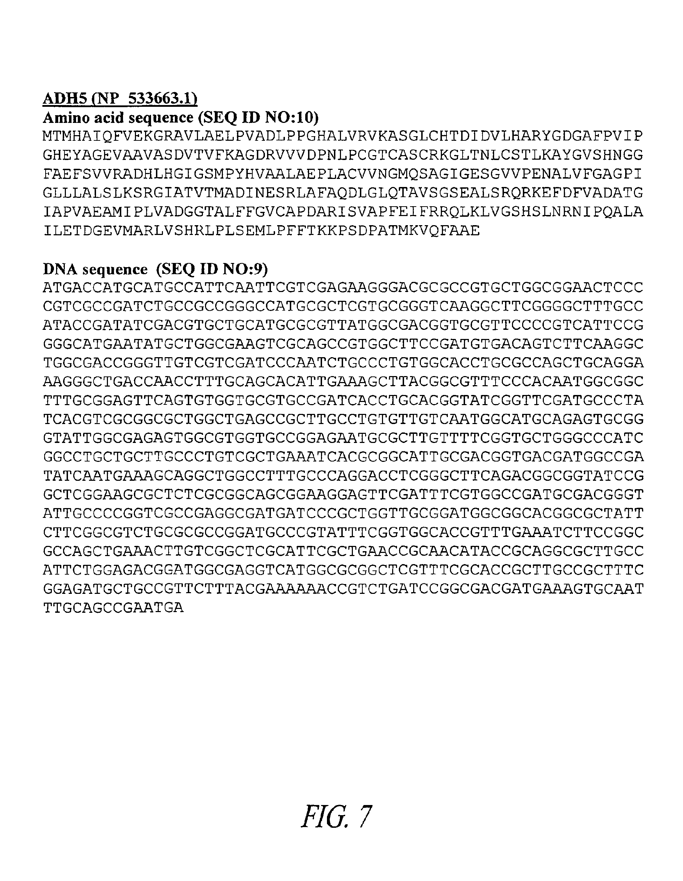

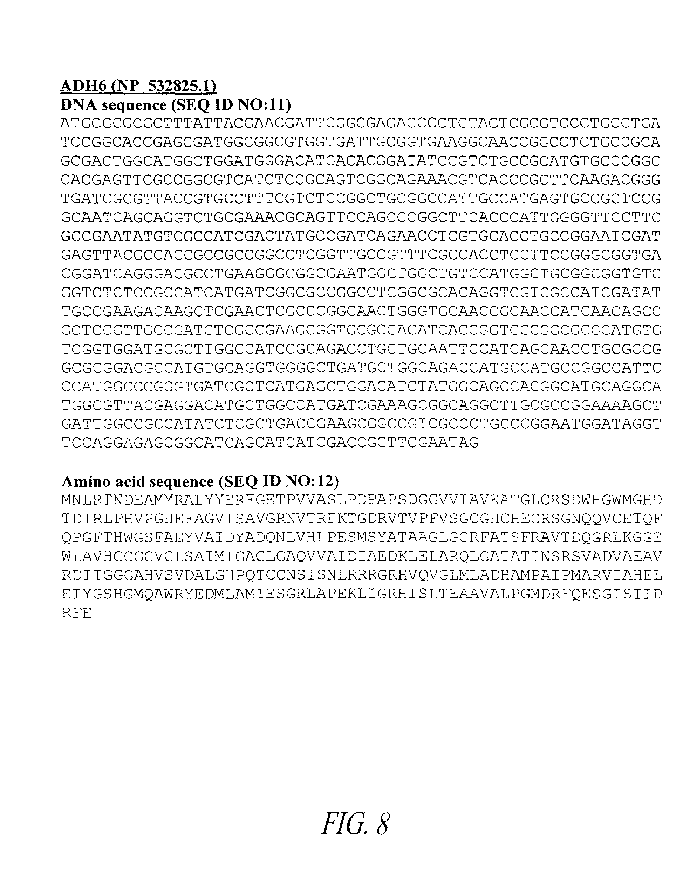

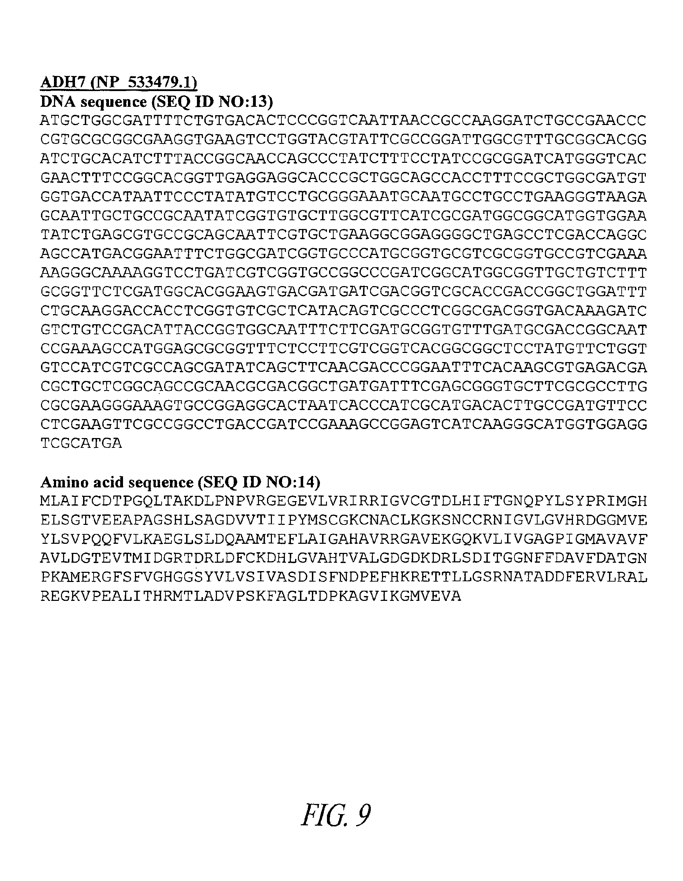

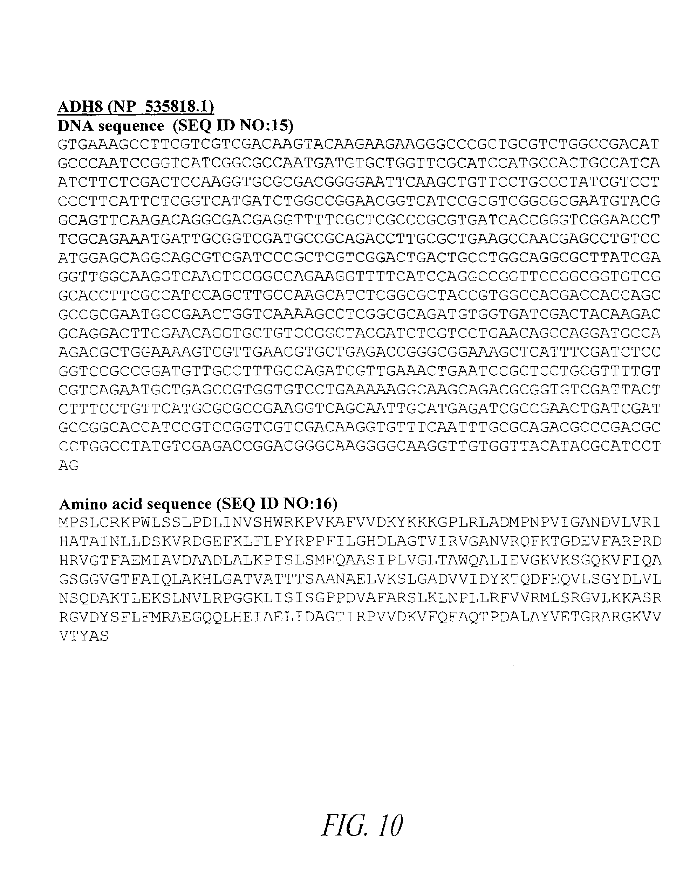

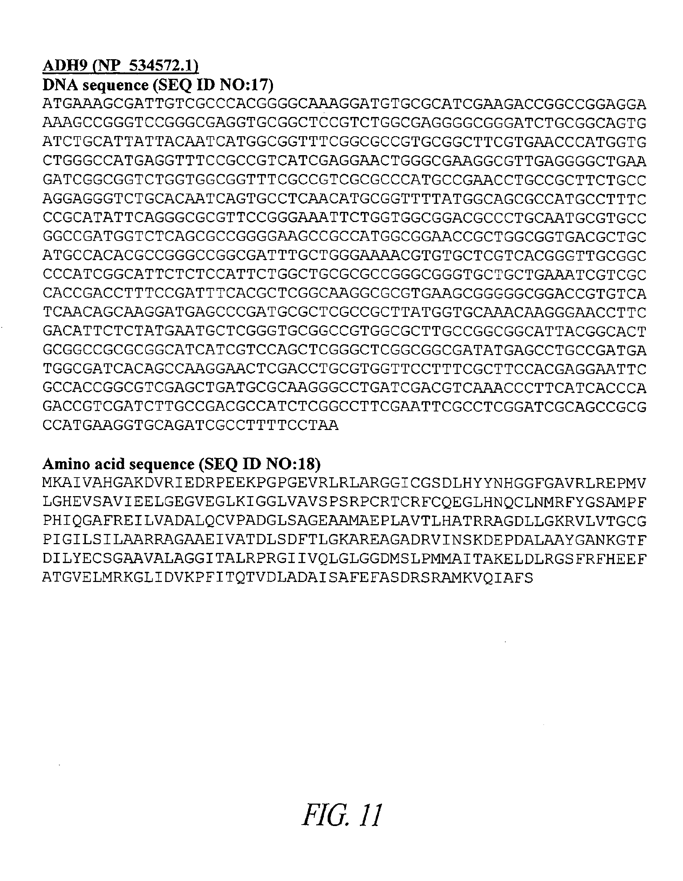

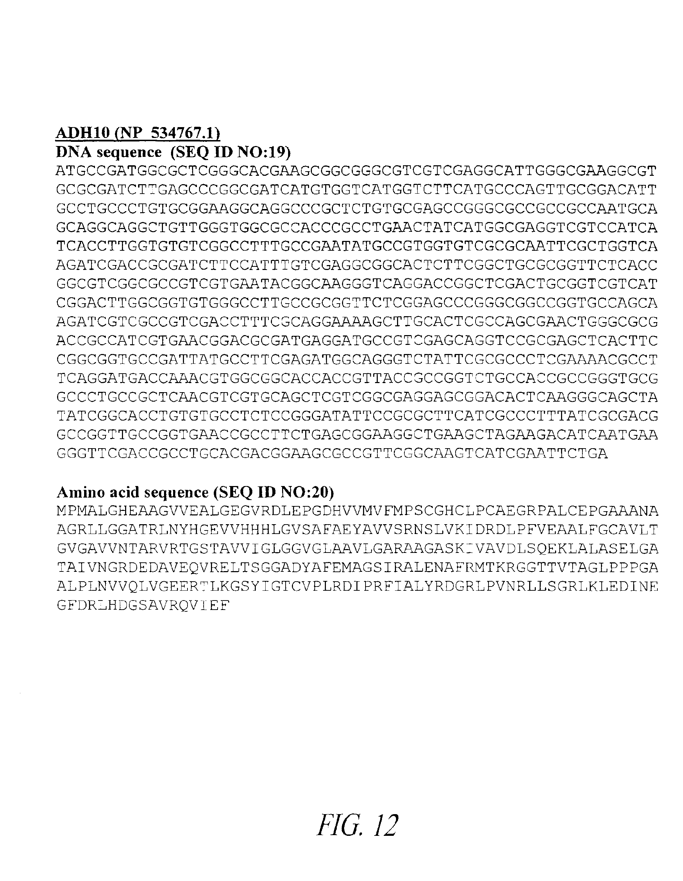

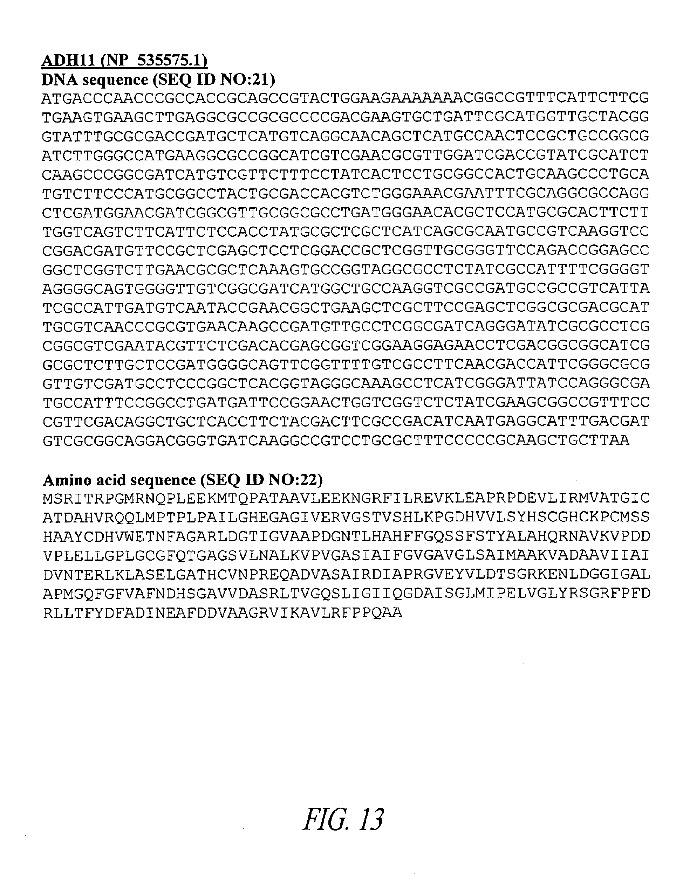

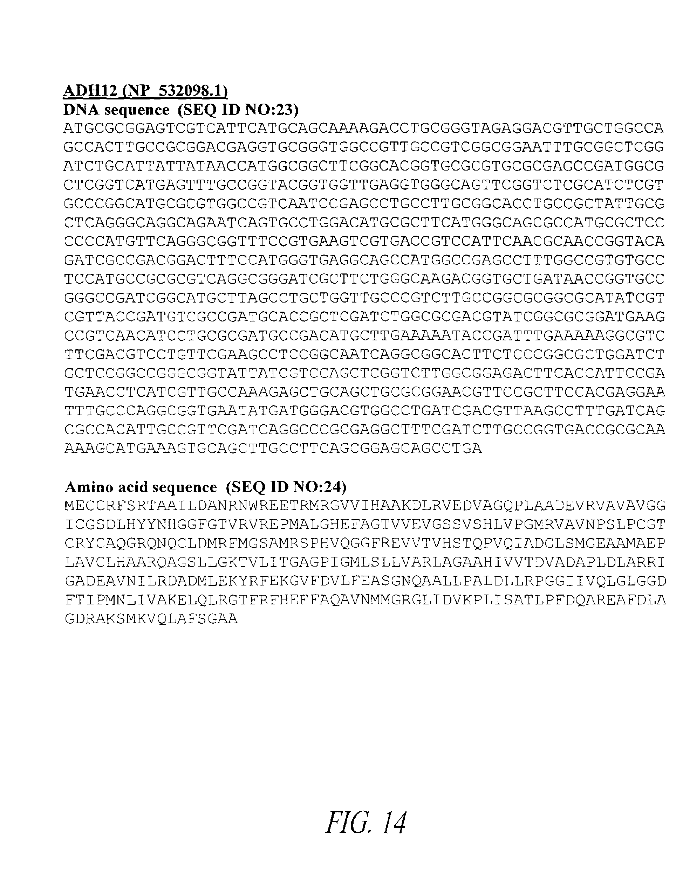

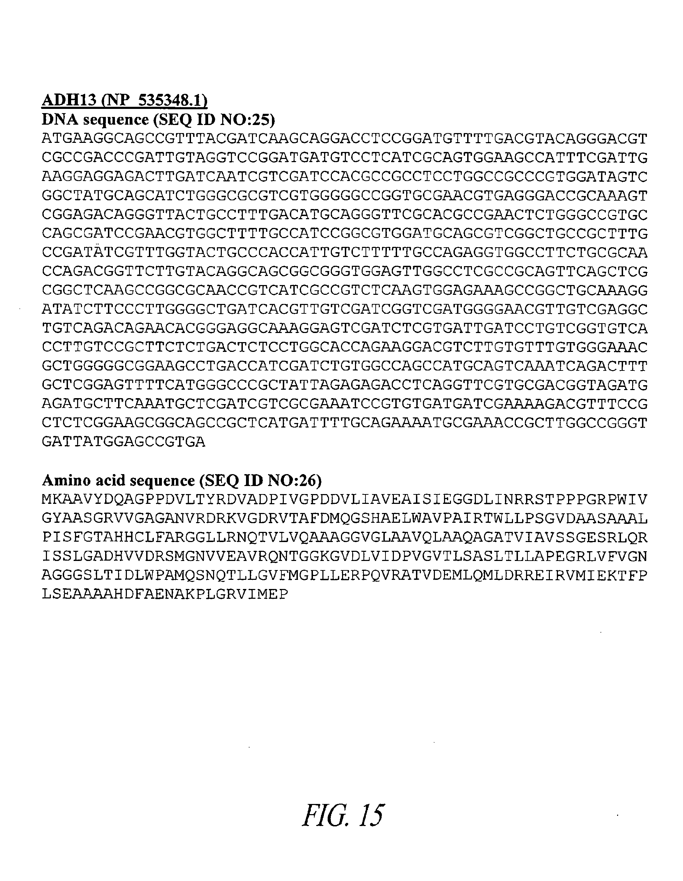

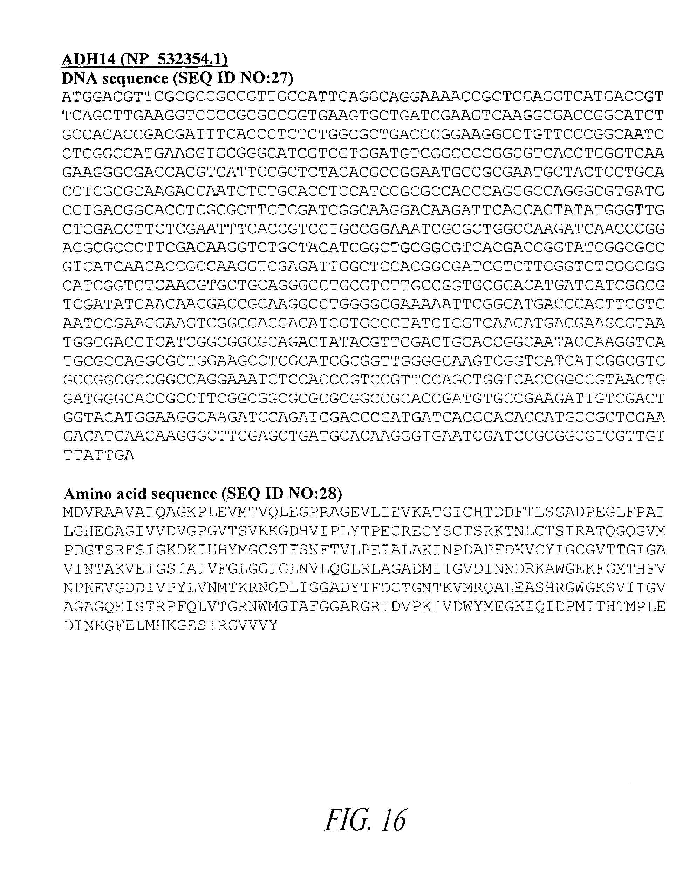

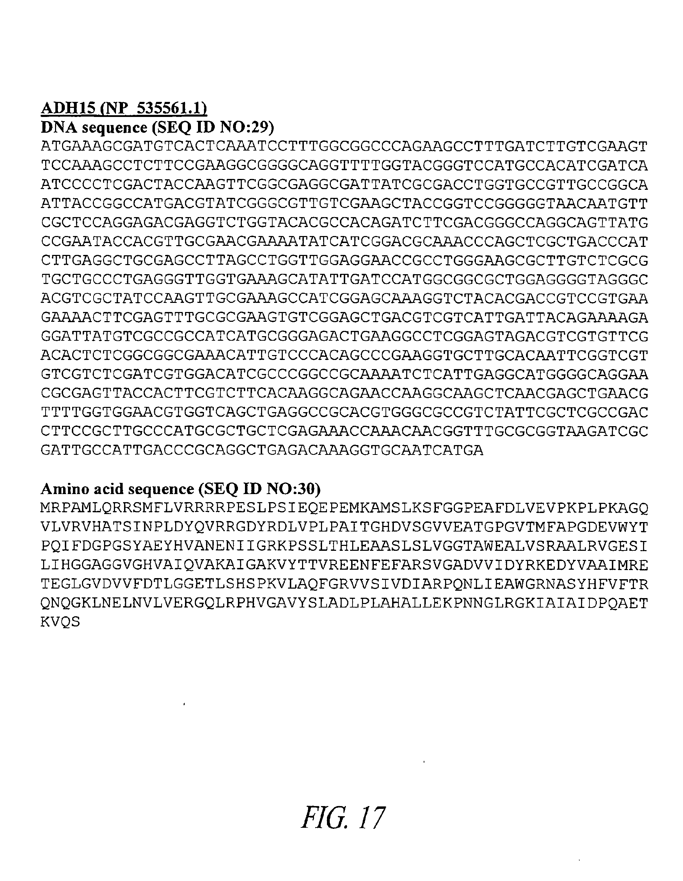

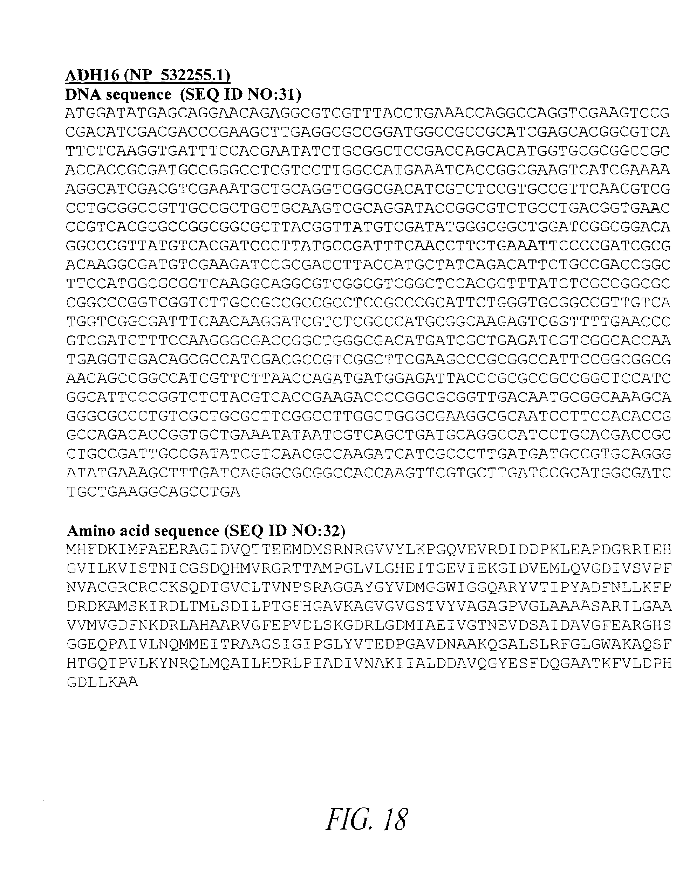

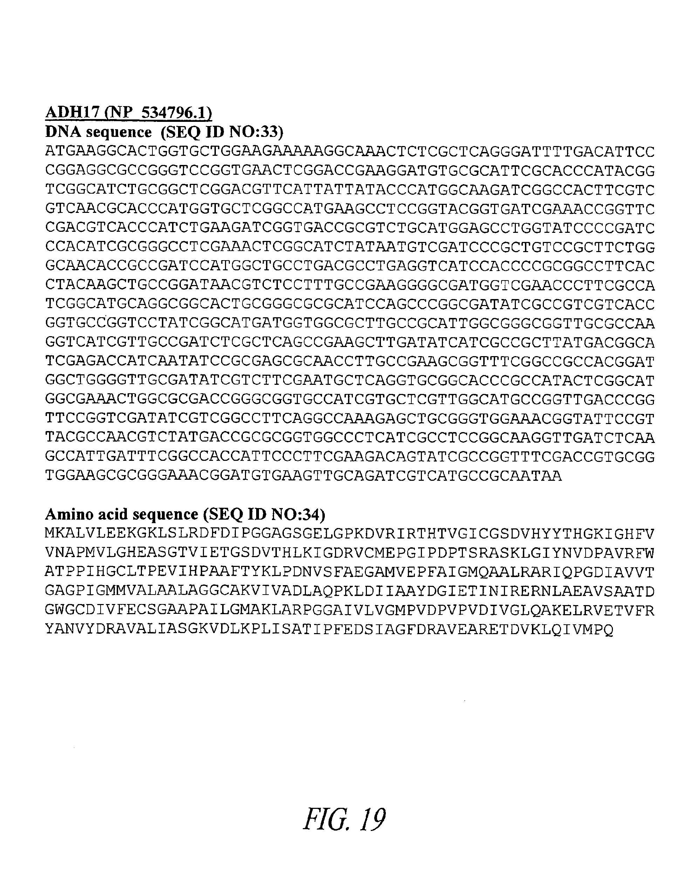

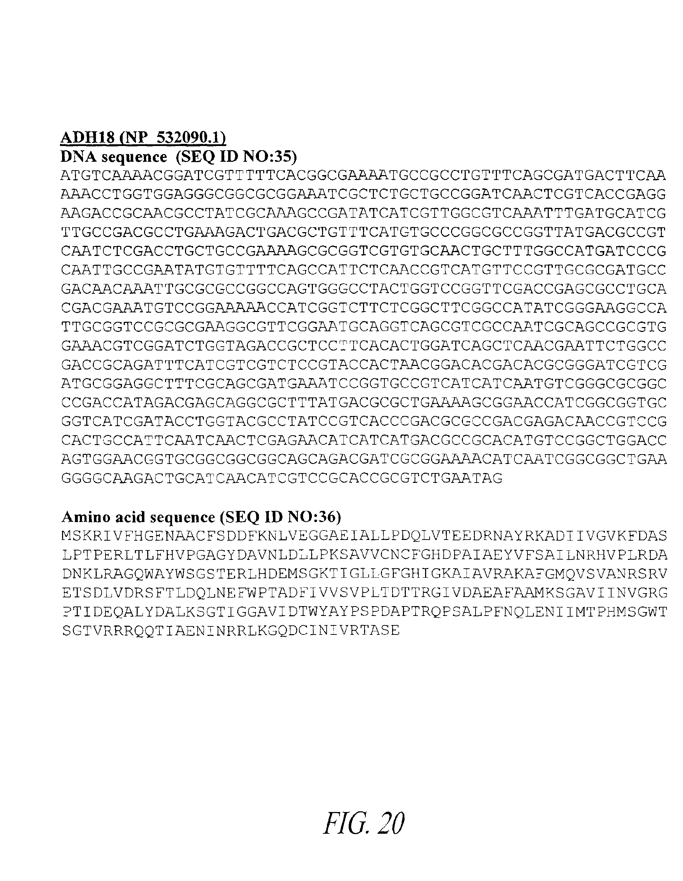

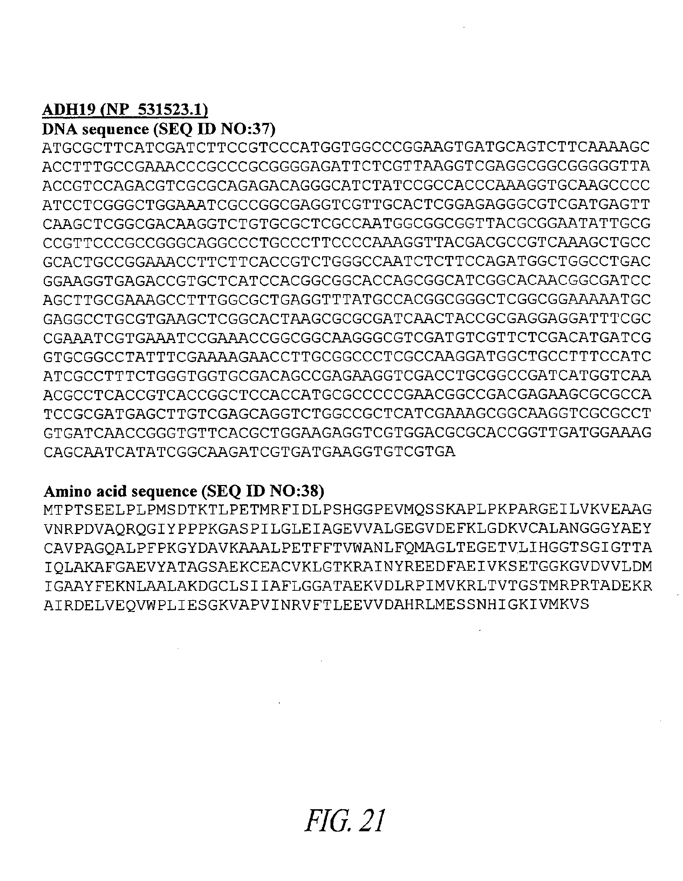

Thus, a predicted non-essential amino acid residue in a ADH polypeptide is typically replaced with another amino acid residue from the same side chain family. Alternatively, mutations can be introduced randomly along all or part of an ADH coding sequence, such as by saturation mutagenesis, and the resultant mutants can be screened for an activity of the parent polypeptide to identify mutants which retain that activity. Following mutagenesis of the coding sequences, the encoded peptide can be expressed recombinantly and the activity of the peptide can be determined. A "non-essential" amino acid residue is a residue that can be altered from the wild-type sequence of an embodiment polypeptide without abolishing or substantially altering one or more of its activities. Suitably, the alteration does not substantially alter one of these activities, for example, the activity is at least 20%, 40%, 60%, 70% or 80% of wild-type. Illustrative non-essential amino acid residues include any one or more of the amino acid residues that differ at the same position between the wild-type ADH polypeptides shown in Figures 2-21. An "essential" amino acid residue is a residue that, when altered from the wild-type sequence of a reference ADH polypeptide, results in abolition of an activity of the parent molecule such that less than 20% of the wild-type activity is present. For example, such essential amino acid residues include those that are conserved in ADH polypeptides across different species, *e.g.,* G-X-G-G-X-G (SEQ ID NO:77) that is conserved in the NADH-binding site of the ADH polypeptides from various bacterial sources.

Accordingly, embodiments of the present invention also contemplate as ADH polypeptides, variants of the naturally-occurring ADH polypeptide sequences or their biologically-active fragments, wherein the variants are distinguished from the naturally-occurring sequence by the addition, deletion, or substitution of one or more amino acid residues. In general, variants will display at least about 30, 40, 50, 55, 60, 65, 70, 75, 80, 85, 90, 91, 92, 93, 94, 95, 96, 97, 98, 99 % similarity to a parent ADH polypeptide sequence as, for example, set forth in SEQ ID NO: 2, 4, 6, 8, 10, 12, 14, 16, 18, 20, 22, 24, 26, 28, 30, 32, 34, 36, 38, or 78. Certain variants will have at least 30, 40, 50, 55, 60, 65, 70, 75, 80, 85, 90, 91, 92, 93, 94, 95, 96, 97, 98, 99% sequence identity to a parent ADH polypeptide sequence as, for example, set forth in SEQ ID NO: 2, 4, 6, 8, 10, 12, 14, 16, 18, 20, 22, 24, 26, 28, 30, 32, 34, 36, 38, or 78. Moreover, sequences differing from the native or parent sequences by the addition, deletion, or substitution of 1, 2, 3, 4, 5, 6, 7, 8, 9, 10, 11, 12, 13, 14, 15, 16, 17, 18, 19, 20, 30, 40, 50, 60, 70, 80, 90, 100 or more amino acids but which retain the properties of the parent ADH polypeptide are contemplated.

In some embodiments, variant polypeptides differ from a reference ADH sequence by at least one but by less than 50, 40, 30, 20, 15, 10, 8, 6, 5, 4, 3 or 2 amino acid residue(s). In other embodiments, variant polypeptides differ from the corresponding sequences of SEQ ID NO: 2, 4, 6, 8, 10 and 12 by at least 1% but less than 20%, 15%, 10% or 5% of the residues. (If this comparison requires alignment, the sequences should be aligned for maximum similarity. "Looped" out sequences from deletions or insertions, or mismatches, are considered differences.) The differences are, suitably, differences or changes at a non-essential residue or a conservative substitution.

In certain embodiments, a variant polypeptide includes an amino acid sequence having at least about 50%, 55%, 60%, 65%, 70%, 75%, 80%, 85%, 90%, 91%, 92%, 93%, 94% 95%, 96%, 97%, 98% or more similarity to a corresponding sequence of an ADH polypeptide as, for example, set forth in SEQ ID NO:2, 4, 6, 8, 10, 12, 14, 16, 18, 20, 22, 24, 26, 28, 30, 32, 34, 36, 38, or 78 and has the activity of an ADH polypeptide.

Calculations of sequence similarity or sequence identity between sequences (the terms are used interchangeably herein) are performed as follows.

To determine the percent identity of two amino acid sequences, or of two nucleic acid sequences, the sequences are aligned for optimal comparison purposes (*e.g.,* gaps can be introduced in one or both of a first and a second amino acid or nucleic acid sequence for optimal alignment and non-homologous sequences can be disregarded for comparison purposes). In certain embodiments, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch, (1970, J. Mol. Biol. 48: 444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al., (1990, J. Mol. Biol, 215: 403-10). BLAST nucleotide searches can be performed with the NBLAST program, score = 100, wordlength = 12 to obtain nucleotide sequences homologous to 53010 nucleic acid molecules of the invention. BLAST protein searches can be performed with the XBLAST program, score = 50, wordlength = 3 to obtain amino acid sequences homologous to 53010 protein molecules of the invention. To obtain gapped alignments for comparison purposes, Gapped BLAST can be utilized as described in Altschul et al., (1997, Nucleic Acids Res, 25: 3389-3402). When utilizing BLAST and Gapped BLAST programs, the default parameters of the respective programs (*e.g.,* XBLAST and NBLAST) can be used.

Variants of an ADH polypeptide can be identified by screening combinatorial libraries of mutants, *e.g.,* truncation mutants, of an ADH polypeptide. Libraries or fragments *e.g.,* N terminal, C terminal, or internal fragments, of an ADH protein coding sequence can be used to generate a variegated population of fragments for screening and subsequent selection of variants of an ADH polypeptide.

Methods for screening gene products of combinatorial libraries made by point mutation or truncation, and for screening cDNA libraries for gene products having a selected property are known in the art. Such methods are adaptable for rapid screening of the gene libraries generated by combinatorial mutagenesis of ADH polypeptides.

The ADH polypeptides of the application may be prepared by any suitable procedure known to those of skill in the art, such as by recombinant techniques. For example, ADH polypeptides may be prepared by a procedure including the steps of: (a) preparing a construct comprising a polynucleotide sequence that encodes an ADH polypeptdie and that is operably linked to a regulatory element; (b) introducing the construct into a host cell; (c) culturing the host cell to express the ADH polypeptide; and (d) isolating the ADH polypeptide from the host cell. In illustrative examples, the nucleotide sequence encodes at least a biologically active portion of the sequences set forth in SEQ ID NO:2, 4, 6, 8, 10, 12, 14, 16, 18, 20, 22, 24, 26, 28, 30, 32, 34, 36, 38, or 78, or a variant thereof. Recombinant ADH polypeptides can be conveniently prepared using standard protocols as described for example in Sambrook, *et al.,* (1989, *supra*), in particular Sections 16 and 17; Ausubel *et al.,* (1994, *supra*), in particular Chapters 10 and 16; and Coligan et al., Current Protocols in Protein Science (John Wiley & Sons, Inc. 1995-1997), in particular Chapters 1, 5 and 6.

Exemplary nucleotide sequences that encode the ADH polypeptides of the application encompass full-length ADH genes as well as portions of the full-length or substantially full-length nucleotide sequences of the ADH genes or their transcripts or DNA copies of these transcripts. Portions of an ADH nucleotide sequence may encode polypeptide portions or segments that retain the biological activity of the native polypeptide. A portion of an ADH nucleotide sequence that encodes a biologically active fragment of an ADH polypeptide may encode at least about 20, 21, 22, 23, 24, 25, 30, 40, 50, 60, 70, 80, 90, 100, 120, 150, 300 or 400 contiguous amino acid residues, or almost up to the total number of amino acids present in a full-length ADH polypeptide.

The invention also contemplates variants of the ADH nucleotide sequences. Nucleic acid variants can be naturally-occurring, such as allelic variants (same locus), homologs (different locus), and orthologs (different organism) or can be non naturally-occurring. Naturally occurring variants such as these can be identified with the use of well-known molecular biology techniques, as, for example, with polymerase chain reaction (PCR) and hybridization techniques as known in the art. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of a reference ADH polypeptide. Variant nucleotide sequences also include synthetically derived nucleotide sequences, such as those generated, for example, by using site-directed mutagenesis but which still encode an ADH polypeptide. Generally, variants of a particular ADH nucleotide sequence will have at least about 30%, 40% 50%, 55%, 60%, 65%, 70%, generally at least about 75%, 80%, 85%, desirably about 90% to 95% or more, and more suitably about 98% or more sequence identity to that particular nucleotide sequence as determined by sequence alignment programs described elsewhere herein using default parameters.

ADH nucleotide sequences can be used to isolate corresponding sequences and alleles from other organisms, particularly other microorganisms. Methods are readily available in the art for the hybridization of nucleic acid sequences. Coding sequences from other organisms may be isolated according to well known techniques based on their sequence identity with the coding sequences set forth herein. In these techniques all or part of the known coding sequence is used as a probe which selectively hybridizes to other ADH-coding sequences present in a population of cloned genomic DNA fragments or cDNA fragments (*i.e.,* genomic or cDNA libraries) from a chosen organism (*e.g.,* a snake). Accordingly, the present invention also contemplates polynucleotides that hybridize to reference ADH nucleotide sequences, or to their complements, under stringency conditions described below. As used herein, the term "hybridizes under low stringency, medium stringency, high stringency, or very high stringency conditions" describes conditions for hybridization and washing. Guidance for performing hybridization reactions can be found in *Ausubel et al.,* (1998, *supra),* Sections 6.3.1-6.3.6. Aqueous and nonaqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M NaHPO$_4$ (pH 7.2), 7% SDS for hybridization at 65° C, and (i) 2 x SSC, 0.1% SDS; or (ii) 0.5% BSA, 1 mM EDTA, 40 mM NaHPO$_4$ (pH 7.2), 5% SDS for washing at room temperature. One embodiment of low stringency conditions includes hybridization in 6 x sodium chloride/sodium citrate (SSC) at about 45°C, followed by two washes in 0.2 x SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M NaHPO$_4$ (pH 7.2), 7% SDS for hybridization at 65° C, and (i) 2 x SSC, 0.1% SDS; or (ii) 0.5% BSA, 1 mM EDTA, 40 mM NaHPO$_4$ (pH 7.2), 5% SDS for washing at 60-65° C. One embodiment of medium stringency conditions includes hybridizing in 6 x SSC at about 45°C, followed by one or more washes in 0.2 x SSC, 0.1% SDS at 60°C. High stringency conditions include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M NaHPO$_4$ (pH 7.2), 7% SDS for hybridization at 65° C, and (i) 0.2 x SSC, 0.1% SDS; or (ii) 0.5% BSA, 1 mM EDTA, 40 mM NaHPO$_4$ (pH 7.2), 1 % SDS for washing at a temperature in excess of 65° C. One embodiment of high stringency conditions includes hybridizing in 6 x SSC at about 45°C, followed by one or more washes in 0.2 x SSC, 0.1% SDS at 65° C.

In certain embodiments, an ADH polypeptide is encoded by a polynucleotide that hybridizes to a disclosed nucleotide sequence under very high stringency conditions. One embodiment of very high stringency conditions includes hybridizing 0.5 M sodium phosphate, 7% SDS at 65° C, followed by one or more washes at 0.2 x SSC, 1% SDS at 65° C.

Other stringency conditions are well known in the art and a skilled addressee will recognize that various factors can be manipulated to optimize the specificity of the hybridization. Optimization of the stringency of the final washes can serve to ensure a high degree of hybridization. For detailed examples, see Ausubel *et al., supra* at pages 2.10.1 to 2.10.16 and Sambrook *et al.* (1989, *supra*) at sections 1.101 to 1.104.

While stringent washes are typically carried out at temperatures from about 42° C to 68° C, one skilled in the art will appreciate that other temperatures may be suitable for stringent conditions. Maximum hybridization rate typically occurs at about 20° C to 25° C below the $T_m$ for formation of a DNA-DNA hybrid. It is well known in the art that the $T_m$ is the melting temperature, or temperature at which two complementary polynucleotide sequences dissociate. Methods for estimating $T_m$ are well known in the art (see Ausubel *et al., supra* at page 2.10.8). In general, the $T_m$ of a perfectly matched duplex of DNA may be predicted as an approximation by the formula:

$$T_m = 81.5 + 16.6 \, (\log_{10} M) + 0.41 \, (\%G{+}C) - 0.63 \, (\% \text{ formamide}) - (600/\text{length})$$

wherein: M is the concentration of Na$^+$, preferably in the range of 0.01 molar to 0.4 molar; %G+C is the sum of guanosine and cytosine bases as a percentage of the total number of bases, within the range between 30% and 75% G+C; % formamide is the percent formamide concentration by volume; length is the number of base pairs in the DNA

duplex. The $T_m$ of a duplex DNA decreases by approximately 1° C with every increase of 1% in the number of randomly mismatched base pairs. Washing is generally carried out at $T_m$ - 15° C for high stringency, or $T_m$ - 30° C for moderate stringency.

In one example of a hybridization procedure, a membrane (e.g., a nitrocellulose membrane or a nylon membrane) containing immobilized DNA is hybridized overnight at 42° C in a hybridization buffer (50% deionized formamide, 5 × SSC, 5 × Denhardt's solution (0.1% ficoll, 0.1% polyvinylpyrollidone and 0.1% bovine serum albumin), 0.1 % SDS and 200 mg/mL denatured salmon sperm DNA) containing labeled probe. The membrane is then subjected to two sequential medium stringency washes (i.e., 2 × SSC, 0.1% SDS for 15 min at 45° C, followed by 2 × SSC, 0.1% SDS for 15 min at 50° C), followed by two sequential higher stringency washes (i.e., 0.2 × SSC, 0.1% SDS for 12 min at 55° C followed by 0.2 × SSC and 0.1% SDS solution for 12 min at 65-68° C.

Embodiments of the present invention also include the use of ADH chimeric or fusion proteins for converting a polysaccharide or oligosaccharide to a suitable monosaccharide or a suitable oligosaccharide. As used herein, an ADH "chimeric protein" or "fusion protein" includes an ADH polypeptide linked to a non-ADH polypeptide. A "non-ADH polypeptide" refers to a polypeptide having an amino acid sequence corresponding to a protein which is different from the ADH protein and which is derived from the same or a different organism. The ADH polypeptide of the fusion protein can correspond to all or a portion *e.g.,* a fragment described herein of an ADH amino acid sequence. In a preferred embodiment, an ADH fusion protein includes at least one (or two) biologically active portion of an ADH protein. The non-ADH polypeptide can be fused to the N-terminus or C-terminus of the ADH polypeptide.

The fusion protein can include a moiety which has a high affinity for a ligand. For example, the fusion protein can be a GST-ADH fusion protein in which the ADH sequences are fused to the C-terminus of the GST sequences. Such fusion proteins can facilitate the purification of recombinant ADH polypeptide. Alternatively, the fusion protein can be a ADH protein containing a heterologous signal sequence at its N-terminus. In certain host cells, expression and/or secretion of ADH proteins can be increased through use of a heterologous signal sequence.

In certain embodiments, the ADH molecules of the present invention may be employed in microbial systems or isolated/recombinant microorganisms to convert polysaccharides and oligosaccharides from biomass, such as alginate, to suitable monosaccharides or suitable oligosaccharides, such as 2-keto-3-deoxy-D-gluconate-6-phosphate (KDG), which may be further converted to commodity chemicals, such as biofuels.

By way of background, large-scale aquatic-farming can generate a significant amount of biomass without replacing food crop production with energy crop production, deforestation, and recultivating currently uncultivated land, as most of hydrosphere including oceans, rivers, and lakes remains untapped. As one example, the Pacific coast of North America is abundant in minerals necessary for large-scale aqua-farming. Giant kelp, which lives in the area, grows as fast as 1m/day, the fastest among plants on earth, and grows up to 50 m. Additionally, aqua-farming has other benefits including the prevention of a red tide outbreak and the creation of a fish-friendly environment.

In contrast to lignocellulolic biomass, aquatic biomass is easy to degrade. Aquatic biomass lacks lignin and is significantly more fragile than lignocellulolic biomass and can thus be easily degraded using either enzymes or chemical catalysts (*e.g.,* formate). Seaweed may be easily converted to monosaccharides using either enzymes or chemical catalysis, as seaweed has significantly simpler major sugar components (Alginate: 30%, Mannitol: 15%) as compared to lignocellulose (Glucose: 24.1-39%, Mannose: 0.2-4.6%, Galactose: 0.5-2.4%, Xylose: 0.4-22.1 %, Arabinose 1.5-2.8%, and Uronates: 1.2-20.7%, and total sugar contents are corresponding to 36.5-70% of dried weight). Saccharification and fermentation using aquatic biomass such as seaweed is much easier than using lignocellulose.

*n*-alkanes, for example, are major components of all oil products including gasoline, diesels, kerosene, and heavy oils. Microbial systems or recombinant microorganisms may be used to produce *n*-alkanes with different carbon lengths ranging, for example, from C7 to over C20: C7 for gasoline (*e.g.,* motor vehicles), C10-C15 for diesels (*e.g.,* motor vehicles, trains, and ships), and C8-C16 for kerosene (*e.g.,* aviations and ships), and for all heavy oils.

Medium and cyclic alcohols may also substitute for gasoline and diesels. For example, medium and cyclic alcohols have a higher oxygen content that reduces carbon monoxide (CO) emission, they have higher octane number that reduces engine knock, upgrades the quality of many lower grade U.S. crude oil products, and substitute harmful aromatic octane enhancers (e.g. benzene), have an energy density comparable to that of gasoline, their immiscibility significantly reduces the capitol expenditure, a lower latent heat of vaporization is favored for cold starting, and 4-octanol is significantly less toxic compared to ethanol and butanol.

As an early step in converting marine biomass to commodity chemicals such as biofuels, a microbial system or recombinant microorganism that is able to grow using a polysaccharide (*e.g.,* alginate) as a source of carbon and energy may be employed. Merely by way of explanation, approximately 50 percent of seaweed dry-weight comprises various sugar components, among which alginate and mannitol are major components corresponding to 30 and 15 percent of seaweed dry-weight, respectively. Although microorganisms such as *E. coli* are generally considered as a host organisms in synthetic biology, such microorganism are able to metabolize mannitol, but they completely lack the ability to degrade and metabolize alginate. Embodiments of the present application include microorganisms such as *E. coli,* which microorganisms contain ADH molecules of the present application, that are capable of using polysaccharides such as alginate as a source of carbon and energy.

A microbial system able to degrade or depolymerize alginate (a major component of aquatic or marine-sphere biomass) and to use it as a source of carbon and energy may incorporate a set of aquatic or marine biomass-degrading enzymes (*e.g.,* polysaccharide degrading or depolymerizing enzymes such as alginate lyases (ALs)), to the microbial system. Merely by way of explanation, alginate is a block co-polymer of β-D-mannuronate (M) and α-D-gluronate (G) (M and G are epimeric about the C5-carboxyl group). Each alginate polymer comprises regions of all M (polyM), all G (polyG), and/or the mixture of M and G (polyMG). ALs are mainly classified into two distinctive subfamilies depending on their acts of catalysis: endo-(EC 4.2.2.3) and exo-acting (EC 4.2.2.-) ALs. Endo-acting ALs are further classified based on their catalytic specificity; M specific and G specific ALs. The endo-acting ALs randomly cleave alginate via a β-elimination mechanism and mainly depolymerize alginate to di-, tri- and tetrasaccharides. The uronate at the nonreducing terminus of each oligosaccharide are converted to unsaturated sugar uronate, 4-deoxy-L-erythro-hex-4-ene pyranosyl uronate. The exo-acting ALs catalyze further depolymerization of these oligosaccharides and release unsaturated monosaccharides, which may be non-enzymatically converted to monosaccharides, including uronate, 4-deoxy-L-erythro-5-hexoseulose uronate (DEHU). Certain embodiments of a microbial system or isolated microorganism may include endoM-, endoG- and exo-acting ALs to degrade or depolymerize aquatic or marine-biomass polysaccharides such as alginate to a monosaccharide such as DEHU.

Alginate lyases may depolymerize alginate to monosaccharides (e.g., DEGU) in the cytosol, or may be secreted to depolymerize alginate in the media. When alginate is depolymerized in the media, certain embodiments may include a microbial system or isolated microorganism that is able to transport monosaccharides (e.g., DEHU) from the media to the cytosol to efficiently utilize these monosaccharides as a source of carbon and energy. Merely by way of one example, genes encoding monosaccharide permeases such as DEHU permeases may be isolated from bacteria that grow on polysaccharides such as alginate as a source of carbon and energy, and may be incorporated into embodiments of the present microbial system or isolated microorganism. By way of additional example, embodiments may also include redesigned native permeases with altered specificity for monosaccharide (e.g., DEHU) transportation.

Certain embodiments of a microbial system or an isolated microorganism may incorporate genes encoding ADH polypeptides, or variants thereoef, as disclosed herein, in which the microbial system or microorganisms may be growing on polysaccharides such as alginate as a source of carbon and energy. Certain embodiments include a microbial system or isolated microorganism comprising ADH polypeptides, such as ADH polypeptides having DEHU dehyodrogenase activity, in which various monosaccharides, such as DEHU, may be reduced to a monosaccharide suitable for biofuel biosynthesis, such as 2-keto-3-deoxy-D-gluconate-6-phosphate (KDG) or D-mannitol.

In other embodiments, aquatic or marine-biomass polysaccharides such as alginate may be chemically degraded using chemical catalysts such as acids. Merely by way of explanation, the reaction catalyzed by chemical catalysts is hydrolysis rather than β-elimination catalyzed by enzymatic catalysts. Acid catalysts cleave glycosidic bonds via hydrolysis, release oligosaccharides, and further depolymerize these oligosaccharides to unsaturated monosaccharides, which are often converted to D-Mannuronate. Certain embodiments may include boiling alginate with strong mineral acids, which may liberate carbon dioxide from D-mannuronate and form D-lyxose, which is a common sugar used by many microbes. Certain embodiments may use, for example, formate, hydrochloric acid, sulfuric acid, and other suitable acids known in the art as chemical catalysts.

Certain embodiments may use variations of chemical catalysis similar to those described herein or known to a person skilled in the art, including improved or redesigned methods of chemical catalysis suitable for use with aquatic or marine-biomass related polysaccharides. Certain embodiments include those wherein the resulting monosaccharide uronate is D-mannuronate.

A microbial system or isolated microorganism according to certain embodiments of the present invention may also comprise permeases that catalyze the transport of monosaccharides (e.g., D-mannuronate and D-lyxose) from media to the microbial system. Merely by way of example, the genes encoding the permeases of D-mannuronate in soil *Aeromonas* may be incorporated into a microbial system as described herein.

As one alternative example, a microbial system or microorganism may comprise native permeases that are redesigned to alter their specificity for efficient monosaccharide transportation, such as for D-mannuronate and D-lyxose transportation. For example, *E.coli* contains several permeases that are able to transport monosaccharides or sugars such as D-mannuronate and D-lyxose, including KdgT for 2-keto-3-deoxy-D-gluconate (KDG) transporter, ExuT for aldohexuronates such as D-galacturonate and D-glucuronate transporter, GntPTU for gluconate/fructuronate transporter, uidB for glucuronide transporter, fucP for L-fucose transporter, galP for galactose transporter, yghK for glycolate transporter, dgoT for D-galactonate transporter, uhpT for hexose phosphate transporter, dctA for orotate/citrate transporter, gntUT for gluconate transporter, malEGF for maltose transporter: alsABC for D-allose transporter, idnT for L-idonate/D-gluconate transporter, KgtP for proton-driven α-ketoglutarate transporter, lacY for lactose/galactose transporter, xylEFGH for D-xylose transporter, araEFGH for L-arabinose transporter, and rbsABC for D-ribose transporter. In certain embodiments, a microbial system or isolated microorganism may comprise permeases as described above that are redesigned for transporting certain monosaccharides such as D-mannuronate and D-lyxose.

Certain embodiments may include a microbial system or isolated microorganism efficiently growing on monosaccharides such as D-mannuronate or D-lyxose as a source of carbon and energy, and include microbial systems or microorganisms comprising ADH molecules of the present application, including ADH polypeptides having a D-mannonurate dehydrogenase activity.

Certain embodiments may include a microbial system or isolated microorganism with enhanced efficiency for converting monosaccharides such as DEHU, D-mannuronate and D-xylulose into monosaccharides suitable for a biofuel biosynthesis pathway such as KDG. Merely by way of explanation, D-mannuronate and D-xylulose are metabolites in microbes such as *E. coli*. D-mannuronate is converted by a D-mannuronate dehydratase to KDG. D-xylulose enters the pentose phosphate pathway. In certain embodiments, D-mannuronate dehydratase (uxuA) may be over expressed. In other embodiments, suitable genes such as kgdK, nad, and kdgA may be overexpressed as well.

Certain embodiments of the present invention may also include methods for converting a polysaccharide to a suitable monosaccharide or oligosaccharide, comprising contacting the polysaccharide with a microbial system, wherein the microbial system comprises a microorganism, and wherein the microorganism comprises an ADH polynucleotide according to the present disclosure, wherein the ADH polynucleotide encodes an ADH polypeptide having a hydrogenase activity, such as an alcohol dehydrogenase activity, a DEHU hydrogenase activity, and/or a D-mannuronate hydrogenase activity.

Additional embodiments include methods for catalyzing the reduction (hydrogenation) of D-mannuronate, comprising contacting D-mannuronate with a microbial system, wherein the microbial system comprises a microorganism, and wherein the microorganism comprises an ADH polynucleotide according to the present disclosure.

Additional embodiments include methods for catalyzing the reduction (hydrogenation) of (DEHU), comprising contacting DEHU with a microbial system, wherein the microbial system comprises a microorganism, and wherein the microorganism comprises an ADH polynucleotide according to the present disclosure.

Additional embodiments include a vector comprising an isolated polynucleotide, and may include such a vector wherein the isolated polynucleotide is operably linked to an expression control region, and wherein the polynucleotide encodes an ADH polypeptide having a hydrogenase activity, such as an alcohol dehydrogenase activity, a DEHU hydrogenase activity, and/or a D-mannuronate hydrogenase activity.

Additional embodiments include methods for converting a polysaccharide to a suitable monosaccharide or oligosaccharide, comprising contacting the polysaccharide with a microbial system, wherein the microbial system comprises a microorganism, and wherein the microorganism comprises an ADH polypeptide according to the present disclosure.

Additional embodiments include methods for catalyzing the reduction (hydrogenation) of D-mannuronate, comprising contacting D-mannuronate with a microbial system, wherein the microbial system comprises a microorganism, and wherein the microorganism comprises an ADH polypeptide according to the present disclosure.

Additional embodiments include methods for catalyzing the reduction (hydrogenation) of uronate, 4-deoxy-L-erythro-5-hexoseulose uronate (DEHU), comprising contacting DEHU with a microbial system, wherein the microbial system comprises a microorganism, and wherein the microorganism comprises an ADH polypeptide according to the present disclosure.

Additional embodiments include microbial systems for converting a polysaccharide to a suitable monosaccharide or oligosaccharide, wherein the microbial system comprises a microorganism, and wherein the microorganism comprises an isolated polynucleotide selected from

(a) an isolated polynucleotide comprising a nucleotide sequence at least 80% identical to the nucleotide sequence set forth in SEQ ID NO:1, 3, 5, 7, 9, 11, 13, 15, 17, 19, 21, 23, 25, 27, 29, 31, 33, 35, or 37;

(b) an isolated polynucleotide comprising a nucleotide sequence at least 90% identical to the nucleotide sequence set forth in SEQ ID NO:1, 3, 5, 7, 9, 11, 13, 15, 17, 19, 21, 23, 25, 27, 29, 31, 33, 35 or 37;

(c) an isolated polynucleotide comprising a nucleotide sequence at least 95% identical to the nucleotide sequence set forth in SEQ ID NO:1, 3, 5, 7, 9, 11, 13, 15, 17, 19, 21, 23, 25, 27, 29, 31, 33, 35, or 37;

(d) an isolated polynucleotide comprising a nucleotide sequence at least 97% identical to the nucleotide sequence set forth in SEQ ID NO:1, 3, 5, 7, 9, 11, 13, 15, 17, 19, 21, 23, 25, 27, 29, 31, 33, 35, or 37;

(e) an isolated polynucleotide comprising a nucleotide sequence at least 99% identical to the nucleotide sequence set forth in SEQ ID NO:1, 3, 5, 7, 9, 11, 13, 15, 17, 19, 21, 23, 25, 27, 29, 31, 33, 35, or 37; and

(f) an isolated polynucleotide comprising the nucleotide sequence set forth in SEQ ID NO:1, 3, 5, 7, 9, 11, 13, 15, 17, 19, 21, 23, 25, 27, 29, 31, 33, 35, or 37.

Additional embodiments include microbial systems for converting a polysaccharide to a suitable monosaccharide or oligosaccharide, wherein the microbial system comprises a microorganism, and wherein the microorganism comprises an isolated polypeptide selected from

(a) an isolated polypeptide comprising an amino acid sequence at least 80% identical to the amino acid sequence set forth in SEQ ID NO:2, 4, 6, 8, 10, 12, 14, 16, 18, 20, 22, 24, 26, 28, 30, 32, 34, 36, 38, or 78;

(b) an isolated polypeptide comprising an amino acid sequence at least 90% identical to the amino acid sequence set forth in SEQ ID NO:2, 4, 6, 8, 10, 12, 14, 16, 18, 20, 22, 24, 26, 28, 30, 32, 34, 36, 38, or 78;

(c) an isolated polypeptide comprising an amino acid sequence at least 95% identical to the amino acid sequence set forth in SEQ ID NO:2, 4, 6, 8, 10, 12, 14, 16, 18, 20, 22, 24, 26, 28, 30, 32, 34, 36, 38, or 78;

(d) an isolated polypeptide comprising an amino acid sequence at least 97% identical to the amino acid sequence set forth in SEQ ID NO:2, 4, 6, 8, 10, 12, 14, 16, 18, 20, 22, 24, 26, 28, 30, 32, 34, 36, 38, or 78;

(e) an isolated polypeptide comprising an amino acid sequence at least 99% identical to the amino acid sequence set forth in SEQ ID NO:2, 4, 6, 8, 10, 12, 14, 16, 18, 20, 22, 24, 26, 28, 30, 32, 34, 36, 38, or 78; and

(e) an isolated polypeptide comprising the amino acid sequence set forth in SEQ ID NO:2, 4, 6, 8, 10, 12, 14, 16, 18, 20, 22, 24, 26, 28, 30, 32, 34, 36, 38, or 78.

In certain embodiments, the microbial system comprises a recombinant microorganism, wherein the recombinant microorganism comprises the vectors, polynucleotides, and/or polypeptides as described herein. Given its rapid growth rate, well-understood genetics, the variety of available genetic tools, and its capability in producing heterologous proteins, genetically modified *E. coli* may be used in certain embodiments of a microbial system as described herein, whether for degradation of a polysaccharide, such as alginate, or formation or biosynthesis of biofuels. Other microorganisms may be used according to the present description, based in part on the compatibility of enzymes and metabolites to host organisms. For example, other microorganisms such as *Acetobacter aceti, Achromobacter, Acidiphilium, Acinetobacter, Actinomadura, Actinoplanes, Aeropyrum pernix, Agrobacterium, Alcaligenes, Ananas comosus (M), Arthrobacter, Aspargillus niger, Aspargillus oryze, Aspergillus melleus, Aspergillus pulverulentus, Aspergillus saitoi, Aspergillus sojea, Aspergillus usamii, Bacillus alcalophilus, Bacillus amyloliquefaciens, Bacillus brevis, Bacillus circulans, Bacillus clausii, Bacillus lentus, Bacillus licheniformis, Bacillus macerans, Bacillus stearothermophilus, Bacillus subtilis, Bifidobacterium, Brevibacillus brevis, Burkholderia cepacia, Candida cylindracea, Candida rugosa, Carica papaya (L), Cellulosimicrobium, Cephalosporium, Chaetomium erraticum, Chaetomium gracile, Clostridium, Clostridium butyricum, Clostridium acetobutylicum, Clostridium thermocellum, Corynebacterium (glutamicum), Corynebacterium efficiens, Escherichia coli, Enterococcus, Erwina chrysanthemi, Gliconobacter, Gluconacetobacter, Haloarcula, Humicola insolens, Humicola nsolens, Kitasatospora setae, Klebsiella, Klebsiella oxytoca, Kluyveromyces, Kluyveromyces fragilis, Kluyveromyces lactis, Kocuria, Lactlactis, Lactobacillus, Lactobacillus fermentum, Lactobacillus sake, Lactococcus, Lactococcus lactis, Leuconostoc, Methylocystis, Methanolobus siciliae, Methanogenium organophilum, Methanobacterium bryantii, Microbacterium imperiale, Micrococcus lysodeikticus, Microlunatus, Mucor javanicus, Mycobacterium, Myrothecium, Nitrobacter, Nitrosomonas, Nocardia, Papaya carica, Pediococcus, Pediococcus halophilus, Penicillium, Penicillium camemberti, Penicillium citrinum, Penicillium emersonii, Penicillium roqueforti, Penicillum lilactinum, Penicillum multicolor, Paracoccus pantotrophus, Propionibacterium, Pseudomonas, Pseudomonas fluorescens.*

*Pseudomonas denitrificans, Pyrococcus, Pyrococcus furiosus, Pyrococcus horikoshii, Rhizobium, Rhizomucor miehei, Rhizomucor pusillus Lindt, Rhizopus, Rhizopus delemar, Rhizopus japonicus, Rhizopus niveus, Rhizopus oryzae, Rhizopus oligosporus, Rhodococcus, Saccharomyces cerevisiae, Sclerotina libertina, Sphingobacterium multivorum, Sphingobium, Sphingomonas, Streptococcus, Streptococcus thermophilus Y-1, Streptomyces, Streptomyces griseus, Streptomyces lividans, Streptomyces murinus, Streptomyces rubiginosus, Streptomyces violaceoruber, Streptoverticillium mobaraense, Tetragenococcus, Thermus, Thiosphaera pantotropha, Trametes, Trichoderma, Trichoderma longibrachiatum, Trichoderma reesei, Trichoderma viride, Trichosporon penicillatum, Vibrio alginolyticus, Xanthomonas, yeast, Zygosaccharomyces rouxii, Zymomonas,* and *Zymomonus mobilis,* and the like may be used according to the present invention.

In order that the invention may be readily understood and put into practical effect, particular preferred embodiments will now be described by way of the following non-limiting examples.

EXAMPLES

EXAMPLE 1

CLONING OF ALCOHOL DEHYDROGENASES

All chemicals and enzymes were purchased from Sigma-Aldrich, Co. and New England Biolabs, Inc., respectively, unless otherwise stated. Since mannitol 1-dehydrogenase (MTDH) catalyzes a similar reaction to DEHU hydrogenase, primers were designed using the amino acid sequences MTDHs derived from *Apium graveolens* and *Arabidopsis thaliana*. Using these primers as queries (*see* Table 1), homogeneous gene sequences were searched in the genome sequence of *Agrobacterium tumefaciens* C58. Approximately 16 genes encoding zinc-dependent alcohol dehydrogenases were found. Among these genes, top 10 gene sequences with high E-value were amplified by PCR : 98°C for 10 sec, 55°C for 15 sec, and 72°C for 60 sec, repeated for 30 times. The reaction mixture contained 1 x Phusion buffer, 2 mM dNTP, 0.5 $\mu$M forward and reverse primers (listed in the table 1), 2.5 U Phusion DNA polymerase (Finezyme), and an aliquot of *Agrobacterium tumefaciens* C58 cells as a template in total volume of 100 $\mu$l. As the ADH1 and ADH4 had internal NdeI site, and ADH3 had BamHI site, these genes were amplified using over-lap PCR method using the above PCR protocols. The forward (5'-GCGGCCTCGGCCACATGGCCGTCAAGC-3') (SEQ ID NO:39) and reverse (5'-GCTTGACGGCCATGTGGCCGAGGCCGC-3') (SEQ ID NO:40) primers were used to delete NdeI site from ADH1. The forward (5'-TGGCAATACCGGACCCCGGCCCCGGTG -3') (SEQ ID NO:41) and reverse (5'-CACCGGGGCCGGGGTCCGGTATTGCCA -3') (SEQ ID NO:42) primers were used to delete BamHI site from ADH3. The forward (5'-AGGCAACCGAGGCGTATGAGCGGCTAT -3') (SEQ ID NO:43) and reverse (5'-ATAGCCGCTCATACGCCTCGGTTGCCT -3') (SEQ ID NO:44) primers were used to delete NdeI site from ADH4. These amplified fragments were digested with NdeI and BamHI and ligated into pET29 pre-digested with the same enzymes using T4 DNA ligase to form 10 different plasmids, pETADH1 through pETADH10. The constructed plasmids were sequenced (Elim Biophamaceuticals) and the DNA sequences of these inserts were confirmed.

All plasmids were transformed into *Escherichia coli* strain BL21(DE3). The single colonies of BL21(DE3) containing respective alcohol dehydrogenase (ADH) genes were inoculated into 50 ml of LB media containing 50 $\mu$g/ml kanamycin (Km[50]). These strains were grown in an orbital shaker with 200 rpm at 37 °C. The 0.2 mM IPTG was added to each culture when the $OD_{600nm}$ reached 0.6, and the induced culture was grown in an orbital shaker with 200 rpm at 20 °C. 24 hours after the induction, the cells were harvested by centrifugation at 4,000 rpm x g for 10 min and the pellet was resuspended into 2 ml of Bugbuster (Novagen) containing 10 $\mu$l of Lysonase™ Bioprocessing Reagent (Novagen). The solution was again centrifuged at 4,000 rpm x g for 10 min and the supernatant was obtained.

Table 1. Primers used for the amplification of ADH

| Ref# | Name | Forward Primer (5' -> 3') | Reverse Primer (5' -> 3') |
|---|---|---|---|
| NP_532245.1 | ADH1 | GGAATTCCATATGTTCACAACGTCCGCCTA (SEQ ID NO:47) | CGGGATCCTTAGGCGGCCTTCTGGCGCG (SEQ ID NO:48) |
| NP_532698.1 | ADH2 | GGAATTCCATATGGCTATTGCAAGAGGTTA (SEQ ID NO:49) | CGGGATCCTTAAGCGTCGAGCGAGGCCA (SEQ ID NO:50) |
| NP_531326.1 | ADH3 | GGAATTCCATATGACTAAAACAATGAAGGC (SEQ ID NO:51) | CGGGATCCTTAGGCGGCGAGATCCACGA (SEQ ID NO:52) |
| NP_535613.1 | ADH4 | GGAATTCCATATGACCGGGGCGAACCAGCC (SEQ ID NO:53) | CGGGATCCTTAAGCGCCGTGCGGAAGGA (SEQ ID NO:54) |
| NP_533663.1 | ADH5 | GGAATTCCATATGACCATGCATGCCATTCA (SEQ ID NO:55) | CGGGATCCTTATTCGGCTGCAAATTGCA (SEQ ID NO:56) |
| NP_532825.1 | ADH6 | GGAATTCCATATGCGCGCGCTTTATTACGA (SEQ ID NO:57) | CGGGATCCTTATTCGAACCGGTCGATGA (SEQ ID NO:58) |
| NP_533479.1 | ADH7 | GGAATTCCATATGCTGGCGATTTTCTGTGA (SEQ ID NO:59) | CGGGATCCTTATGCGACCTCCACCATGC (SEQ ID NO:60) |
| NP_535818.1 | ADH8 | GGAATTCCATATGAAAGCCTTCGTCGTCGA (SEQ ID NO:61) | CGGGATCCTTAGGATGCGTATGTAACCA (SEQ ID NO:62) |
| NP_534572.1 | ADH9 | GGAATTCCATATGAAAGCGATTGTCGCCCA (SEQ ID NO:63) | CGGGATCCTTAGGAAAAGGCGATCTGCA (SEQ ID NO:64) |
| NP_534767.1 | ADH10 | GGAATTCCATATGCCGATGGCGCTCGGGCA (SEQ ID NO:65) | CGGGATCCTTAGAATTCGATGACTTGCC (SEQ ID NO:66) |
| NP_535575.1 | ADH11 | - | - |
| NP_532098.1 | ADH12 | - | - |
| NP_535348.1 | ADH13 | - | - |
| NP_532354.1 | ADH14 | - | - |
| NP_535561.1 | ADH15 | - | - |
| NP_532255.1 | ADH16 | - | - |
| NP_534796.1 | ADH17 | - | - |
| NP_532090.1 | ADH18 | - | - |
| NP_531523.1 | ADH19 | - | - |

EXAMPLE 2

CHARACTERIZATION OF ALCOHOL DEHYDROGENASES

**Preparation of oligoalginate lyase Atu3025 derived from *Agrobacterium tumefaciens* C58.** pETAtu3025 was constructed based on pET29 plasmd backbone (Novagen). The oligoalginate lyase Atu3025 was amplified by PCR: 98°C for 10 sec, 55°C for 15 sec, and 72°C for 60 sec, repeated for 30 times. The reaction mixture contained 1 x Phusion buffer, 2 mM dNTP, 0.5 $\mu$M forward (5'-GGAATTCCATATGCGTCCCTCTGCCCCGGCC-3') (SEQ ID NO:45) and reverse (5'- CGGGATCCTTAGAACTGCTTGGGAAGGGAG-3') (SEQ ID NO:46) primers, 2.5 U Phusion DNA polymerase (Finezyme), and an aliquot of *Agrobacterium tumefaciens* C58 (gift from Professor Eugene Nester, University of Washington) cells as a template in total volume of 100 $\mu$l. The amplified fragment was digested with NdeI and BamHI and ligated into pET29 pre-digested with the same enzymes using T4 DNA ligase to form pETAtu3025. The constructed plasmid was sequenced (Elim Biophamaceuticals) and the DNA sequence of the insert was confirmed.

The pETAtu3025 was transformed into *Escherichia coli* strain BL21(DE3). The single colony of BL21(DE3) containing pETAtu3025 was inoculated into 50 ml of LB media containing 50 $\mu$g/ml kanamycin (Km$^{50}$). This strain was grown in an orbital shaker with 200 rpm at 37 °C. The 0.2 mM IPTG was added to the culture when the $OD_{600nm}$ reached 0.6, and the induced culture was grown in an orbital shaker with 200 rpm at 20 °C. 24 hours after the induction, the cells were harvested by centrifugation at 4,000 rpm x g for 10 min and the pellet was resuspended into 2 ml of Bugbuster (Novagen) containing 10 $\mu$l of Lysonase™ Bioprocessing Reagent (Novagen). The solution was again centrifuged at 4,000 rpm x g for 10 min and the supernatant was obtained.

**Preparation of ~2% DEHU solution.** DEHU solution was enzymatically prepared. The 2 % alginate solution was prepared by adding 10 g of low viscosity alginate into the 500 ml of 20 mM Tris-HCl (pH7.5) solution. An approximately 10 mg of alginate lyase derived from *Flavobacterium sp.* (purchased from Sigma-aldrich) was added to the alginate solution. 250 ml of this solution was then transferred to another bottle and the *E. coli* cell lysate containing Atu3025 prepared above section was added. The alginate degradation was carried out at room temperature over night. The resulting products were analyzed by thin layer chromatography, and DEHU formation was confirmed.

**Preparation of D-mannuronate solution.** D-mannuronate solution was chemically prepared based on the protocol previously described by Spoehr (Archive of Biochemistry, 14: pp153-155). Fifty milligram of alginate was dissolved into 800 $\mu$L of ninety percent formate. This solution was incubated at 100 °C for over night. Formate was then evaporated and the residual substances were washed with absolute ethanol twice. The residual substance was again dissolved into absolute ethanol and filtrated. Ethanol was evaporated and residual substances were resuspended into 20 mL of 20 mM Tris-HCl (pH 8.0) and the solution was filtrated to make a D-mannuronate solution. This D-mannuronate solution was diluted 5-fold and used for assay.

**Assay for DEHU hydrogenase.** To identify DEHU hydrogenase, we carried out NADPH dependent DEHU hydrogenation assay. 20 $\mu$l of prepared cell lysate containing each ADH was added to 160 $\mu$l of 20-fold deluted DEHU solution prepared in the above section. 20 $\mu$l of 2.5 mg/ml of NADPH solution (20 mM Tris-HCl, pH 8.0) was added to initiate the hydrogenation reaction, as a preliminary study using cell lysate of *A. tumefaciens* C58 have shown that DEHU hydrogenation requires NADPH as a co-factor. The consumption of NADPH was monitored an absorbance at 340 nm for 30 min using the kinetic mode of ThermoMAX 96 well plate reader (Molecular Devises). *E. coli* cell lysate containing alcohol dehydrogenase (ADH) 10 lacking a portion of N-terminal domain was used in a control reaction mixture.

**Assay for D-mannuronate hydrogenase.** To identify D-mannuronate hydrogenase, we carried out NADPH dependent D-mannuronate hydrogenation assay. 20 $\mu$l of prepared cell lysate containing each ADH was added to 160 $\mu$l of D-mannuronate solution prepared in the above section. 20 $\mu$l of 2.5 mg/ml of NADPH solution (20 mM Tris-HCl, pH 8.0) was added to initiate the hydrogenation reaction. The consumption of NADPH was monitored an absorbance at 340 nm for 30 min using the kinetic mode of ThermoMAX 96 well plate reader (Molecular Devises). *E. coli* cell lysate containing alcohol dehydrogenase (ADH) 10 lacking a portion of N-terminal domain was used in a control reaction mixture.

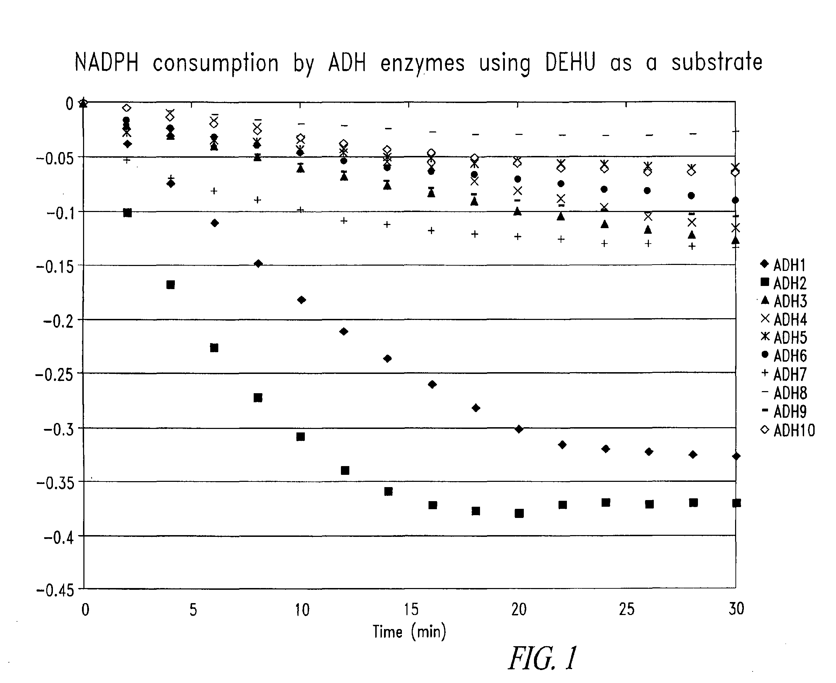

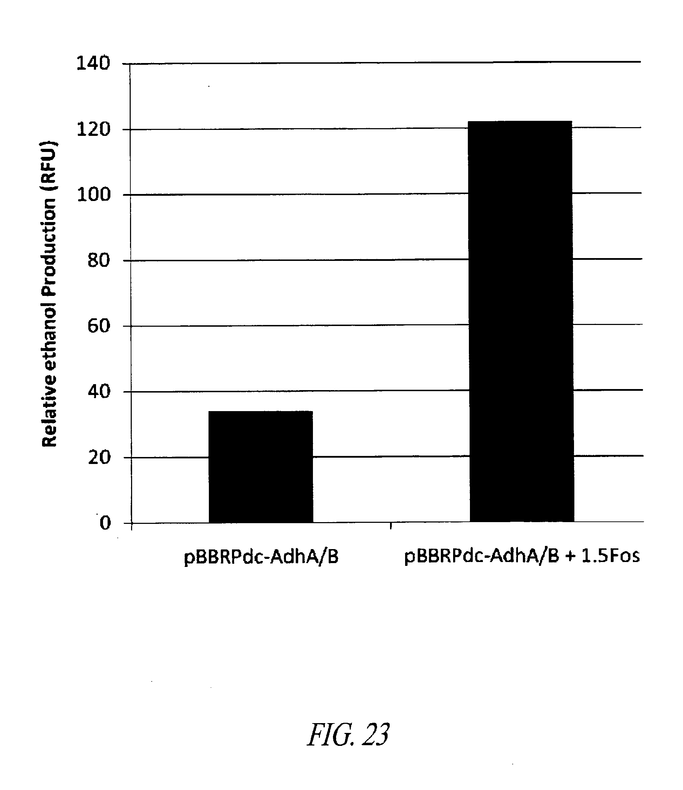

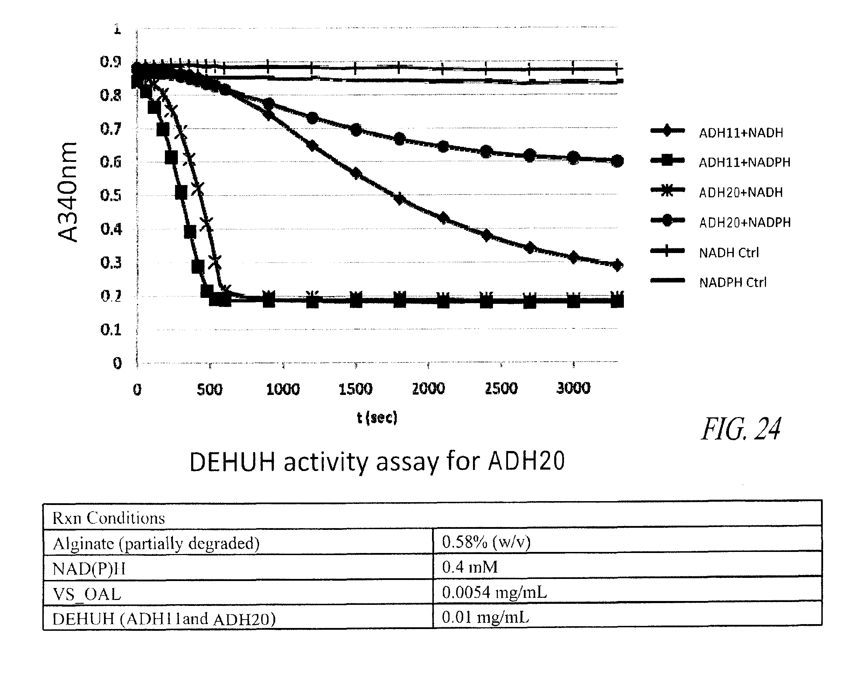

The results are shown in Figure 1, Figure 2, and Figure 24. ADH1 and ADH2 showed remarkably higher DEHU hydrogenation activity compared to other hydrogenases (Figure 1). In addition, ADH3, ADH4, and ADH9 showed remarkably higher D-mannuronate hydrogenation activity compared to other hydrogenases (Figure 2). ADH11 and ADH20 also show significant DEHU hydrogenation activity (Figure 23).

EXAMPLE 3

ENGINEERING *E. COLI* TO GROW ON ALGINATE AS A SOLE SOURCE OF CARBON

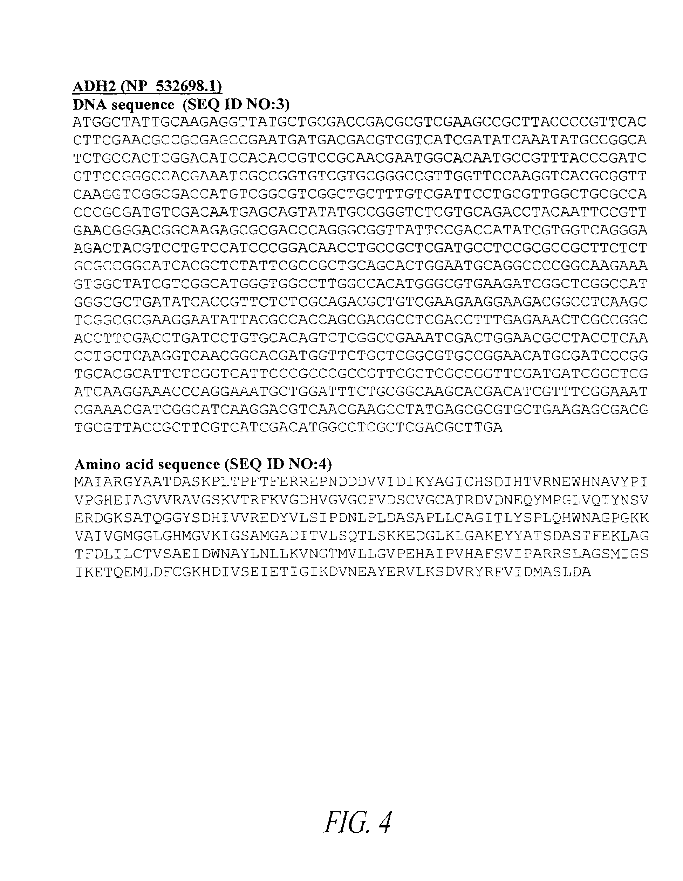

Wild type *E. coli* cannot use alginate polymer or degraded alginate as its sole carbon source *(see* Figure 4). *Vibrio splendidus,* however, is known to be able to metabolize alginate to support growth. To generate recombinant *E. coli* that use degraded alginate as its sole carbon source, a *Vibrio splendidus* fosmid library was constructed and cloned into *E. coli.* (*see, e.g.,* related U.S. Application No. 12/245,537, which is incorporated by reference in its entirety).

To prepare the *Vibrio splendidus* fosmid library, genomic DNA was isolated from *Vibrio Splendidus* BO1 (gift from Dr. Martin Polz, MIT) using the DNeasy Blood and Tissue Kit (Qiagen, Valencia, CA). A fosmid library was then constructed using Copy Control Fosmid Library Production Kit (Epicentre, Madison, WI). This library consisted of random genomic fragments of approximately 40kb inserted into the vector pCCIFOS (Epicentre, Madison, WI).

The fosmid library was packaged into phage, and *E. coli* DH10B cells harboring a pDONR221 plasmid (Invitrogen, Carlsbad, CA) carrying certain *Vibrio splendidus* genes (V12B01_02425 to V12B01_02480; encoding a type II secretion apparatus) were transfected with the phage library. This secretome region encodes a type II secretion apparatus derived from *Vibrio splendidus,* which was cloned into a pDONR221 plasmid and introduced into *E. coli* strain DH10B.

Transformants were selected for chloramphenicol resistance and then screened for their ability to grow on degraded alginate. The resultant transformants were screened for growth on degraded alginate media. Degraded alginate media was prepared by incubating 2% Alginate (Sigma-Aldrich, St. Louis, MO) 10 mM Na-Phosphate buffer, 50 mM KCl, 400 mM NaCl with alginate lyase from *Flavobacterium sp.* (Sigma-Aldrich, St. Louis, MO) at room temperature for at least one week. This degraded alginate was diluted to a concentration of 0.8% to make growth media that had a final concentration of 1X M9 salts, 2 mM MgSO4, 100 $\mu$M CaCl2, 0.007% Leucine, 0.01% casamino acids, 1.5% NaCl (this includes all sources of sodium: M9, diluted alginate and added NaCl).

One fosmid-containing *E. coli* clone was isolated that grew well on this media. The fosmid DNA from this clone was isolated and prepared using FosmidMAX DNA Purification Kit (Epicentre, Madison, WI). This isolated fosmid was transferred back into DH10B cells, and these cells were tested for the ability to grown on alginate.

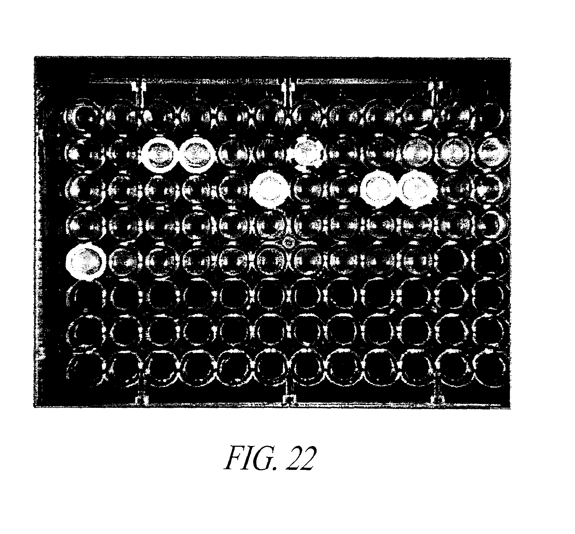

The results are illustrated in Figure 22, which shows that certain fosmid-containing *E. coli* clones are capable of growing on alginate as a sole source of carbon. *Agrobacterium tumefaciens* provides a positive control (*see* hatched circles). As a negative control, *E. coli* DH10B cells are not capable of growing on alginate (*see* immediate left of positive control).

These results also demonstrate that the sequences contained within this *Vibrio splendidus* derived fosmid clone are sufficient to confer on *E. coli* the ability to grow on degraded alginate as a sole source of carbon. Accordingly, the type II secretion machinery sequences contained within the pDONR221 vector, which was harbored by the original DH10B cells, were not necessary for growth on degraded alginate.

The isolated fosmid sufficient to confer growth alginate as a sole source of carbon was sequenced by Elim Biopharmaceuticals (Hayward, CA). Sequencing showed that the vector contained a genomic DNA section that contained the full length genes V12B01_24189 to V12B01_24249. In this sequence, there is a large gene before V12B01_24189 that is truncated in the fosmid clone. The large gene V12B01_24184 is a putative protein with similarity to autotransporters and belongs to COG3210, which is a cluster of orthologous proteins that include large exoproteins involved in heme utilization or adhesion. In the fosmid clone, V12B01_24184 is N-terminally truncated such that the first 5893 bp are missing from the predicted open reading frame (which is predicted to contain 22889 bp in total).

EXAMPLE 4

PRODUCTION OF ETHANOL FROM ALGINATE

The ability of recombinant *E. coli* to produce ethanol by growing on alginate on a source of carbon was tested. To generate recombinant *E. coli,* DNA sequences encoding pyruvate decarboxylase (*pdc*), and two alcohol dehydrogenase (*adhA* and *adhB*) *of Zymomonas mobilis* were amplified by polymerase chain reaction (PCR). For an exemplary *pdc* sequence from Z. *mobilis, see* U.S. Patent No. 7,189,545, which is hereby incorporated by reference for its information on these sequences. For exemplary *adhA* and *adhB* sequences from Z. *mobilis, see* Keshav et al., J Bacteriol. 172: 2491-2497, 1990, which is hereby incorporated by reference for its information on these sequences.

These amplified fragments were gel purified and spliced together by another round of PCR. The final amplified DNA fragment was digested with BamHI and Xbal ligated into cloning vector pBBRIMCS-2 pre-digested with the same restriction enzymes. The resulting plasmid is referred to as pBBRPdc-AdhA/B.

*E. coli* was transformed with either pBBRPdc-AdhA/B or pBBRPdc-AdhA/B + 1.5 Fos (fosmid clone containing genomic region between V12B01_24189 and V12B01_24249; these sequences confer on *E. coli* the ability to use alginate as a sole source of carbon, *see* Example 3), grown in m9 media containing alginate, and tested for the production of ethanol. The results are shown in Figure 23, which demonstrates that the strain harboring pBBRPdc-AdhA/B + 1.5 FOS showed significantly higher ethanol production when growing on alginate. These results indicate that the pBBRPdc-AdhA/B + 1.5 FOS was able to utilize alginate as a source of carbon in the production of ethanol.

**Claims**

1. A microbial system with enhanced efficiency for converting 4-deoxy-L-erythro-5-hexoseulose uronate (DEHU) into 2-keto-3-deoxy-D-gluconate (KDG),
wherein the microbial system comprises a recombinant microorganism, wherein the the recombinant microorganism comprises an isolated polynucleotide operably linked to an expression control region, wherein the polynucleotide encodes an ADH polypeptide having a hydrogenase activity, wherein the hydrogenase activity is DEHU hydrogenase activity.

2. The microbial system according to claim 1, wherein the encoded ADH polypeptide has an amino-acid sequence that shares at least 80 % sequence identity with the sequence set forth in any one of SEQ ID NOs:2, 4, 6, 8, 10, 12, 14, 16, 18, 20, 22, 24, 26, 28, 30, 32, 34, 36, 38, or 78.

3. The microbial system according to claim 1, wherein the encoded ADH polypeptide has an amino-acid sequence that shares at least 80 % sequence identity with the sequence set forth in SEQ ID NO:78

4. The microbial system according to any of claims 1-3 wherein the encoded ADH polypeptide comprises at least one of a nicotinamide adenine dinucleotide (NAD+), NADH, nicotinamide adenine dinucleotide phosphate (NADP+), or NADPH binding motif selected from the group consisting of Y-X-G-G-X-Y (SEQ ID NO:67), Y-X-X-G-G-X-Y (SEQ ID NO:68), Y-X-X-X-G-G-X-Y (SEQ ID NO:69), Y-X-G-X-X-Y (SEQ ID NO:70), Y-X-X-G-G-X-X-Y (SEQ ID NO:71), Y-X-X-X-G-X-X-Y (SEQ ID NO:72), Y-X-G-X-Y (SEQ ID NO:73), Y-X-X-G-X-Y (SEQ ID NO:74), Y-X-X-X-G-X-Y (SEQ ID NO:75), and Y-X-X-X-X-G-X-Y (SEQ ID NO:76); wherein Y is independently selected from alanine, glycine, and serine, wherein G is glycine, and wherein X is independently selected from a genetically encoded amino acid.

5. The microbial system according to any of claims 1-4, wherein the microorganism is *E. coli.*

6. The microbial system according to any of claims 1-4, wherein the microorganism is yeast.

7. The microbial system according to any of claims 1-4 wherein the microorganism is selected from *Acetobacter aceti, Achromobacter, Acidiphilium, Acinetobacter, Actinomadura, Actinoplanes, Aeropyrum pernix, Agrobacterium, Alcaligenes, Ananas comosus (M), Arthrobacter, Aspargillus niger, Aspargillus oryze, Aspergillus melleus, Aspergillus pulverulentus, Aspergillus saitoi, Aspergillus sojea, Aspergillus usamii, Bacillus alcalophilus, Bacillus amyloliquefaciens, Bacillus brevis, Bacillus circulans, Bacillus clausii, Bacillus lentus, Bacillus licheniformis, Bacillus macerans, Bacillus stearothermophilus, Bacillus subtilis, Bifidobacterium, Brevibacillus brevis, Burkholderia cepacia, Candida cylindracea, Candida rugosa, Carica papaya (L), Cellulosimicrobium, Cephalosporium, Chaetomium erraticum, Chaetomium gracile, Clostridium, Clostridium butyricum, Clostridium acetobutylicum, Clostridium thermocellum, Corynebacterium (glutamicum), Corynebacterium efficiens, Escherichia coli, Enterococcus, Erwina chrysanthemi, Gliconobacter, Gluconacetobacter, Haloarcula, Humicola insolens, Humicola nsolens, Kitasatospora setae, Klebsiella, Klebsiella oxytoca, Kluyveromyces, Kluyveromyces fragilis, Kluyveromyces lactis, Kocuria, Lactlactis, Lactobacillus, Lactobacillus fermentum, Lactobacillus sake, Lactococcus, Lactococcus lactis, Leuconostoc, Methylocystis, Methanolobus siciliae, Methanogenium organophilum, Methanobacterium bryantii, Microbacterium imperiale, Micrococcus lysodeikticus, Microlunatus, Mucor javanicus, Mycobacterium, Myrothecium, Nitrobacter, Nitrosomonas, Nocardia, Papaya carica, Pediococcus, Pediococcus halophilus, Penicillium, Penicillium camemberti, Penicillium citrinum, Penicillium emersonii, Penicillium roqueforti, Penicillum lilactinum, Penicillum multicolor, Paracoccus pantotrophus, Propionibacterium, Pseudomonas, Pseudomonas fluorescens, Pseudomonas denitrificans, Pyrococcus, Pyrococcus furiosus, Pyrococcus horikoshii, Rhizobium, Rhizomucor miehei, Rhizomucor pusillus Lindt, Rhizopus, Rhizopus delemar, Rhizopus japonicus, Rhizopus niveus, Rhizopus oryzae, Rhizopus oligosporus, Rhodococcus, Saccharomyces cerevisiae, Sclerotina libertina, Sphingobacterium multivorum, Sphingobium, Sphingomonas, Streptococcus, Streptococcus thermophilus Y-1, Streptomyces, Streptomyces griseus, Streptomyces lividans, Streptomyces murinus, Streptomyces rubiginosus, Streptomyces violaceoruber, Streptoverticillium mobaraense, Tetragenococcus, Thermus, Thiosphaera pantotropha, Trametes, Trichoderma, Trichoderma longibrachiatum, Trichoderma reesei, Trichoderma viride, Trichosporon penicillatum, Vibrio alginolyticus, Xanthomonas, yeast, Zygosaccharomyces rouxii, Zymomonas, and Zymomonus mobilis Rhizomucor pusillus Lindt, Rhizopus, Rhizopus delemar, Rhizopus japonicus, Rhizopus niveus, Rhizopus oryzae, Rhizopus oligosporus, Rhodococcus, Saccharomyces cerevisiae, Sclerotina libertina, Sphingobacterium multivorum, Sphingobium, Sphingomonas, Streptococcus, Streptococcus thermophilus Y-1, Streptomyces, Streptomyces griseus, Streptomyces lividans, Streptomyces murinus, Streptomyces rubiginosus, Streptomyces violaceoruber, Streptoverticillium mobaraense, Tetragenococcus, Thermus, Thiosphaera pantotropha, Trametes, Trichoderma, Trichoderma longibrachiatum, Tri-*

*choderma reesei, Trichoderma viride, Trichosporon; penicillatum, Vibrio alginolyticus, Xanthomonas, yeast, Zygosaccharomyces rouxii, Zymomonas,* and *Zymomonus mobilis.*

**8.** A use of a microbial system comprising a polypeptide having 4-deoxy-L-erythro-5-hexoseulose uronate (DEHU) hydrogenase activity for converting aquatic or marine sphere biomass to a commodity chemical.

**9.** The use according to claim 6, wherein the microbial system is as defined in any of claims 1-7.

**10.** The use according to claim 8 or 9, wherein the commodity chemical is a biofuel.

**11.** The use according to any of claims 8-10 wherein the commodity chemical is selected from methane, methanol, ethane, ethene, ethanol, n-propane, 1-propene, 1-propanol, propanal, acetone, propionate, n-butane, 1-butene, 1-butanol, butanal, butanoate, isobutanal, isobutanol, 2-methylbutanal, 2-methylbutanol, 3-methylbutanal, 3-methylbutanol, 2-butene, 2-butanol, 2-butanone, 2,3-butanediol, 3-hydroxy-2-butanone, 2,3-butanedione, ethylbenzene, ethenylbenzene, 2-phenylethanol, phenylacetaldehyde, 1-phenylbutane, 4-phenyl-l-butene, 4-phenyl-2-butene, 1-phenyl-2-butene, 1-phenyl-2-butanol, 4-phenyl-2-butanol, 1-phenyl-2-butanone, 4-phenyl-2-butanone, 1-phenyl-2,3-butandiol, 1-phenyl-3-hydroxy-2-butanone, 4-phenyl-3-hydroxy-2-butanone, 1-phenyl-2,3-butanedione, n-pentane, ethylphenol, ethenylphenol, 2-(4-hydroxyphenyl) ethanol, 4-hydroxyphenylacetaldehyde, 1-(4-hydroxyphenyl) butane, 4-(4-hydroxyphenyl)-1-butene, 4-(4-hydroxyphenyl)-2-butene, 1-(4-hydroxyphenyl)-1-butene, 1-(4-hydroxyphenyl)-2-butanol, 4-(4-hydroxyphenyl)-2-butanol, 1-(4-hydroxyphenyl)-2-butanone, 4-(4-hydroxyphenyl)-2-butanone, 1-(4-hydroxyphenyl)-2,3-butandiol, 1-(4-hydroxyphenyl)-3-hydroxy-2-butanone, 4-(4-hydroxyphenyl)-3-hydroxy-2-butanone, 1-(4-hydroxyphenyl)-2,3-butanonedione, indolylethane, indolylethene, 2-(indole-3-)ethanol, n-pentane,l-pentene, 1-pentanol, pentanal, pentanoate, 2-pentene, 2-pentanol, 3-pentanol, 2-pentanone, 3-pentanone, 4-methylpentanal, 4-methylpentanol, 2,3-pentanediol, 2-hydroxy-3-pentanone, 3-hydroxy-2-pentanone, 2,3-pentanedione, 2-methylpentane, 4-methyl-l-pentene, 4-methyl-2-pentene, 4-methyl-3-pentene, 4-methyl-2-pentanol, 2-methyl-3-pentanol, 4-methyl-2-pentanone, 2-methyl-3-pentanone, 4-methyl-2,3-pentanediol, 4-methyl-2-hydroxy-3-pentanone, 4-methyl-3-hydroxy-2-pentanone, 4-methyl-2,3-pentanedione, 1-phenylpentane, 1-phenyl-l-pentene, 1-phenyl-2-pentene, 1-phenyl-3-pentene, 1-phenyl-2-pentanol, 1-phenyl-3-pentanol, 1-phenyl-2-pentanone, 1-phenyl-3-pentanone, 1-phenyl-2,3-pentanediol, 1-phenyl-2-hydroxy-3-pentanone, 1-phenyl-3-hydroxy-2-pentanone, 1-phenyl-2,3-pentanedione, 4-methyl-1-phenylpentane, 4-methyl-1-phenyl-1-pentene, 4-methyl-l-phenyl-2-pentene, 4-methyl-1-phenyl-3-pentene, 4-methyl-1-phenyl-3-pentanol, 4-methyl-1-phenyl-2-pentanol, 4-methyl-1-phenyl-3-pentanone, 4-methyl-1-phenyl-2-pentanone, 4-methyl-1-phenyl-2,3-pentanediol, 4-methyl-1-phenyl-2,3-pentanedione , 4-methyl-1-phenyl-3-hydroxy-2-pentanone, 4-methyl-1-phenyl-2-hydroxy-3-pentanone, 1-(4-hydroxyphenyl) pentane, 1-(4-hydroxyphenyl)-1-pentene, 1-(4-hydroxyphenyl)-2-pentene, 1-(4-hydroxyphenyl)-3-pentene, 1-(4-hydroxyphenyl)-2-pentanol, 1-(4-hydroxyphenyl)-3-pentanol, 1-(4-hydroxyphenyl)-2-pentanone, 1-(4-hydroxyphenyl)-3-pentanone, 1-(4-hydroxyphenyl)-2,3-pentanediol, 1-(4-hydroxyphenyl)-2-hydroxy-3-pentanone, 1-(4-hydroxyphenyl)-3-hydroxy-2-pentanone, 1-(4-hydroxyphenyl)-2,3-pentanedione, 4-methyl-1-(4-hydroxyphenyl) pentane, 4-methyl-1-(4-hydroxyphenyl)-2-pentene, 4-methyl-1-(4-hydroxyphenyl)-3-pentene, 4-methyl-1-(4-hydroxyphenyl)-1-pentene, 4-methyl-1-(4-hydroxyphenyl)-3-pentanol, 4-methyl-1-(4-hydroxyphenyl)-2-pentanol, 4-methyl-1-(4-hydroxyphenyl)-3-pentanone, 4-methyl-1-(4-hydroxyphenyl)-2-pentanone, 4-methyl-1-(4-hydroxyphenyl)-2,3-pentanediol, 4-methyl-1-(4-hydroxyphenyl)-2,3-pentanedione , 4-methyl-1-(4-hydroxyphenyl)-3-hydroxy-2-pentanone, 4-methyl-1-(4-hydroxyphenyl)-2-hydroxy-3-pentanone, 1-indole-3-pentane, 1-(indole-3)-1-pentene, 1-(indole-3)-2-pentene, 1-(indole-3)-3-pentene, 1-(indole-3)-2-pentanol, 1-(indole-3)-3-pentanol, 1-(indole-3)-2-pentanone, 1-(indole-3)-3-pentanone, 1-(indole-3)-2,3-pentanediol, 1-(indole-3)-2-hydroxy-3-pentanone, 1-(indole-3)-3-hydroxy-2-pentanone, 1-(indole-3)-2,3-pentanedione, 4-methyl-1-(indole-3-)pentane, 4-methyl-1-(indole-3)-2-pentene, 4-methyl-1-(indole-3)-3-pentene, 4-methyl-1-(indole-3)-1-pentene, 4-methyl-2-(indole-3)-3-pentanol, 4-methyl-1-(indole-3)-2-pentanol, 4-methyl-1-(indole-3)-3-pentanone, 4-methyl-1-(indole-3)-2-pentanone, 4-methyl-1-(indole-3)-2,3-pentanediol, 4-methyl-1-(indole-3)-2,3-pentanedione, 4-methyl-1-(indole-3)-3-hydroxy-2-pentanone, 4-methyl-1-(indole-3)-2-hydroxy-3-pentanone, n-hexane, 1-hexene, 1-hexanol, hexanal, hexanoate, 2-hexene, 3-hexene, 2-hexanol, 3-hexanol, 2-hexanone, 3-hexanone, 2,3-hexanediol, 2,3-hexanedione, 3,4-hexanediol, 3,4-hexanedione, 2-hydroxy-3-hexanone, 3-hydroxy-2-hexanone, 3-hydroxy-4-hexanone, 4-hydroxy-3-hexanone, 2-methylhexane, 3-methylhexane, 2-methyl-2-hexene, 2-methyl-3-hexene, 5-methyl-l-hexene, 5-methyl-2-hexene, 4-methyl-l-hexene, 4-methyl-2-hexene, 3-methyl-3-hexene, 3-methyl-2-hexene, 3-methyl-l-hexene, 2-methyl-3-hexanol, 5-methyl-2-hexanol, 5-methyl-3-hexanol, 2-methyl-3-hexanone, 5-methyl-2-hexanone, 5-methyl-3-hexanone, 2-methyl-3,4-hexanediol, 2-methyl-3,4-hexanedione , 5-methyl-2,3-hexanediol, 5-methyl-2,3-hexanedione, 4-methyl-2,3-hexanediol, 4-methyl-2,3-hexanedione, 2-methyl-3-hydroxy-4-hexanone, 2-methyl-4-hydroxy-3-hexanone, 5-methyl-2-hydroxy-3-hexanone, 5-methyl-3-hydroxy-2-hexanone, 4-methyl-2-hydroxy-3-hexanone, 4-methyl-3-hydroxy-2-hexanone, 2,5-dimethylhexane, 2,5-dimethyl-2-hexene, 2,5-dimethyl-3-hexene, 2,5-dimethyl-3-hexanol, 2,5-dimethyl-3-hexanone, 2,5-dimethyl-3,4-hexanediol, 2,5-dimethyl-3,4-hexanedione, 2,5-dimethyl-3-hydroxy-4-hexanone, 5-methyl-l-phenylhexane, 4-methyl-1-phenylhexane, 5-methyl-1-phenyl-l-hexene, 5-methyl-1-phenyl-2-hexene, 5-methyl-1-phenyl-3-hexene, 4-methyl-1-phenyl-1-hexene, 4-methyl-1-phenyl-2-hexene, 4-methyl-1-phenyl-3-hexene, 5-methyl-1-phenyl-2-hexanol, 5-methyl-1-phenyl-3-hexanol, 4-methyl-1-phenyl-2-hexanol, 4-methyl-1-phenyl-3-hexanol, 5-methyl-1-phenyl-2-hexanone, 5-methyl-1-phenyl-3-hexanone, 4-methyl-1-phenyl-2-hexanone, 4-methyl-1-phenyl-3-hexanone, 5-methyl-1-phenyl-2,3-hexanediol, 4-methyl-1-phenyl-2,3-hexanediol, 5-methyl-1-phenyl-3-hydroxy-2-hexanone, 5-methyl-1-phenyl-2-hydroxy-3-hexanone, 4-methyl-1-phenyl-3-hydroxy-2-hexanone, 4-methyl-1-phenyl-2-hydroxy-3-hexanone, 5-methyl-1-phenyl-2,3-hexanedione, 4-methyl-1-phenyl-2,3-hexanedione, 4-methyl-1-(4-hydroxyphenyl)hexane, 5-methyl-1-(4-hydroxyphenyl)-1-hexene, 5-methyl-1-(4-hydroxyphenyl)-2-hexene, 5-methyl-1-(4-hydroxyphenyl)-3-hexene, 4-methyl-1-(4-hydroxyphenyl)-1-hexene, 4-methyl-1-(4-hydroxyphenyl)-2-hexene, 4-methyl-1-(4-hydroxyphenyl)-3-hexene, 5-methyl-1-(4-hydroxyphenyl)-2-hexanol, 5-methyl-1-(4-hydroxyphenyl)-3-hexanol, 4-methyl-1-(4-hydroxyphenyl)-2-hexanol, 4-methyl-1-(4-hydroxyphenyl)-3-hexanol, 5-methyl-1-(4-hydroxyphenyl)-2-hexanone, 5-methyl-1-(4-hydroxyphenyl)-3-hexanone, 4-methyl-1-(4-hydroxyphenyl)-2-hexanone, 4-methyl-1-(4-hydroxyphenyl)-3-hexanone, 5-methyl-1-(4-hydroxyphenyl)-2,3-hexanediol, 4-methyl-1-(4-hydroxyphenyl)-2,3-hexanediol, 5-methyl-1-(4-hydroxyphenyl)-3-hydroxy-2-hexanone, 5-methyl-1-(4-hydroxyphenyl)-2-hydroxy-3-hexanone, 4-methyl-1-(4-hydroxyphenyl)-3-hydroxy-2-hexanone, 4-methyl-1-(4-hydroxyphenyl)-2-hydroxy-3-hexanone, 5-methyl-1-(4-hydroxyphenyl)-2,3-hexanedione, 4-methyl-1-(4-hydroxyphenyl)-2,3-hexanedione, 4-methyl-1-(indole-3-)hexane, 5-methyl-1-(indole-3)-1-hexene, 5-methyl-1-(indole-3)-2-hexene, 5-methyl-1-(indole-3)-3-hexene, 4-methyl-1-(indole-3)-1-hexene, 4-methyl-1-(indole-3)-2-hexene, 4-methyl-1-(indole-3)-3-hexene, 5-methyl-1-(indole-3)-2-hexanol, 5-methyl-1-(indole-3)-3-hexanol, 4-methyl-1-(indole-3)-2-hexanol, 4-methyl-1-(indole-3)-3-hexanol, 5-methyl-1-(indole-3)-2-hexanone, 5-methyl-1-(indole-3)-3-hexanone, 4-methyl-1-(indole-3)-2-hexanone, 4-methyl-1-(indole-3)-3-hexanone, 5-methyl-1-(indole-3)-2,3-hexanediol, 4-methyl-1-(indole-3)-2,3-hexanediol, 5-methyl-1-(indole-3)-3-hydroxy-2-hexanone, 5-methyl-1-(indole-3)-2-hydroxy-3-hexanone, 4-methyl-1-(indole-3)-3-hydroxy-2-hexanone, 4-methyl-1-(indole-3)-2-hydroxy-3-hexanone, 5-methyl-1-(indole-3)-2,3-hexanedione, 4-methyl-1-(indole-3)-2,3-hexanedione, n-heptane, 1-heptene, 1-heptanol, heptanal, heptanoate, 2-heptene, 3-heptene, 2-heptanol, 3-heptanol, 4-heptanol, 2-heptanone, 3-heptanone, 4-heptanone, 2,3-heptanediol, 2,3-heptanedione, 3,4-heptanediol, 3,4-heptanedione, 2-hydroxy-3-heptanone, 3-hydroxy-2-heptanone, 3-hydroxy-4-heptanone, 4-hydroxy-3-heptanone, 2-methylheptane, 3-methylheptane, 6-methyl-2-heptene, 6-methyl-3-heptene, 2-methyl-3-heptene, 2-methyl-2-heptene, 5-methyl-2-heptene, 5-methyl-3-heptene, 3-methyl-3-heptene, 2-methyl-3-heptanol, 2-methyl-4-heptanol, 6-methyl-3-heptanol, 5-methyl-3-heptanol, 3-methyl-4-heptanol, 2-methyl-3-heptanone, 2-methyl-4-heptanone, 6-methyl-3-heptanone, 5-methyl-3-heptanone, 3-methyl-4-heptanone, 2-methyl-3,4-heptanediol, 2-methyl-3,4-heptanedione, 6-methyl-3,4-heptanediol, 6-methyl-3,4-heptanedione, 5-methyl-3,4-heptanediol, 5-methyl-3,4-heptanedione, 2-methyl-3-hydroxy-4-heptanone, 2-methyl-4-hydroxy-3-heptanone, 6-methyl-3-hydroxy-4-heptanone, 6-methyl-4-hydroxy-3-heptanone, 5-methyl-3-hydroxy-4-heptanone, 5-methyl-4-hydroxy-3-heptanone, 2,6-dimethylheptane, 2,5-dimethylheptane, 2,6-dimethyl-2-heptene, 2,6-dimethyl-3-heptene, 2,5-dimethyl-2-heptene, 2,5-dimethyl-3-heptene, 3,6-dimethyl-3-heptene, 2,6-dimethyl-3-heptanol, 2,6-dimethyl-4-heptanol, 2,5-dimethyl-3-heptanol, 2,5-dimethyl-4-heptanol, 2,6-dimethyl-3,4-heptanediol, 2,6-dimethyl-3,4-heptanedione, 2,5-dimethyl-3,4-heptanediol, 2,5-dimethyl-3,4-heptanedione, 2,6-dimethyl-3-hydroxy-4-heptanone, 2,6-dimethyl-4-hydroxy-3-heptanone, 2,5-dimethyl-3-hydroxy-4-heptanone, 2,5-dimethyl-4-hydroxy-3-heptanone, n-octane, 1-octene, 2-octene, 1-octanol, octanal, octanoate, 3-octene, 4-octene, 4-octanol, 4-octanone, 4,5-octanediol, 4,5-octanedione, 4-hydroxy-5-octanone, 2-methyloctane, 2-methyl-3-octene, 2-methyl-4-octene, 7-methyl-3-octene, 3-methyl-3-octene, 3-methyl-4-octene, 6-methyl-3-octene, 2-methyl-4-octanol, 7-methyl-4-octanol, 3-methyl-4-octanol, 6-methyl-4-octanol, 2-methyl-4-octanone, 7-methyl-4-octanone, 3-methyl-4-octanone, 6-methyl-4-octanone, 2-methyl-4,5-octanediol, 2-methyl-4,5-octanedione, 3-methyl-4,5-octanediol, 3-methyl-4,5-octanedione, 2-methyl-4-hydroxy-5-octanone, 2-methyl-5-hydroxy-4-octanone, 3-methyl-4-hydroxy-5-octanone, 3-methyl-5-hydroxy-4-octanone, 2,7-dimethyloctane, 2,7-dimethyl-3-octene, 2,7-dimethyl-4-octene, 2,7-dimethyl-4-octanol, 2,7-dimethyl-4-octanone, 2,7-dimethyl-4,5-octanediol, 2,7-dimethyl-4,5-octanedione, 2,7-dimethyl-4-hydroxy-5-octanone, 2,6-dimethyloctane, 2,6-dimethyl-3-octene, 2,6-dimethyl-4-octene, 3,7-dimethyl-3-octene, 2,6-dimethyl-4-octanol, 3,7-dimethyl-4-octanol, 2,6-dimethyl-4-octanone, 3,7-dimethyl-4-octanone, 2,6-dimethyl-4,5-octanediol, 2,6-dimethyl-4,5-octanedione, 2,6-dimethyl-4-hydroxy-5-octanone, 2,6-dimethyl-5-hydroxy-4-octanone, 3,6-dimethyloctane, 3,6-dimethyl-3-octene, 3,6-dimethyl-4-octene, 3,6-dimethyl-4-octanol, 3,6-dimethyl-4-octanone, 3,6-dimethyl-4,5-octanediol, 3,6-dimethyl-4,5-octanedione, 3,6-dimethyl-4-hydroxy-5-octanone, n-nonane, 1-nonene, 1-nonanol, nonanal, nonanoate, 2-methylnonane, 2-methyl-4-nonene, 2-methyl-5-nonene, 8-methyl-4-nonene, 2-methyl-5-nonanol, 8-methyl-4-nonanol, 2-methyl-5-nonanone, 8-methyl-4-nonanone, 8-methyl-4,5-nonanediol, 8-methyl-4,5-nonanedione, 8-methyl-4-hydroxy-5-nonanone, 8-methyl-5-hydroxy-4-nonanone, 2,8-dimethylnonane, 2,8-dimethyl-3-nonene, 2,8-dimethyl-4-nonene, 2,8-dimethyl-5-nonene, 2,8-dimethyl-4-nonanol, 2,8-dimethyl-5-nonanol, 2,8-dimethyl-4-nonanone, 2,8-dimethyl-5-nonanone, 2,8-dimethyl-4,5-nonanediol, 2,8-dimethyl-4,5-nonanedione, 2,8-dimethyl-4-hydroxy-5-nonanone, 2,8-dimethyl-5-hydroxy-4-nonanone, 2,7-dimethylnonane, 3,8-dimethyl-3-nonene, 3,8-dimethyl-4-nonene, 3,8-dimethyl-5-nonene, 3,8-dimethyl-4-nonanol, 3,8-dimethyl-5-nonanol, 3,8-dimethyl-4-nonanone, 3,8-dimethyl-5-nonanone, 3,8-dimethyl-4,5-nonanediol, 3,8-dimethyl-4,5-nonanedione, 3,8-dimethyl-4-hydroxy-5-nonanone, 3,8-dimethyl-5-hydroxy-4-nonanone, n-decane, 1-decene, 1-decanol, decanoate, 2,9-dimethyldecane, 2,9-dimethyl-3-decene, 2,9-dimethyl-4-decene, 2,9-dimethyl-5-decanol, 2,9-dimethyl-5-decanone, 2,9-dimethyl-5,6-decanediol, 2,9-dimethyl-6-hydroxy-5-decanone, 2,9-dimethyl-5,6-decanedionen-undecane, 1-undecene, 1-undecanol, undecanal, undecanoate, n-dodecane, 1-dodecene, 1-dodecanol, dodecanal, dodecanoate, n-dodecane, 1-decadecene, 1-dodecanol, dodecanal, dodecanoate, n-tridecane, 1-tridecene, 1-tridecanol, tridecanal, tridecanoate, n-tetradecane, 1-tetradecene, 1-tetradecanol, tetradecanal, tetradecanoate, n-pentadecane, 1-pentadecene, 1-pentadecanol, pentadecanal, pentadecanoate, n-hexadecane, 1-hexadecene, 1-hexadecanol, hexadecanal, hexadecanoate, n-heptadecane, 1-heptadecene, 1-heptadecanol, heptadecanal, heptadecanoate, n-octadecane, 1-octadecene, 1-octadecanol, octadecanal, octadecanoate, n-nonadecane, 1-nonadecene, 1-nonadecanol, nonadecanal, nonadecanoate, eicosane, 1-eicosene, 1-eicosanol, eicosanal, eicosanoate, 3-hydroxy propanal, 1, 3-propanediol, 4-hydroxybutanal, 1,4-butanediol, 3-hydroxy-2-butanone, 2, 3-butandiol, 1,5-pentane diol, homocitrate, homoisocitorate, b-hydroxy adipate, glutarate, glutarsemialdehyde, glutaraldehyde, 2-hydroxy-1-cyclopentanone, 1,2-cyclopentanediol, cyclopentanone, cyclopentanol, (S)-2-acetolactate, (R)-2,3-Dihydroxy-isovalerate, 2-oxoisovalerate, isobutyryl-CoA, isobutyrate, isobutyraldehyde, 5-amino pentaldehyde, 1,10-diaminodecane, 1,10-diamino-5-decene, 1,10-diamino-5-hydroxydecane, 1,10-diamino-5-decanone, 1,10-diamino-5,6-decanediol, 1,10-diamino-6-hydroxy-5-decanone, phenylacetoaldehyde, 1,4-diphenylbutane, 1,4-diphenyl-1-butene, 1,4-diphenyl-2-butene, 1,4-diphenyl-2-butanol, 1,4-diphenyl-2-butanone, 1,4-diphenyl-2,3-butanediol, 1,4-diphenyl-3-hydroxy-2-butanone, 1-(4-hydeoxyphenyl)-4-phenylbutane, 1-(4-hydeoxyphenyl)-4-phenyl-1-butene, 1-(4-hydeoxyphenyl)-4-phenyl-2-butene, 1-(4-hydeoxyphenyl)-4-phenyl-2-butanol, 1-(4-hydeoxyphenyl)-4-phenyl-2-butanone, 1-(4-hydeoxyphenyl)-4-phenyl-2,3-butanediol, 1-(4-hydeoxyphenyl)-4-phenyl-3-hydroxy-2-butanone, 1-(indole-3)-4-phenylbutane, 1-(indole-3)-4-phenyl-1-butene, 1-(indole-3)-4-phenyl-2-butene, 1-(indole-3)-4-phenyl-2-butanol, 1-(indole-3)-4-phenyl-2-butanone, 1-(indole-3)-4-phenyl-2,3-butanediol, 1-(indole-3)-4-phenyl-3-hydroxy-2-butanone, 4-hydroxyphenylacetoaldehyde, 1,4-di(4-hydroxyphenyl)butane, 1,4-di(4-hydroxyphenyl)-l-butene, 1,4-di(4-hydroxyphenyl)-2-butene, 1,4-di(4-hydroxyphenyl)-2-butanol, 1,4-di(4-hydroxyphenyl)-2-butanone, 1,4-di(4-hydroxyphenyl)-2,3-butanediol, 1,4-di(4-hydroxyphenyl)-3-hydroxy-2-butanone, 1-(4-hydroxyphenyl)-4-(indole-3-)butane, 1-(4-hydroxyphenyl)-4-(indole-3)-1-butene, 1-di(4-hydroxyphenyl)-4-(indole-3)-2-butene, 1-(4-hydroxyphenyl)-4-(indole-3)-2-butanol, 1-(4-hydroxyphenyl)-4-(indole-3)-2-butanone, 1-(4-hydroxyphenyl)-4-(indole-3)-2,3-butanediol, 1-(4-hydroxyphenyl-4-(indole-3)-3-hydroxy-2-butanone, indole-3-acetoaldehyde, 1,4-di(indole-3-)butane, 1,4-di(indole-3)-1-butene, 1,4-di(indole-3)-2-butene, 1,4-di(indole-3)-2-butanol, 1,4-di(indole-3)-2-butanone, 1,4-di(indole-3)-2,3-butanediol, 1,4-di(indole-3)-3-hydroxy-2-butanone, succinate semialdehyde, hexane-1,8-dicarboxylic acid, 3-hexene-1,8-dicarboxylic acid, 3-hydroxy-hexane-1,8-dicarboxylic acid, 3-hexanone-1,8-dicarboxylic acid, 3,4-hexanediol-1,8-dicarboxylic acid, and 4-hydroxy-3-hexanone-1,8-dicarboxylic acid.

12. The use according to any of claims 8-11 wherein the commodity chemical is 1-butanol, 2-butanol or isobutanol.

13. The use according to any of claims 8-11 wherein the commodity chemical is ethanol.

14. The use according to any of claims 8-13, wherein the aquatic or marine sphere biomass is selected from kelp, giant kelp, sargasso, seaweed, algae, marine microflora, microalgae, and sea grass.

NADPH consumption by ADH enzymes using DEHU as a substrate

Time (min)

◆ ADH1
■ ADH2
▲ ADH3
✕ ADH4
✳ ADH5
● ADH6
+ ADH7
− ADH8
− ADH9
◇ ADH10

*FIG. 1*

EP 2 463 374 A2

NADPH consumption by ADH enzymes using D-mannuronate as a substrate

FIG. 2

EP 2 463 374 A2

**ADH1 (NP 532245.1)**
**DNA sequence (SEQ ID NO:1)**
ATGTTCACAACGTCCGCCTATGCCTGCGATGACGGCTCTTCGCCGATGAAGCTCGCGAC
CATCAGGCGCCGCGATCCCGGTCCGCGCGATGTCGAAATCGAGATAGAATTCTGTGGCG
TCTGCCACTCGGACATCCATACGGCCCGCAGCGAATGGCCGGGCTCCCTCTACCCTTGC
GTCCCCGGCCACGAAATCGTCGGCCGTGTCGGTCGGGTGGGCGCGCAAGTCACCCGGTT
CAAGACGGGTGACCGCGTCGGTGTCGGCTGTATCGTCGATAGCTGCCGCGAATGCGCAA
GCTGCGCCGAAGGGCTGGAGCAATATTGCGAAAACGGCATGACCGGCACCTATAACTCC
CCTGACAAGGCGATGGGCGGCGGCGCGCATACGCTTGGCGGCTATTCCGCCCATGTGGT
GGTGGATGACCGCTATGTGCTCAATATTCCCGAAGGGCTCGATCCGGCGGCAGCAGCAC
CGCTACTCTGCGCTGGTATCACCACCTACTCGCCGCTGCGCCACTGGAATGCCGGCCCC
GGCAAACGCGTCGGCGTCGTCGGTCTGGGCGGCCTCGGCCATATGGCCGTCAAGCTCGC
CAATGCCATGGGTGCGACTGTCGTGATGATCACCACCTCGCCCGGCAAGGCGGAGGATG
CCAAAAAACTCGGCGCACACGAGGTGATCATCTCCCGCGATGCGGAGCAGATGAAGAAG
GCTACCTCGAGCCTCGATCTCATCATCGATGCTGTCGCCGCCGACCACGACATCGACGC
CTATCTGGCGCTGCTGAAACGCGATGGCGCGCTGGTGCAGGTGGGCGCGCCGGAAAAGC
CACTTTCGGTGATGGCCTTCAGCCTCATCCCCGGCCGCAAGACCTTTGCCGGCTCGATG
ATCGGCGGTATTCCCGAGACTCAGGAAATGCTGGATTTCTGCGCCGAAAAAGGCATCGC
CGGCGAAATCGAGATGATCGATATCGATCAGATCAATGACGCTTATGAACGCATGATAA
AAAGCGATGTGCGTTATCGTTTCGTCATTGATATGAAGAGCCTGCCGCGCCAGAAGGCC
GCCTGA

**Amino acid sequence (SEQ ID NO:2)**
MFTTSAYACDDGSSPMKLATIRRRDPGPRDVEIEIEFCGVCHSDIHTARSEWPGSLYPC
VPGHEIVGRVGRVGAQVTRFKTGDRVGVGCIVDSCRECASCAEGLEQYCENGMTGTYNS
PDKAMGGGAHTLGGYSAHVVVDDRYVLNIPEGLDPAAAAPLLCAGITTYSPLRHWNAGP
GKRVGVVGLGGLGHMAVKLANAMGATVVMITTSPGKAEDAKKLGAHEVIISRDAEQMKK
ATSSLDLIIDAVAADHDIDAYLALLKRDGALVQVGAPEKPLSVMAFSLIPGRKTFAGSM
IGGIPETQEMLDFCAEKGIAGEIEMIDIDQINDAYERMIKSDVRYRFVIDMKSLPRQKA
A

*FIG. 3*

<u>ADH2 (NP_532698.1)</u>
**DNA sequence (SEQ ID NO:3)**
ATGGCTATTGCAAGAGGTTATGCTGCGACCGACGCGTCGAAGCCGCTTACCCCGTTCAC
CTTCGAACGCCGCGAGCCGAATGATGACGACGTCGTCATCGATATCAAATATGCCGGCA
TCTGCCACTCGGACATCCACACCGTCCGCAACGAATGGCACAATGCCGTTTACCCGATC
GTTCCGGGCCACGAAATCGCCGGTGTCGTGCGGGCCGTTGGTTCCAAGGTCACGCGGTT
CAAGGTCGGCGACCATGTCGGCGTCGGCTGCTTTGTCGATTCCTGCGTTGGCTGCGCCA
CCCGCGATGTCGACAATGAGCAGTATATGCCGGGTCTCGTGCAGACCTACAATTCCGTT
GAACGGGACGGCAAGAGCGCGACCCAGGGCGGTTATTCCGACCATATCGTGGTCAGGGA
AGACTACGTCCTGTCCATCCCGGACAACCTGCCGCTCGATGCCTCCGCGCCGCTTCTCT
GCGCCGGCATCACGCTCTATTCGCCGCTGCAGCACTGGAATGCAGGCCCCGGCAAGAAA
GTGGCTATCGTCGGCATGGGTGGCCTTGGCCACATGGGCGTGAAGATCGGCTCGGCCAT
GGGCGCTGATATCACCGTTCTCTCGCAGACGCTGTCGAAGAAGGAAGACGGCCTCAAGC
TCGGCGCGAAGGAATATTACGCCACCAGCGACGCCTCGACCTTTGAGAAACTCGCCGGC
ACCTTCGACCTGATCCTGTGCACAGTCTCGGCCGAAATCGACTGGAACGCCTACCTCAA
CCTGCTCAAGGTCAACGGCACGATGGTTCTGCTCGGCGTGCCGGAACATGCGATCCCGG
TGCACGCATTCTCGGTCATTCCCGCCCGCCGTTCGCTCGCCGGTTCGATGATCGGCTCG
ATCAAGGAAACCCAGGAAATGCTGGATTTCTGCGGCAAGCACGACATCGTTTCGGAAAT
CGAAACGATCGGCATCAAGGACGTCAACGAAGCCTATGAGCGCGTGCTGAAGAGCGACG
TGCGTTACCGCTTCGTCATCGACATGGCCTCGCTCGACGCTTGA

**Amino acid sequence (SEQ ID NO:4)**
MAIARGYAATDASKPLTPFTFERREPNDDDVVIDIKYAGICHSDIHTVRNEWHNAVYPI
VPGHEIAGVVRAVGSKVTRFKVGDHVGVGCFVDSCVGCATRDVDNEQYMPGLVQTYNSV
ERDGKSATQGGYSDHIVVREDYVLSIPDNLPLDASAPLLCAGITLYSPLQHWNAGPGKK
VAIVGMGGLGHMGVKIGSAMGADITVLSQTLSKKEDGLKLGAKEYYATSDASTFEKLAG
TFDLILCTVSAEIDWNAYLNLLKVNGTMVLLGVPEHAIPVHAFSVIPARRSLAGSMIGS
IKETQEMLDFCGKHDIVSEIETIGIKDVNEAYERVLKSDVRYRFVIDMASLDA

*FIG. 4*

<u>ADH3 (NP_531326.1)</u>
**DNA sequence (SEQ ID NO:5)**
ATGACTAAAACAATGAAGGCGGCGGTTGTCCGCGCATTTGGAAAACCGCTGACCATCGA
GGAAGTGGCAATACCGGATCCCGGCCCCGGTGAAATTCTCATCAACTACAAGGCGACGG
GCGTTTGCCACACCGACCTGCACGCCGCAACGGGGGATTGGCCGGTCAAGCCCAACCCG
CCCTTCATTCCCGGACATGAAGGTGCAGGTTACGTCGCCAAGATCGGCGCTGGCGTCAC
CGGCATCAAGGAGGGCGACCGCGCCGGCACGCCCTGGCTCTACACCGCCTGCGGATGCT
GCATTCCCTGCCGTACCGGCTGGGAAACCCTGTGCCCGAGCCAGAAGAACTCAGGTTAT
TCCGTCAACGGCAGCTTTGCCGAATATGGCCTTGCCGATCCGAAATTCGTCGGCCGCCT
GCCTGACAATCTCGATTTCGGCCCAGCCGCACCCGTGCTCTGCGCCGGCGTTACAGTCT
ATAAGGGCCTGAAGGAAACCGAAGTCAGGCCCGGTGAATGGGTGGTCATTTCAGGCATT
GGCGGGCTTGGCCACATGGCCGTGCAATATGCGAAAGCCATGGGCATGCATGTGGTTGC
CGCCGATATTTTCGACGACAAGCTGGCGCTTGCCAAAAAGCTCGGAGCCGACGTCGTCG
TCAACGGCCGCGCGCCTGACGCGGTGGAGCAAGTGCAAAAGGCAACCGGCGGCGTCCAT
GGCGCGCTGGTGACGGCGGTTTCACCGAAGGCCATGGAGCAGGCTTATGGCTTCCTGCG
CTCCAAGGGCACGATGGCGCTTGTCGGTCTGCCGCCGGGCTTCATCTCCATTCCGGTGT
TCGACACGGTGCTGAAGCGCATCACGGTGCGTGGCTCCATCGTCGGCACGCGGCAGGAT
CTGGAGGAGGCGTTGACCTTCGCCGGTGAAGGCAAGGTGGCCGCCCACTTCTCGTGGGA
CAAGCTCGAAAACATCAATGATATCTTCCATCGCATGGAAGAGGGCAAGATCGACGGCC
GTATCGTCGTGGATCTCGCCGCCTGA

**Amino acid sequence (SEQ ID NO:6)**
MTKTMKAAVVRAFGKPLTIEEVAIPDPGPGEILINYKATGVCHTDLHAATGDWPVKPNP
PFIPGHEGAGYVAKIGAGVTGIKEGDRAGTPWLYTACGCCIPCRTGWETLCPSQKNSGY
SVNGSFAEYGLADPKFVGRLPDNLDFGPAAPVLCAGVTVYKGLKETEVRPGEWVVISGI
GGLGHMAVQYAKAMGMHVVAADIFDDKLALAKKLGADVVVNGRAPDAVEQVQKATGGVH
GALVTAVSPKAMEQAYGFLRSKGTMALVGLPPGFISIPVFDTVLKRITVRGSIVGTRQD
LEEALTFAGEGKVAAHFSWDKLENINDIFHRMEEGKIDGRIVVDLAA

*FIG. 5*

36

**ADH4 (NP_535613.1)**
**DNA sequence (SEQ ID NO:7)**
ATGACCGGGGCGAACCAGCCTTGGGAGGTTCAAGAGGTTCCCGTTCCGAAGGCAGAGCC
AGGACTTGTCCTTGTTAAAATCCACGCCTCCGGCATGTGCTACACGGACGTGTGGGCGA
CGCAGGGTGCCGGTGGCGACATCTATCCGCAGACCCCCGGCCATGAGGTTGTCGGCGAG
ATCATCGAGGTCGGCGCGGGCGTTCATACGCGCAAGGTGGGAGACCGGGTCGGCACCAC
CTGGGTGCAGTCCTCTTGTGGACGATGCTCCTACTGCCGCCAGAACCGTCCGTTGACCG
GCCAGACAGCCATGAACTGCGATTCACCCAGGACAACGGGGTTCGCGACGCAAGGCGGG
CACGCAGAGTACATCGCGATCTCTGCTGAAGGCACAGTGTTATTACCCGACGGGCTCGA
CTACACGGATGCCGCACCCATGATGTGCGCAGGCTACACGACCTGGAGCGGCTTGCGCG
ACGCCGAGCCCAAACCTGGTGACAGAATTGCGGTACTTGGCATCGGCGGGCTGGGGCAC
GTCGCCGTGCAGTTCTCCAAAGCCTTGGGGTTTGAGACCATCGCGATCACGCATTCACC
CGACAAGCACAAGTTGGCCACCGATCTTGGTGCAGACATCGTCGTCGCCGATGGCAAAG
AGTTATTGGAGGCCGGCGGTGCGGACGTTCTTCTGGTTACGACCAACGACTTCGACACC
GCCGAAAAAGCGATGGCGGGCGTAAGGCCTGACGGGCGCATCGTTCTTTGCGCGCTCGA
CTTCAGCAAGCCGTTCTCGATCCCGTCCGACGGCAAGCCGTTCCACATGATGCGCCAAC
GCGTGGTTGGGTCCACGCATGGCGGACAGCACTATCTCGCCGAAATCCTCGATCTCGCC
GCCAAGGGCAAGGTCAAGCCGATTGTCGAGACCTTCGCCCTCGAGCAGGCAACCGAGGC
ATATGAGCGGCTATCCACCGGGAAGATGCGCTTCCGGGGCGTGTTCCTTCCGCACGGCG
CTTGA

**Amino acid sequence (SEQ ID NO:8)**
MTGANQPWEVQEVPVPKAEPGLVLVKIHASGMCYTDVWATQGAGGDIYPQTPGHEVVGE
IIEVGAGVHTRKVGDRVGTTWVQSSCGRCSYCRQNRPLTGQTAMNCDSPRTTGFATQGG
HAEYIAISAEGTVLLPDGLDYTDAAPMMCAGYTTWSGLRDAEPKPGDRIAVLGIGGLGH
VAVQFSKALGFETIAITHSPDKHKLATDLGADIVVADGKELLEAGGADVLLVTTNDFDT
AEKAMAGVRPDGRIVLCALDFSKPFSIPSDGKPFHMMRQRVVGSTHGGQHYLAEILDLA
AKGKVKPIVETFALEQATEAYERLSTGKMRFRGVFLPHGA

*FIG. 6*

**ADH5 (NP_533663.1)**
**Amino acid sequence (SEQ ID NO:10)**
MTMHAIQFVEKGRAVLAELPVADLPPGHALVRVKASGLCHTDIDVLHARYGDGAFPVIP
GHEYAGEVAAVASDVTVFKAGDRVVVDPNLPCGTCASCRKGLTNLCSTLKAYGVSHNGG
FAEFSVVRADHLHGIGSMPYHVAALAEPLACVVNGMQSAGIGESGVVPENALVFGAGPI
GLLLALSLKSRGIATVTMADINESRLAFAQDLGLQTAVSGSEALSRQRKEFDFVADATG
IAPVAEAMIPLVADGGTALFFGVCAPDARISVAPFEIFRRQLKLVGSHSLNRNIPQALA
ILETDGEVMARLVSHRLPLSEMLPFFTKKPSDPATMKVQFAAE

**DNA sequence (SEQ ID NO:9)**
ATGACCATGCATGCCATTCAATTCGTCGAGAAGGGACGCGCCGTGCTGGCGGAACTCCC
CGTCGCCGATCTGCCGCCGGGCCATGCGCTCGTGCGGGTCAAGGCTTCGGGGCTTTGCC
ATACCGATATCGACGTGCTGCATGCGCGTTATGGCGACGGTGCGTTCCCCGTCATTCCG
GGGCATGAATATGCTGGCGAAGTCGCAGCCGTGGCTTCCGATGTGACAGTCTTCAAGGC
TGGCGACCGGGTTGTCGTCGATCCCAATCTGCCCTGTGGCACCTGCGCCAGCTGCAGGA
AAGGGCTGACCAACCTTTGCAGCACATTGAAAGCTTACGGCGTTTCCCACAATGGCGGC
TTTGCGGAGTTCAGTGTGGTGCGTGCCGATCACCTGCACGGTATCGGTTCGATGCCCTA
TCACGTCGCGGCGCTGGCTGAGCCGCTTGCCTGTGTTGTCAATGGCATGCAGAGTGCGG
GTATTGGCGAGAGTGGCGTGGTGCCGGAGAATGCGCTTGTTTTCGGTGCTGGGCCCATC
GGCCTGCTGCTTGCCCTGTCGCTGAAATCACGCGGCATTGCGACGGTGACGATGGCCGA
TATCAATGAAAGCAGGCTGGCCTTTGCCCAGGACCTCGGGCTTCAGACGGCGGTATCCG
GCTCGGAAGCGCTCTCGCGGCAGCGGAAGGAGTTCGATTTCGTGGCCGATGCGACGGGT
ATTGCCCCGGTCGCCGAGGCGATGATCCCGCTGGTTGCGGATGGCGGCACGGCGCTATT
CTTCGGCGTCTGCGCGCCGGATGCCCGTATTTCGGTGGCACCGTTTGAAATCTTCCGGC
GCCAGCTGAAACTTGTCGGCTCGCATTCGCTGAACCGCAACATACCGCAGGCGCTTGCC
ATTCTGGAGACGGATGGCGAGGTCATGGCGCGGCTCGTTTCGCACCGCTTGCCGCTTTC
GGAGATGCTGCCGTTCTTTACGAAAAAACCGTCTGATCCGGCGACGATGAAAGTGCAAT
TTGCAGCCGAATGA

*FIG. 7*

**ADH6 (NP 532825.1)**
**DNA sequence (SEQ ID NO:11)**
```
ATGCGCGCGCTTTATTACGAACGATTCGGCGAGACCCCTGTAGTCGCGTCCCTGCCTGA
TCCGGCACCGAGCGATGGCGGCGTGGTGATTGCGGTGAAGGCAACCGGCCTCTGCCGCA
GCGACTGGCATGGCTGGATGGGACATGACACGGATATCCGTCTGCCGCATGTGCCCGGC
CACGAGTTCGCCGGCGTCATCTCCGCAGTCGGCAGAAACGTCACCCGCTTCAAGACGGG
TGATCGCGTTACCGTGCCTTTCGTCTCCGGCTGCGGCCATTGCCATGAGTGCCGCTCCG
GCAATCAGCAGGTCTGCGAAACGCAGTTCCAGCCCGGCTTCACCCATTGGGGTTCCTTC
GCCGAATATGTCGCCATCGACTATGCCGATCAGAACCTCGTGCACCTGCCGGAATCGAT
GAGTTACGCCACCGCCGCCGGCCTCGGTTGCCGTTTCGCCACCTCCTTCCGGGCGGTGA
CGGATCAGGGACGCCTGAAGGGCGGCGAATGGCTGGCTGTCCATGGCTGCGGCGGTGTC
GGTCTCTCCGCCATCATGATCGGCGCCGGCCTCGGCGCACAGGTCGTCGCCATCGATAT
TGCCGAAGACAAGCTCGAACTCGCCCGGCAACTGGGTGCAACCGCAACCATCAACAGCC
GCTCCGTTGCCGATGTCGCCGAAGCGGTGCGCGACATCACCGGTGGCGGCGCGCATGTG
TCGGTGGATGCGCTTGGCCATCCGCAGACCTGCTGCAATTCCATCAGCAACCTGCGCCG
GCGCGGACGCCATGTGCAGGTGGGGCTGATGCTGGCAGACCATGCCATGCCGGCCATTC
CCATGGCCCGGGTGATCGCTCATGAGCTGGAGATCTATGGCAGCCACGGCATGCAGGCA
TGGCGTTACGAGGACATGCTGGCCATGATCGAAAGCGGCAGGCTTGCGCCGGAAAAGCT
GATTGGCCGCCATATCTCGCTGACCGAAGCGGCCGTCGCCCTGCCCGGAATGGATAGGT
TCCAGGAGAGCGGCATCAGCATCATCGACCGGTTCGAATAG
```

**Amino acid sequence (SEQ ID NO:12)**
```
MNLRTNDEAMMRALYYERFGETPVVASLPDPAPSDGGVVIAVKATGLCRSDWHGWMGHD
TDIRLPHVPGHEFAGVISAVGRNVTRFKTGDRVTVPFVSGCGHCHECRSGNQQVCETQF
QPGFTHWGSFAEYVAIDYADQNLVHLPESMSYATAAGLGCRFATSFRAVTDQGRLKGGE
WLAVHGCGGVGLSAIMIGAGLGAQVVAIDIAEDKLELARQLGATATINSRSVADVAEAV
RDITGGGAHVSVDALGHPQTCCNSISNLRRRGRHVQVGLMLADHAMPAIPMARVIAHEL
EIYGSHGMQAWRYEDMLAMIESGRLAPEKLIGRHISLTEAAVALPGMDRFQESGISIID
RFE
```

*FIG. 8*

## ADH7 (NP_533479.1)
### DNA sequence (SEQ ID NO:13)
ATGCTGGCGATTTTCTGTGACACTCCCGGTCAATTAACCGCCAAGGATCTGCCGAACCC
CGTGCGCGGCGAAGGTGAAGTCCTGGTACGTATTCGCCGGATTGGCGTTTGCGGCACGG
ATCTGCACATCTTTACCGGCAACCAGCCCTATCTTTCCTATCCGCGGATCATGGGTCAC
GAACTTTCCGGCACGGTTGAGGAGGCACCCGCTGGCAGCCACCTTTCCGCTGGCGATGT
GGTGACCATAATTCCCTATATGTCCTGCGGGAAATGCAATGCCTGCCTGAAGGGTAAGA
GCAATTGCTGCCGCAATATCGGTGTGCTTGGCGTTCATCGCGATGGCGGCATGGTGGAA
TATCTGAGCGTGCCGCAGCAATTCGTGCTGAAGGCGGAGGGGCTGAGCCTCGACCAGGC
AGCCATGACGGAATTTCTGGCGATCGGTGCCCATGCGGTGCGTCGCGGTGCCGTCGAAA
AAGGGCAAAAGGTCCTGATCGTCGGTGCCGGCCCGATCGGCATGGCGGTTGCTGTCTTT
GCGGTTCTCGATGGCACGGAAGTGACGATGATCGACGGTCGCACCGACCGGCTGGATTT
CTGCAAGGACCACCTCGGTGTCGCTCATACAGTCGCCCTCGGCGACGGTGACAAAGATC
GTCTGTCCGACATTACCGGTGGCAATTTCTTCGATGCGGTGTTTGATGCGACCGGCAAT
CCGAAAGCCATGGAGCGCGGTTTCTCCTTCGTCGGTCACGGCGGCTCCTATGTTCTGGT
GTCCATCGTCGCCAGCGATATCAGCTTCAACGACCCGGAATTTCACAAGCGTGAGACGA
CGCTGCTCGGCAGCCGCAACGCGACGGCTGATGATTTCGAGCGGGTGCTTCGCGCCTTG
CGCGAAGGGAAAGTGCCGGAGGCACTAATCACCCATCGCATGACACTTGCCGATGTTCC
CTCGAAGTTCGCCGGCCTGACCGATCCGAAAGCCGGAGTCATCAAGGGCATGGTGGAGG
TCGCATGA

### Amino acid sequence (SEQ ID NO:14)
MLAIFCDTPGQLTAKDLPNPVRGEGEVLVRIRRIGVCGTDLHIFTGNQPYLSYPRIMGH
ELSGTVEEAPAGSHLSAGDVVTIIPYMSCGKCNACLKGKSNCCRNIGVLGVHRDGGMVE
YLSVPQQFVLKAEGLSLDQAAMTEFLAIGAHAVRRGAVEKGQKVLIVGAGPIGMAVAVF
AVLDGTEVTMIDGRTDRLDFCKDHLGVAHTVALGDGDKDRLSDITGGNFFDAVFDATGN
PKAMERGFSFVGHGGSYVLVSIVASDISFNDPEFHKRETTLLGSRNATADDFERVLRAL
REGKVPEALITHRMTLADVPSKFAGLTDPKAGVIKGMVEVA

# FIG. 9

<u>**ADH8 (NP 535818.1)**</u>
**DNA sequence (SEQ ID NO:15)**
```
GTGAAAGCCTTCGTCGTCGACAAGTACAAGAAGAAGGGCCCGCTGCGTCTGGCCGACAT
GCCCAATCCGGTCATCGGCGCCAATGATGTGCTGGTTCGCATCCATGCCACTGCCATCA
ATCTTCTCGACTCCAAGGTGCGCGACGGGGAATTCAAGCTGTTCCTGCCCTATCGTCCT
CCCTTCATTCTCGGTCATGATCTGGCCGGAACGGTCATCCGCGTCGGCGCGAATGTACG
GCAGTTCAAGACAGGCGACGAGGTTTTCGCTCGCCCGCGTGATCACCGGGTCGGAACCT
TCGCAGAAATGATTGCGGTCGATGCCGCAGACCTTGCGCTGAAGCCAACGAGCCTGTCC
ATGGAGCAGGCAGCGTCGATCCCGCTCGTCGGACTGACTGCCTGGCAGGCGCTTATCGA
GGTTGGCAAGGTCAAGTCCGGCCAGAAGGTTTTCATCCAGGCCGGTTCCGGCGGTGTCG
GCACCTTCGCCATCCAGCTTGCCAAGCATCTCGGCGCTACCGTGGCCACGACCACCAGC
GCCGCGAATGCCGAACTGGTCAAAAGCCTCGGCGCAGATGTGGTGATCGACTACAAGAC
GCAGGACTTCGAACAGGTGCTGTCCGGCTACGATCTCGTCCTGAACAGCCAGGATGCCA
AGACGCTGGAAAAGTCGTTGAACGTGCTGAGACCGGGCGGAAAGCTCATTTCGATCTCC
GGTCCGCCGGATGTTGCCTTTGCCAGATCGTTGAAACTGAATCCGCTCCTGCGTTTTGT
CGTCAGAATGCTGAGCCGTGGTGTCCTGAAAAAGGCAAGCAGACGCGGTGTCGATTACT
CTTTCCTGTTCATGCGCGCCGAAGGTCAGCAATTGCATGAGATCGCCGAACTGATCGAT
GCCGGCACCATCCGTCCGGTCGTCGACAAGGTGTTTCAATTTGCGCAGACGCCCGACGC
CCTGGCCTATGTCGAGACCGGACGGGCAAGGGGCAAGGTTGTGGTTACATACGCATCCT
AG
```

**Amino acid sequence (SEQ ID NO:16)**
```
MPSLCRKPWLSSLPDLINVSHWRKPVKAFVVDKYKKKGPLRLADMPNPVIGANDVLVR1
HATAINLLDSKVRDGEFKLFLPYRPPFILGHDLAGTVIRVGANVRQFKTGDEVFARPRD
HRVGTFAEMIAVDAADLALKPTSLSMEQAASIPLVGLTAWQALIEVGKVKSGQKVFIQA
GSGGVGTFAIQLAKHLGATVATTTSAANAELVKSLGADVVIDYKTQDFEQVLSGYDLVL
NSQDAKTLEKSLNVLRPGGKLISISGPPDVAFARSLKLNPLLRFVVRMLSRGVLKKASR
RGVDYSFLFMRAEGQQLHEIAELIDAGTIRPVVDKVFQFAQTPDALAYVETGRARGKVV
VTYAS
```

*FIG. 10*

**ADH9 (NP_534572.1)**
**DNA sequence (SEQ ID NO:17)**
ATGAAAGCGATTGTCGCCCACGGGGCAAAGGATGTGCGCATCGAAGACCGGCCGGAGGA
AAAGCCGGGTCCGGGCGAGGTGCGGCTCCGTCTGGCGAGGGGCGGGATCTGCGGCAGTG
ATCTGCATTATTACAATCATGGCGGTTTCGGCGCCGTGCGGCTTCGTGAACCCATGGTG
CTGGGCCATGAGGTTTCCGCCGTCATCGAGGAACTGGGCGAAGGCGTTGAGGGGCTGAA
GATCGGCGGTCTGGTGGCGGTTTCGCCGTCGCGCCCATGCCGAACCTGCCGCTTCTGCC
AGGAGGGTCTGCACAATCAGTGCCTCAACATGCGGTTTTATGGCAGCGCCATGCCTTTC
CCGCATATTCAGGGCGCGTTCCGGGAAATTCTGGTGGCGGACGCCCTGCAATGCGTGCC
GGCCGATGGTCTCAGCGCCGGGGAAGCCGCCATGGCGGAACCGCTGGCGGTGACGCTGC
ATGCCACACGCCGGGCCGGCGATTTGCTGGGAAAACGTGTGCTCGTCACGGGTTGCGGC
CCCATCGGCATTCTCTCCATTCTGGCTGCGCGCCGGGCGGGTGCTGCTGAAATCGTCGC
CACCGACCTTTCCGATTTCACGCTCGGCAAGGCGCGTGAAGCGGGGGCGGACCGTGTCA
TCAACAGCAAGGATGAGCCCGATGCGCTCGCCGCTTATGGTGCAAACAAGGGAACCTTC
GACATTCTCTATGAATGCTCGGGTGCGGCCGTGGCGCTTGCCGGCGGCATTACGGCACT
GCGGCCGCGCGGCATCATCGTCCAGCTCGGGCTCGGCGGCGATATGAGCCTGCCGATGA
TGGCGATCACAGCCAAGGAACTCGACCTGCGTGGTTCCTTTCGCTTCCACGAGGAATTC
GCCACCGGCGTCGAGCTGATGCGCAAGGGCCTGATCGACGTCAAACCCTTCATCACCCA
GACCGTCGATCTTGCCGACGCCATCTCGGCCTTCGAATTCGCCTCGGATCGCAGCCGCG
CCATGAAGGTGCAGATCGCCTTTTCCTAA

**Amino acid sequence (SEQ ID NO:18)**
MKAIVAHGAKDVRIEDRPEEKPGPGEVRLRLARGGICGSDLHYYNHGGFGAVRLREPMV
LGHEVSAVIEELGEGVEGLKIGGLVAVSPSRPCRTCRFCQEGLHNQCLNMRFYGSAMPF
PHIQGAFREILVADALQCVPADGLSAGEAAMAEPLAVTLHATRRAGDLLGKRVLVTGCG
PIGILSILAARRAGAAEIVATDLSDFTLGKAREAGADRVINSKDEPDALAAYGANKGTF
DILYECSGAAVALAGGITALRPRGIIVQLGLGGDMSLPMMAITAKELDLRGSFRFHEEF
ATGVELMRKGLIDVKPFITQTVDLADAISAFEFASDRSRAMKVQIAFS

*FIG. 11*

42

**ADH10 (NP_534767.1)**
**DNA sequence (SEQ ID NO:19)**
ATGCCGATGGCGCTCGGGCACGAAGCGGCGGGCGTCGTCGAGGCATTGGGCGAAGGCGT
GCGCGATCTTGAGCCCGGCGATCATGTGGTCATGGTCTTCATGCCCAGTTGCGGACATT
GCCTGCCCTGTGCGGAAGGCAGGCCCGCTCTGTGCGAGCCGGGCGCCGCCGCCAATGCA
GCAGGCAGGCTGTTGGGTGGCGCCACCCGCCTGAACTATCATGGCGAGGTCGTCCATCA
TCACCTTGGTGTGTCGGCCTTTGCCGAATATGCCGTGGTGTCGCGCAATTCGCTGGTCA
AGATCGACCGCGATCTTCCATTTGTCGAGGCGGCACTCTTCGGCTGCGCGGTTCTCACC
GGCGTCGGCGCCGTCGTGAATACGGCAAGGGTCAGGACCGGCTCGACTGCGGTCGTCAT
CGGACTTGGCGGTGTGGGCCTTGCCGCGGTTCTCGGAGCCCGGGCGGCCGGTGCCAGCA
AGATCGTCGCCGTCGACCTTTCGCAGGAAAAGCTTGCACTCGCCAGCGAACTGGGCGCG
ACCGCCATCGTGAACGGACGCGATGAGGATGCCGTCGAGCAGGTCCGCGAGCTCACTTC
CGGCGGTGCCGATTATGCCTTCGAGATGGCAGGGTCTATTCGCGCCCTCGAAAACGCCT
TCAGGATGACCAAACGTGGCGGCACCACCGTTACCGCCGGTCTGCCACCGCCGGGTGCG
GCCCTGCCGCTCAACGTCGTGCAGCTCGTCGGCGAGGAGCGGACACTCAAGGGCAGCTA
TATCGGCACCTGTGTGCCTCTCCGGGATATTCCGCGCTTCATCGCCCTTTATCGCGACG
GCCGGTTGCCGGTGAACCGCCTTCTGAGCGGAAGGCTGAAGCTAGAAGACATCAATGAA
GGGTTCGACCGCCTGCACGACGGAAGCGCCGTTCGGCAAGTCATCGAATTCTGA

**Amino acid sequence (SEQ ID NO:20)**
MPMALGHEAAGVVEALGEGVRDLEPGDHVVMVFMPSCGHCLPCAEGRPALCEPGAAANA
AGRLLGGATRLNYHGEVVHHHLGVSAFAEYAVVSRNSLVKIDRDLPFVEAALFGCAVLT
GVGAVVNTARVRTGSTAVVIGLGGVGLAAVLGARAAGASKIVAVDLSQEKLALASELGA
TAIVNGRDEDAVEQVRELTSGGADYAFEMAGSIRALENAFRMTKRGGTTVTAGLPPPGA
ALPLNVVQLVGEERTLKGSYIGTCVPLRDIPRFIALYRDGRLPVNRLLSGRLKLEDINE
GFDRLHDGSAVRQVIEF

*FIG. 12*

**ADH11 (NP_535575.1)**
**DNA sequence (SEQ ID NO:21)**
ATGACCCAACCCGCCACCGCAGCCGTACTGGAAGAAAAAAACGGCCGTTTCATTCTTCG
TGAAGTGAAGCTTGAGGCGCCGCGCCCCGACGAAGTGCTGATTCGCATGGTTGCTACGG
GTATTTGCGCGACCGATGCTCATGTCAGGCAACAGCTCATGCCAACTCCGCTGCCGGCG
ATCTTGGGCCATGAAGGCGCCGGCATCGTCGAACGCGTTGGATCGACCGTATCGCATCT
CAAGCCCGGCGATCATGTCGTTCTTTCCTATCACTCCTGCGGCCACTGCAAGCCCTGCA
TGTCTTCCCATGCGGCCTACTGCGACCACGTCTGGGAAACGAATTTCGCAGGCGCCAGG
CTCGATGGAACGATCGGCGTTGCGGCGCCTGATGGGAACACGCTCCATGCGCACTTCTT
TGGTCAGTCTTCATTCTCCACCTATGCGCTCGCTCATCAGCGCAATGCCGTCAAGGTCC
CGGACGATGTTCCGCTCGAGCTCCTCGGACCGCTCGGTTGCGGGTTCCAGACCGGAGCC
GGCTCGGTCTTGAACGCGCTCAAAGTGCCGGTAGGCGCCTCTATCGCCATTTTCGGGGT
AGGGGCAGTGGGGTTGTCGGCGATCATGGCTGCCAAGGTCGCCGATGCCGCCGTCATTA
TCGCCATTGATGTCAATACCGAACGGCTGAAGCTCGCTTCCGAGCTCGGCGCGACGCAT
TGCGTCAACCCGCGTGAACAAGCCGATGTTGCCTCGGCGATCAGGGATATCGCGCCTCG
CGGCGTCGAATACGTTCTCGACACGAGCGGTCGGAAGGAGAACCTCGACGGCGGCATCG
GCGCTCTTGCTCCGATGGGGCAGTTCGGTTTTGTCGCCTTCAACGACCATTCGGGCGCG
GTTGTCGATGCCTCCCGGCTCACGGTAGGGCAAAGCCTCATCGGGATTATCCAGGGCGA
TGCCATTTCCGGCCTGATGATTCCGGAACTGGTCGGTCTCTATCGAAGCGGCCGTTTCC
CGTTCGACAGGCTGCTCACCTTCTACGACTTCGCCGACATCAATGAGGCATTTGACGAT
GTCGCGGCAGGACGGGTGATCAAGGCCGTCCTGCGCTTTCCCCCGCAAGCTGCTTAA

**Amino acid sequence (SEQ ID NO:22)**
MSRITRPGMRNQPLEEKMTQPATAAVLEEKNGRFILREVKLEAPRPDEVLIRMVATGIC
ATDAHVRQQLMPTPLPAILGHEGAGIVERVGSTVSHLKPGDHVVLSYHSCGHCKPCMSS
HAAYCDHVWETNFAGARLDGTIGVAAPDGNTLHAHFFGQSSFSTYALAHQRNAVKVPDD
VPLELLGPLGCGFQTGAGSVLNALKVPVGASIAIFGVGAVGLSAIMAAKVADAAVIIAI
DVNTERLKLASELGATHCVNPREQADVASAIRDIAPRGVEYVLDTSGRKENLDGGIGAL
APMGQFGFVAFNDHSGAVVDASRLTVGQSLIGIIQGDAISGLMIPELVGLYRSGRFPFD
RLLTFYDFADINEAFDDVAAGRVIKAVLRFPPQAA

*FIG. 13*

**ADH12 (NP_532098.1)**
**DNA sequence (SEQ ID NO:23)**
ATGCGCGGAGTCGTCATTCATGCAGCAAAAGACCTGCGGGTAGAGGACGTTGCTGGCCA
GCCACTTGCCGCGGACGAGGTGCGGGTGGCCGTTGCCGTCGGCGGAATTTGCGGCTCGG
ATCTGCATTATTATAACCATGGCGGCTTCGGCACGGTGCGCGTGCGCGAGCCGATGGCG
CTCGGTCATGAGTTTGCCGGTACGGTGGTTGAGGTGGGCAGTTCGGTCTCGCATCTCGT
GCCCGGCATGCGCGTGGCCGTCAATCCGAGCCTGCCTTGCGGCACCTGCCGCTATTGCG
CTCAGGGCAGGCAGAATCAGTGCCTGGACATGCGCTTCATGGGCAGCGCCATGCGCTCC
CCCCATGTTCAGGGCGGTTTCCGTGAAGTCGTGACCGTCCATTCAACGCAACCGGTACA
GATCGCCGACGGACTTTCCATGGGTGAGGCAGCCATGGCCGAGCCTTTGGCCGTGTGCC
TCCATGCCGCGCGTCAGGCGGGATCGCTTCTGGGCAAGACGGTGCTGATAACCGGTGCC
GGGCCGATCGGCATGCTTAGCCTGCTGGTTGCCCGTCTTGCCGGCGCGGCGCATATCGT
CGTTACCGATGTCGCCGATGCACCGCTCGATCTGGCGCGACGTATCGGCGCGGATGAAG
CCGTCAACATCCTGCGCGATGCCGACATGCTTGAAAAATACCGATTTGAAAAAGGCGTC
TTCGACGTCCTGTTCGAAGCCTCCGGCAATCAGGCGGCACTTCTCCCGGCGCTGGATCT
GCTCCGGCCGGGCGGTATTATCGTCCAGCTCGGTCTTGGCGGAGACTTCACCATTCCGA
TGAACCTCATCGTTGCCAAAGAGCTGCAGCTGCGCGGAACGTTCCGCTTCCACGAGGAA
TTTGCCCAGGCGGTGAATATGATGGGACGTGGCCTGATCGACGTTAAGCCTTTGATCAG
CGCCACATTGCCGTTCGATCAGGCCCGCGAGGCTTTCGATCTTGCCGGTGACCGCGCAA
AAAGCATGAAAGTGCAGCTTGCCTTCAGCGGAGCAGCCTGA

**Amino acid sequence (SEQ ID NO:24)**
MECCRFSRTAAILDANRNWREETRMRGVVIHAAKDLRVEDVAGQPLAADEVRVAVAVGG
ICGSDLHYYNHGGFGTVRVREPMALGHEFAGTVVEVGSSVSHLVPGMRVAVNPSLPCGT
CRYCAQGRQNQCLDMRFMGSAMRSPHVQGGFREVVTVHSTQPVQIADGLSMGEAAMAEP
LAVCLHAARQAGSLLGKTVLITGAGPIGMLSLLVARLAGAAHIVVTDVADAPLDLARRI
GADEAVNILRDADMLEKYRFEKGVFDVLFEASGNQAALLPALDLLRPGGIIVQLGLGGD
FTIPMNLIVAKELQLRGTFRFHEEFAQAVNMMGRGLIDVKPLISATLPFDQAREAFDLA
GDRAKSMKVQLAFSGAA

*FIG. 14*

**ADH13 (NP_535348.1)**
**DNA sequence (SEQ ID NO:25)**
ATGAAGGCAGCCGTTTACGATCAAGCAGGACCTCCGGATGTTTTGACGTACAGGGACGT
CGCCGACCCGATTGTAGGTCCGGATGATGTCCTCATCGCAGTGGAAGCCATTTCGATTG
AAGGAGGAGACTTGATCAATCGTCGATCCACGCCGCCTCCTGGCCGCCCGTGGATAGTC
GGCTATGCAGCATCTGGGCGCGTCGTGGGGGCCGGTGCGAACGTGAGGGACCGCAAAGT
CGGAGACAGGGTTACTGCCTTTGACATGCAGGGTTCGCACGCCGAACTCTGGGCCGTGC
CAGCGATCCGAACGTGGCTTTTGCCATCCGGCGTGGATGCAGCGTCGGCTGCCGCTTTG
CCGATATCGTTTGGTACTGCCCACCATTGTCTTTTTGCCAGAGGTGGCCTTCTGCGCAA
CCAGACGGTTCTTGTACAGGCAGCGGCGGGTGGAGTTGGCCTCGCCGCAGTTCAGCTCG
CGGCTCAAGCCGGCGCAACCGTCATCGCCGTCTCAAGTGGAGAAAGCCGGCTGCAAAGG
ATATCTTCCCTTGGGGCTGATCACGTTGTCGATCGGTCGATGGGGAACGTTGTCGAGGC
TGTCAGACAGAACACGGGAGGCAAAGGAGTCGATCTCGTGATTGATCCTGTCGGTGTCA
CCTTGTCCGCTTCTCTGACTCTCCTGGCACCAGAAGGACGTCTTGTGTTTGTGGGAAAC
GCTGGGGGCGGAAGCCTGACCATCGATCTGTGGCCAGCCATGCAGTCAAATCAGACTTT
GCTCGGAGTTTTCATGGGCCCGCTATTAGAGAGACCTCAGGTTCGTGCGACGGTAGATG
AGATGCTTCAAATGCTCGATCGTCGCGAAATCCGTGTGATGATCGAAAAGACGTTTCCG
CTCTCGGAAGCGGCAGCCGCTCATGATTTTGCAGAAAATGCGAAACCGCTTGGCCGGGT
GATTATGGAGCCGTGA

**Amino acid sequence (SEQ ID NO:26)**
MKAAVYDQAGPPDVLTYRDVADPIVGPDDVLIAVEAISIEGGDLINRRSTPPPGRPWIV
GYAASGRVVGAGANVRDRKVGDRVTAFDMQGSHAELWAVPAIRTWLLPSGVDAASAAAL
PISFGTAHHCLFARGGLLRNQTVLVQAAAGGVGLAAVQLAAQAGATVIAVSSGESRLQR
ISSLGADHVVDRSMGNVVEAVRQNTGGKGVDLVIDPVGVTLSASLTLLAPEGRLVFVGN
AGGGSLTIDLWPAMQSNQTLLGVFMGPLLERPQVRATVDEMLQMLDRREIRVMIEKTFP
LSEAAAAHDFAENAKPLGRVIMEP

*FIG. 15*

### ADH14 (NP_532354.1)

**DNA sequence (SEQ ID NO:27)**

ATGGACGTTCGCGCCGCCGTTGCCATTCAGGCAGGAAAAACCGCTCGAGGTCATGACCGT
TCAGCTTGAAGGTCCCCGCGCCGGTGAAGTGCTGATCGAAGTCAAGGCGACCGGCATCT
GCCACACCGACGATTTCACCCTCTCTGGCGCTGACCCGGAAGGCCTGTTCCCGGCAATC
CTCGGCCATGAAGGTGCGGGCATCGTCGTGGATGTCGGCCCCGGCGTCACCTCGGTCAA
GAAGGGCGACCACGTCATTCCGCTCTACACGCCGGAATGCCGCGAATGCTACTCCTGCA
CCTCGCGCAAGACCAATCTCTGCACCTCCATCCGCGCCACCCAGGGCCAGGGCGTGATG
CCTGACGGCACCTCGCGCTTCTCGATCGGCAAGGACAAGATTCACCACTATATGGGTTG
CTCGACCTTCTCGAATTTCACCGTCCTGCCGGAAATCGCGCTGGCCAAGATCAACCCGG
ACGCGCCCTTCGACAAGGTCTGCTACATCGGCTGCGGCGTCACGACCGGTATCGGCGCC
GTCATCAACACCGCCAAGGTCGAGATTGGCTCCACGGCGATCGTCTTCGGTCTCGGCGG
CATCGGTCTCAACGTGCTGCAGGGCCTGCGTCTTGCCGGTGCGGACATGATCATCGGCG
TCGATATCAACAACGACCGCAAGGCCTGGGGCGAAAAATTCGGCATGACCCACTTCGTC
AATCCGAAGGAAGTCGGCGACGACATCGTGCCCTATCTCGTCAACATGACGAAGCGTAA
TGGCGACCTCATCGGCGGCGCAGACTATACGTTCGACTGCACCGGCAATACCAAGGTCA
TGCGCCAGGCGCTGGAAGCCTCGCATCGCGGTTGGGGCAAGTCGGTCATCATCGGCGTC
GCCGGCGCCGGCCAGGAAATCTCCACCCGTCCGTTCCAGCTGGTCACCGGCCGTAACTG
GATGGGCACCGCCTTCGGCGGCGCGCGCGGCCGCACCGATGTGCCGAAGATTGTCGACT
GGTACATGGAAGGCAAGATCCAGATCGACCCGATGATCACCCACACCATGCCGCTCGAA
GACATCAACAAGGGCTTCGAGCTGATGCACAAGGGTGAATCGATCCGCGGCGTCGTTGT
TTATTGA

**Amino acid sequence (SEQ ID NO:28)**

MDVRAAVAIQAGKPLEVMTVQLEGPRAGEVLIEVKATGICHTDDFTLSGADPEGLFPAI
LGHEGAGIVVDVGPGVTSVKKGDHVIPLYTPECRECYSCTSRKTNLCTSIRATQGQGVM
PDGTSRFSIGKDKIHHYMGCSTFSNFTVLPEIALAKINPDAPFDKVCYIGCGVTTGIGA
VINTAKVEIGSTAIVFGLGGIGLNVLQGLRLAGADMIIGVDINNDRKAWGEKFGMTHFV
NPKEVGDDIVPYLVNMTKRNGDLIGGADYTFDCTGNTKVMRQALEASHRGWGKSVIIGV
AGAGQEISTRPFQLVTGRNWMGTAFGGARGRTDVPKIVDWYMEGKIQIDPMITHTMPLE
DINKGFELMHKGESIRGVVVY

*FIG. 16*

## ADH15 (NP_535561.1)

**DNA sequence (SEQ ID NO:29)**

ATGAAAGCGATGTCACTCAAATCCTTTGGCGGCCCAGAAGCCTTTGATCTTGTCGAAGT
TCCAAAGCCTCTTCCGAAGGCGGGGCAGGTTTTGGTACGGGTCCATGCCACATCGATCA
ATCCCCTCGACTACCAAGTTCGGCGAGGCGATTATCGCGACCTGGTGCCGTTGCCGGCA
ATTACCGGCCATGACGTATCGGGCGTTGTCGAAGCTACCGGTCCGGGGGTAACAATGTT
CGCTCCAGGAGACGAGGTCTGGTACACGCCACAGATCTTCGACGGGCCAGGCAGTTATG
CCGAATACCACGTTGCGAACGAAAATATCATCGGACGCAAACCCAGCTCGCTGACCCAT
CTTGAGGCTGCGAGCCTTAGCCTGGTTGGAGGAACCGCCTGGGAAGCGCTTGTCTCGCG
TGCTGCCCTGAGGGTTGGTGAAAGCATATTGATCCATGGCGGCGCTGGAGGGGTAGGGC
ACGTCGCTATCCAAGTTGCGAAAGCCATCGGAGCAAAGGTCTACACGACCGTCCGTGAA
GAAAACTTCGAGTTTGCGCGAAGTGTCGGAGCTGACGTCGTCATTGATTACAGAAAAGA
GGATTATGTCGCCGCCATCATGCGGGAGACTGAAGGCCTCGGAGTAGACGTCGTGTTCG
ACACTCTCGGCGGCGAAACATTGTCCCACAGCCCGAAGGTGCTTGCACAATTCGGTCGT
GTCGTCTCGATCGTGGACATCGCCCGGCCGCAAAATCTCATTGAGGCATGGGGCAGGAA
CGCGAGTTACCACTTCGTCTTCACAAGGCAGAACCAAGGCAAGCTCAACGAGCTGAACG
TTTTGGTGGAACGTGGTCAGCTGAGGCCGCACGTGGGCGCCGTCTATTCGCTCGCCGAC
CTTCCGCTTGCCCATGCGCTGCTCGAGAAACCAAACAACGGTTTGCGCGGTAAGATCGC
GATTGCCATTGACCCGCAGGCTGAGACAAAGGTGCAATCATGA

**Amino acid sequence (SEQ ID NO:30)**

MRPAMLQRRSMFLVRRRRPESLPSIEQEPEMKAMSLKSFGGPEAFDLVEVPKPLPKAGQ
VLVRVHATSINPLDYQVRRGDYRDLVPLPAITGHDVSGVVEATGPGVTMFAPGDEVWYT
PQIFDGPGSYAEYHVANENIIGRKPSSLTHLEAASLSLVGGTAWEALVSRAALRVGESI
LIHGGAGGVGHVAIQVAKAIGAKVYTTVREENFEFARSVGADVVIDYRKEDYVAAIMRE
TEGLGVDVVFDTLGGETLSHSPKVLAQFGRVVSIVDIARPQNLIEAWGRNASYHFVFTR
QNQGKLNELNVLVERGQLRPHVGAVYSLADLPLAHALLEKPNNGLRGKIAIAIDPQAET
KVQS

FIG. 17

**ADH16 (NP_532255.1)**
**DNA sequence (SEQ ID NO:31)**
ATGGATATGAGCAGGAACAGAGGCGTCGTTTACCTGAAACCAGGCCAGGTCGAAGTCCG
CGACATCGACGACCCGAAGCTTGAGGCGCCGGATGGCCGCCGCATCGAGCACGGCGTCA
TTCTCAAGGTGATTTCCACGAATATCTGCGGCTCCGACCAGCACATGGTGCGCGGCCGC
ACCACCGCGATGCCGGGCCTCGTCCTTGGCCATGAAATCACCGGCGAAGTCATCGAAAA
AGGCATCGACGTCGAAATGCTGCAGGTCGGCGACATCGTCTCCGTGCCGTTCAACGTCG
CCTGCGGCCGTTGCCGCTGCTGCAAGTCGCAGGATACCGGCGTCTGCCTGACGGTGAAC
CCGTCACGCGCCGGCGGCGCTTACGGTTATGTCGATATGGGCGGCTGGATCGGCGGACA
GGCCCGTTATGTCACGATCCCTTATGCCGATTTCAACCTTCTGAAATTCCCCGATCGCG
ACAAGGCGATGTCGAAGATCCGCGACCTTACCATGCTATCAGACATTCTGCCGACCGGC
TTCCATGGCGCGGTCAAGGCAGGCGTCGGCGTCGGCTCCACGGTTTATGTCGCCGGCGC
CGGCCCGGTCGGTCTTGCCGCCGCCGCCTCCGCCCGCATTCTGGGTGCGGCCGTTGTCA
TGGTCGGCGATTTCAACAAGGATCGTCTCGCCCATGCGGCAAGAGTCGGTTTTGAACCC
GTCGATCTTTCCAAGGGCGACCGGCTGGGCGACATGATCGCTGAGATCGTCGGCACCAA
TGAGGTGGACAGCGCCATCGACGCCGTCGGCTTCGAAGCCCGCGGCCATTCCGGCGGCG
AACAGCCGGCCATCGTTCTTAACCAGATGATGGAGATTACCCGCGCCGCCGGCTCCATC
GGCATTCCCGGTCTCTACGTCACCGAAGACCCCGGCGCGGTTGACAATGCGGCAAAGCA
GGGCGCCCTGTCGCTGCGCTTCGGCCTTGGCTGGGCGAAGGCGCAATCCTTCCACACCG
GCCAGACACCGGTGCTGAAATATAATCGTCAGCTGATGCAGGCCATCCTGCACGACCGC
CTGCCGATTGCCGATATCGTCAACGCCAAGATCATCGCCCTTGATGATGCCGTGCAGGG
ATATGAAAGCTTTGATCAGGGCGCGGCCACCAAGTTCGTGCTTGATCCGCATGGCGATC
TGCTGAAGGCAGCCTGA

**Amino acid sequence (SEQ ID NO:32)**
MHFDKIMPAEERAGIDVQTTEEMDMSRNRGVVYLKPGQVEVRDIDDPKLEAPDGRRIEH
GVILKVISTNICGSDQHMVRGRTTAMPGLVLGHEITGEVIEKGIDVEMLQVGDIVSVPF
NVACGRCRCCKSQDTGVCLTVNPSRAGGAYGYVDMGGWIGGQARYVTIPYADFNLLKFP
DRDKAMSKIRDLTMLSDILPTGFHGAVKAGVGVGSTVYVAGAGPVGLAAAASARILGAA
VVMVGDFNKDRLAHAARVGFEPVDLSKGDRLGDMIAEIVGTNEVDSAIDAVGFEARGHS
GGEQPAIVLNQMMEITRAAGSIGIPGLYVTEDPGAVDNAAKQGALSLRFGLGWAKAQSF
HTGQTPVLKYNRQLMQAILHDRLPIADIVNAKIIALDDAVQGYESFDQGAATKFVLDPH
GDLLKAA

*FIG. 18*

**ADH17 (NP_534796.1)**
**DNA sequence (SEQ ID NO:33)**
ATGAAGGCACTGGTGCTGGAAGAAAAAGGCAAACTCTCGCTCAGGGATTTTGACATTCC
CGGAGGCGCCGGGTCCGGTGAACTCGGACCGAAGGATGTGCGCATTCGCACCCATACGG
TCGGCATCTGCGGCTCGGACGTTCATTATTATACCCATGGCAAGATCGGCCACTTCGTC
GTCAACGCACCCATGGTGCTCGGCCATGAAGCCTCCGGTACGGTGATCGAAACCGGTTC
CGACGTCACCCATCTGAAGATCGGTGACCGCGTCTGCATGGAGCCTGGTATCCCCGATC
CCACATCGCGGGCCTCGAAACTCGGCATCTATAATGTCGATCCCGCTGTCCGCTTCTGG
GCAACACCGCCGATCCATGGCTGCCTGACGCCTGAGGTCATCCACCCCGCGGCCTTCAC
CTACAAGCTGCCGGATAACGTCTCCTTTGCCGAAGGGGCGATGGTCGAACCCTTCGCCA
TCGGCATGCAGGCGGCACTGCGGGCGCGCATCCAGCCCGGCGATATCGCCGTCGTCACC
GGTGCCGGTCCTATCGGCATGATGGTGGCGCTTGCCGCATTGGCGGGCGGTTGCGCCAA
GGTCATCGTTGCCGATCTCGCTCAGCCGAAGCTTGATATCATCGCCGCTTATGACGGCA
TCGAGACCATCAATATCCGCGAGCGCAACCTTGCCGAAGCGGTTTCGGCCGCCACGGAT
GGCTGGGGTTGCGATATCGTCTTCGAATGCTCAGGTGCGGCACCCGCCATACTCGGCAT
GGCGAAACTGGCGCGACCGGGCGGTGCCATCGTGCTCGTTGGCATGCCGGTTGACCCGG
TTCCGGTCGATATCGTCGGCCTTCAGGCCAAAGAGCTGCGGGTGGAAACGGTATTCCGT
TACGCCAACGTCTATGACCGCGCGGTGGCCCTCATCGCCTCCGGCAAGGTTGATCTCAA
GCCATTGATTTCGGCCACCATTCCCTTCGAAGACAGTATCGCCGGTTTCGACCGTGCGG
TGGAAGCGCGGGAAACGGATGTGAAGTTGCAGATCGTCATGCCGCAATAA

**Amino acid sequence (SEQ ID NO:34)**
MKALVLEEKGKLSLRDFDIPGGAGSGELGPKDVRIRTHTVGICGSDVHYYTHGKIGHFV
VNAPMVLGHEASGTVIETGSDVTHLKIGDRVCMEPGIPDPTSRASKLGIYNVDPAVRFW
ATPPIHGCLTPEVIHPAAFTYKLPDNVSFAEGAMVEPFAIGMQAALRARIQPGDIAVVT
GAGPIGMMVALAALAGGCAKVIVADLAQPKLDIIAAYDGIETINIRERNLAEAVSAATD
GWGCDIVFECSGAAPAILGMAKLARPGGAIVLVGMPVDPVPVDIVGLQAKELRVETVFR
YANVYDRAVALIASGKVDLKPLISATIPFEDSIAGFDRAVEARETDVKLQIVMPQ

*FIG. 19*

## ADH18 (NP_532090.1)
### DNA sequence (SEQ ID NO:35)
ATGTCAAAACGGATCGTTTTTCACGGCGAAAATGCCGCCTGTTTCAGCGATGACTTCAA
AAACCTGGTGGAGGGCGGCGCGGAAATCGCTCTGCTGCCGGATCAACTCGTCACCGAGG
AAGACCGCAACGCCTATCGCAAAGCCGATATCATCGTTGGCGTCAAATTTGATGCATCG
TTGCCGACGCCTGAAAGACTGACGCTGTTTCATGTGCCCGGCGCCGGTTATGACGCCGT
CAATCTCGACCTGCTGCCGAAAAGCGCGGTCGTGTGCAACTGCTTTGGCCATGATCCCG
CAATTGCCGAATATGTGTTTTCAGCCATTCTCAACCGTCATGTTCCGTTGCGCGATGCC
GACAACAAATTGCGCGCCGGCCAGTGGGCCTACTGGTCCGGTTCGACCGAGCGCCTGCA
CGACGAAATGTCCGGAAAAACCATCGGTCTTCTCGGCTTCGGCCATATCGGGAAGGCCA
TTGCGGTCCGCGCGAAGGCGTTCGGAATGCAGGTCAGCGTCGCCAATCGCAGCCGCGTG
GAAACGTCGGATCTGGTAGACCGCTCCTTCACACTGGATCAGCTCAACGAATTCTGGCC
GACCGCAGATTTCATCGTCGTCTCCGTACCACTAACGGACACGACACGCGGGATCGTCG
ATGCGGAGGCTTTCGCAGCGATGAAATCCGGTGCCGTCATCATCAATGTCGGGCGCGGC
CCGACCATAGACGAGCAGGCGCTTTATGACGCGCTGAAAAGCGGAACCATCGGCGGTGC
GGTCATCGATACCTGGTACGCCTATCCGTCACCCGACGCGCCGACGAGACAACCGTCCG
CACTGCCATTCAATCAACTCGAGAACATCATCATGACGCCGCACATGTCCGGCTGGACC
AGTGGAACGGTGCGGCGGCGGCAGCAGACGATCGCGGAAAACATCAATCGGCGGCTGAA
GGGGCAAGACTGCATCAACATCGTCCGCACCGCGTCTGAATAG

### Amino acid sequence (SEQ ID NO:36)
MSKRIVFHGENAACFSDDFKNLVEGGAEIALLPDQLVTEEDRNAYRKADIIVGVKFDAS
LPTPERLTLFHVPGAGYDAVNLDLLPKSAVVCNCFGHDPAIAEYVFSAILNRHVPLRDA
DNKLRAGQWAYWSGSTERLHDEMSGKTIGLLGFGHIGKAIAVRAKAFGMQVSVANRSRV
ETSDLVDRSFTLDQLNEFWPTADFIVVSVPLTDTTRGIVDAEAFAAMKSGAVIINVGRG
PTIDEQALYDALKSGTIGGAVIDTWYAYPSPDAPTRQPSALPFNQLENIIMTPHMSGWT
SGTVRRRQQTIAENINRRLKGQDCINIVRTASE

*FIG. 20*

**ADH19 (NP_531523.1)**
**DNA sequence (SEQ ID NO:37)**
ATGCGCTTCATCGATCTTCCGTCCCATGGTGGCCCGGAAGTGATGCAGTCTTCAAAAGC
ACCTTTGCCGAAACCCGCCCGCGGGGAGATTCTCGTTAAGGTCGAGGCGGCGGGGGTTA
ACCGTCCAGACGTCGCGCAGAGACAGGGCATCTATCCGCCACCCAAAGGTGCAAGCCCC
ATCCTCGGGCTGGAAATCGCCGGCGAGGTCGTTGCACTCGGAGAGGGCGTCGATGAGTT
CAAGCTCGGCGACAAGGTCTGTGCGCTCGCCAATGGCGGCGGTTACGCGGAATATTGCG
CCGTTCCCGCCGGGCAGGCCCTGCCCTTCCCCAAAGGTTACGACGCCGTCAAAGCTGCC
GCACTGCCGGAAACCTTCTTCACCGTCTGGGCCAATCTCTTCCAGATGGCTGGCCTGAC
GGAAGGTGAGACCGTGCTCATCCACGGCGGCACCAGCGGCATCGGCACAACGGCGATCC
AGCTTGCGAAAGCCTTTGGCGCTGAGGTTTATGCCACGGCGGGCTCGGCGGAAAAATGC
GAGGCCTGCGTGAAGCTCGGCACTAAGCGCGCGATCAACTACCGCGAGGAGGATTTCGC
CGAAATCGTGAAATCCGAAACCGGCGGCAAGGGCGTCGATGTCGTTCTCGACATGATCG
GTGCGGCCTATTTCGAAAAGAACCTTGCGGCCCTCGCCAAGGATGGCTGCCTTTCCATC
ATCGCCTTTCTGGGTGGTGCGACAGCCGAGAAGGTCGACCTGCGGCCGATCATGGTCAA
ACGCCTCACCGTCACCGGCTCCACCATGCGCCCCCGAACGGCCGACGAGAAGCGCGCCA
TCCGCGATGAGCTTGTCGAGCAGGTCTGGCCGCTCATCGAAAGCGGCAAGGTCGCGCCT
GTGATCAACCGGGTGTTCACGCTGGAAGAGGTCGTGGACGCGCACCGGTTGATGGAAAG
CAGCAATCATATCGGCAAGATCGTGATGAAGGTGTCGTGA

**Amino acid sequence (SEQ ID NO:38)**
MTPTSEELPLPMSDTKTLPETMRFIDLPSHGGPEVMQSSKAPLPKPARGEILVKVEAAG
VNRPDVAQRQGIYPPPKGASPILGLEIAGEVVALGEGVDEFKLGDKVCALANGGGYAEY
CAVPAGQALPFPKGYDAVKAAALPETFFTVWANLFQMAGLTEGETVLIHGGTSGIGTTA
IQLAKAFGAEVYATAGSAEKCEACVKLGTKRAINYREEDFAEIVKSETGGKGVDVVLDM
IGAAYFEKNLAALAKDGCLSIIAFLGGATAEKVDLRPIMVKRLTVTGSTMRPRTADEKR
AIRDELVEQVWPLIESGKVAPVINRVFTLEEVVDAHRLMESSNHIGKIVMKVS

*FIG. 21*

*FIG. 22*

*FIG. 23*

DEHUH activity assay for ADH20

FIG. 24

| Rxn Conditions | |
| --- | --- |
| Alginate (partially degraded) | 0.58% (w/v) |
| NAD(P)H | 0.4 mM |
| VS_OAL | 0.0054 mg/mL |
| DEHUH (ADH11 and ADH20) | 0.01 mg/mL |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61024160 A
- US 4873192 A
- US 245537 A
- US 7189545 B

### Non-patent literature cited in the description

- **DEVERAUX et al.** *Nucleic Acids Research,* 1984, vol. 12, 387-395
- **KUNKEL.** *Proc. Natl. Acad. Sci. USA.,* 1985, vol. 82, 488-492
- **KUNKEL et al.** *Methods in Enzymol,* 1987, vol. 154, 367-382
- **WATSON, J. D. et al.** Molecular Biology of the Gene. Benjamin/Cummings, 1987
- **DAYHOFF et al.** Atlas of Protein Sequence and Structure. Natl. Biomed. Res. Found, 1978
- **ARKIN ; YOURVAN.** *Proc. Natl. Acad. Sci. USA,* 1992, vol. 89, 7811-7815
- **DELGRAVE et al.** *Protein Engineering,* 1993, vol. 6, 327-331
- A model of evolutionary change in proteins. Matrices for determining distance relationships. **DAYHOFF et al.** Atlas of protein sequence and structure. National Biomedical Research Foundation, 1978, vol. 5, 345-358
- **GONNET et al.** *Science,* 1992, vol. 256 (5062), 14430-1445
- **NEEDLEMAN ; WUNSCH.** *J. Mol. Biol.,* 1970, vol. 48, 444-453
- **E. MEYERS ; W. MILLER.** *Cabios,* 1989, vol. 4, 11-17
- **ALTSCHUL et al.** *J. Mol. Biol,* 1990, vol. 215, 403-10
- **ALTSCHUL et al.** *Nucleic Acids Res,* 1997, vol. 25, 3389-3402
- **COLIGAN et al.** Current Protocols in Protein Science. John Wiley & Sons, Inc, 1995
- **SPOEHR.** *Archive of Biochemistry,* vol. 14, 153-155
- **KESHAV et al.** *J Bacteriol.,* 1990, vol. 172, 2491-2497